(19) Europäisches Patentamt
European Patent Office
Office européen des brevets

(11) Numéro de publication : **0 450 995 B1**

(12) **FASCICULE DE BREVET EUROPEEN**

(45) Date de publication du fascicule du brevet : **08.06.94 Bulletin 94/23**

(21) Numéro de dépôt : **91400498.1**

(22) Date de dépôt : **25.02.91**

(51) Int. Cl.$^5$ : **C07D 317/28,** A61K 31/335, C07D 317/30, C07C 215/62, C07C 225/18, C07C 205/45, C07D 339/06, A61K 31/165

(54) **Nouvelles propanamines, leurs propriétés pharmacologiques et leur application à des fins thérapeutiques, notamment antidiarrhéiques.**

(30) Priorité : **28.02.90 FR 9002495**

(43) Date de publication de la demande : **09.10.91 Bulletin 91/41**

(45) Mention de la délivrance du brevet : **08.06.94 Bulletin 94/23**

(84) Etats contractants désignés : **AT BE CH DE DK ES FR GB GR IT LI LU NL SE**

(56) Documents cités :
**CH-A- 539 051**
**CH-A- 620 922**
**GB-A- 777 794**
**US-A- 3 182 084**
**US-A- 3 714 159**
**TETRAHEDRON, vol. 46, no. 3, février 1990, pages 1025-1038, Pergamon Press Plc,GB; A. JONCZYK et al.: "Reactions of carbanions from 2-(dialkylamino)-arylacetonitriles with acetylene - simple synthesis of 1,3-dienamines and 1,4-diketones"**

(73) Titulaire : **JOUVEINAL S.A.**
**Tour Maine Montparnasse**
**33 Avenue du Maine**
**F-75755 Paris Cedex 15 (FR)**

(72) Inventeur : **Calvet, Alain**
**Résidence Ronsard,**
**3 Rue de Gatine**
**F-94240 L'Hay les Roses (FR)**
Inventeur : **Grouhel, Agnès**
**2, rue des Peupliers**
**F-92190 Meudon (FR)**
Inventeur : **Jacobelli, Henri**
**65, av. du Général de Gaulle**
**F-91550 Paray Vieille Poste (FR)**
Inventeur : **Junien, Jean-Louis**
**36, avenue Eiffel**
**F-92310 Sevres (FR)**
Inventeur : **Pascaud, Xavier**
**41, rue de Charenton**
**F-75012 Paris (FR)**

(74) Mandataire : **Bourgognon, Jean-Marie et al**
**Cabinet Flechner**
**22, Avenue de Friedland**
**F-75008 Paris (FR)**

**EP 0 450 995 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouvelles propanamines, leurs propriétés pharmacologiques et leur application à des fins thérapeutiques, notamment comme antidiarrhéïques.

Depuis longtemps, la morphine et ses dérivés sont utilisés comme antidiarrhéïques en dépit de leurs effets secondaires néfastes.

Pour remédier à cet état, la recherche de composés de synthèse d'activité plus sélective a abouti au développement de molécules actives sur le péristaltisme intestinal telles que le Diphénoxylate (DCI) et plus récemment le Lopéramide (DCI) qui est le 4-(4-chlorophényl)-4-hydroxy-N,N-diméthyl-α,α-diphényl-1-pipéridine butanamide.

En fait, ces composés sont apparentés à la Mépéridine (DCI) qui est un analgésique connu pour être de type morphinique (The Pharmaceutical Basis of Therapeutics - Goodman and Gilman's - 6ème Ed. 1980 - p. 513-518). Aussi, ces composés antidiarrhéïques peuvent provoquer, lorsqu'ils sont absorbés de façon excessive, des effets secondaires indésirables comme des symptômes de dépendance, ou une augmentation démesurée de la durée du transit intestinal pouvant avoir des conséquences dramatiques : constipation puis, plus gravement distension et, à l'extrême, perforation de l'intestin (Textbook of Pharmacology - W.C. Bowman and M.J. Rand, 2ème Ed. 1980, p. 25-36 et 25-40) .

Or, on a maintenant trouvé des propanamines qui présentent des avantages considérables pour les traitements des états diarrhéïques d'étiologies diverses. En effet, indépendamment de leur faible toxicité et de leur activité indéniable par voie orale dans cette pathologie, elles sont, même à forte dose, pratiquement dépourvues d'effets sur la durée du transit gastrointestinal du rat et n'induisent pas de phénomène de dépendance chez la souris.

Les propanamines de l'invention répondent à la formule générale (I)

```
      R1
      |
R2-C-(CH2)2-W-R5
      |
      N
     / \
R3      R4                (I)
```

dans laquelle :

R1 est un radical phényle, éventuellement mono di ou trisubstitué de manière identique ou différente par des atomes d'halogène, des radicaux alkyle inférieur, haloalkyle inférieur, alkoxy inférieur,

ou est un radical hétéroaryle monocyclique de 5 ou 6 chainons dans lequel l'unique hétéroatome est l'azote, l'oxygène ou le soufre,

R2 est un radical alkyle inférieur

R3 et R4 sont un atome d'hydrogène, un radical alkyle inférieur, alkènyle inférieur, cycloalkylalkyle inférieur, et sont différents ou identiques sans toutefois être tous deux cycloalkylalkyle,

ou forment ensemble, avec l'atome d'azote auquel ils sont reliés, un hétérocycle saturé comprenant un seul hétéroatome et constitué de 5 à 6 chainons,

R5 est un radical cycloalkyle de 5 à 7 chainons, un radical phényle ou un radical hétéroaryle monocyclique de 5 ou 6 chainons dans lequel l'unique hétéroatome est l'azote, l'oxygène ou le soufre et qui sont éventuellement mono, di ou trisubstitués de façon identique ou différente par des atomes d'halogène, des radicaux alkyle inférieur, haloalkyle inférieur ou alkoxy inférieur,

et W représente

un groupe =CH-QH,

ou un hétérocycle =C[Q-(CH2)n-Q],

ou un groupe =C=Q,

dans lesquels :

Q est un atome d'oxygène ou de soufre,

n a pour valeur 2 ou 3,

W n'étant =C=Q que lorsque R3 et R4 ne sont pas tous deux l'hydrogène.

Les propanamines de l'invention comportent au moins un atome de carbone asymétrique responsable de formes énantiomères qui sont incluses dans l'invention au même titre que les formes racémiques.

L'invention se rapporte également aux sels d'addition des propanamines avec des acides, minéraux ou organiques, en particulier avec ceux pharmaceutiquement acceptables.

A cet effet, les sels avec l'acide chlorhydrique et l'acide maléïque sont les plus utilisés, toutefois, des sels d'addition également acceptables sont obtenus avec les acides acétique, benzènesulfonique, camphosulfonique, citrique, éthanesulfonique, fumarique, bromhydrique, lactique, malique, méthanesulfonique, mucique, nitrique, pamoïque, phosphorique, salicylique, stéarique, succinique, sulfurique, tartrique.

Sauf mention expresse contraire, les radicaux alkyle, alkènyle, haloalkyle ou alkoxy inférieurs comprennent de 1 à 4 atomes de carbone, les radicaux cycloalkyle de 3 à 7 atomes de carbone, les atomes d'halogène sont essentiellement le fluor, le chlore et le brome et les radicaux phényle lorsqu'ils sont sustitués le sont de préférence dans les positions méta et para.

Dans le souci de clarification du texte qui suit et pour faciliter le classement par structures des composés de l'invention,

i) selon les significations précédemment précisées pour W les propanamines (I) sont incluses :

- dans un groupe "A" lorsque W est un groupe =CH-QH,
- dans un groupe "D" lorsque W est un hétérocycle =C[Q-(CH2)n-Q],
- dans un groupe "C" lorsque W est un groupe =C=Q, ce qui est illustré par les formules :

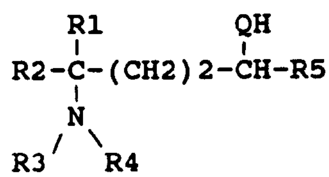

(I.A)

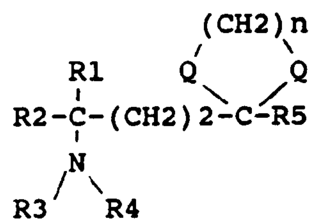

(I.D)

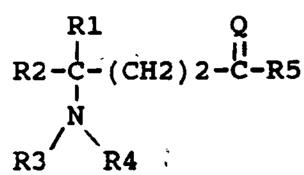

(I.C)

ii) et, pour chaque groupe mentionné, il est indiqué en troisième position,

- l'indice 1 lorsque dans la propanamine R3 et R4 sont tous deux l'hydrogène, ce qui par ailleurs est exclu lorsque W représente le groupe =C=Q,
- l'indice 2 lorsque l'un seul de R3 ou R4 représente l'hydrogène,
- l'indice 3 lorsque R3 et R4 sont tous deux différents de l'hydrogène,

iii) enfin, lorsqu'il est expressément nécessaire d'indiquer l'identité de l'atome Q, un dernier indice : /O ou /S en donne la précision.

Dans l'ensemble de propanamines (I) on préfére celles dans lesquelles R1 est un radical phényle éventuellement mono ou disubstitué,

R2 est un radical alkyle inférieur linéaire,

R3 et R4 identiques ou différents sont un atome d'hydrogène, un radical alkyle inférieur, alkènyle inférieur ou cycloalkylalkyle inférieur,

R5 est un radical phényle éventuellement substitué et, parmi celles ci, les propanamines (I) dans lesquelles :

W représente des groupes dans lesquels :

- Q est un atome d'oxygène et n a pour valeur 2 lorsqu'il représente un groupe 1,3-dioxolane-2,2-diyle (formules appartenant au groupe I.D).
- ou représente un groupe =C=Q dans lequel Q est l'oxygène, R3 et R4 n'étant pas tous deux l'hydrogène

(formules I.C.2/O ou I.C.3/O).

On préfère particulièremment les composés de formule (I.D.2) qui comprend les produits dans lesquels W est 1,3-dioxolane 2,2-diyle, R3 est un radical alkyle inférieur ou cycloalkylalkyle inférieur et R4 est l'hydrogène et, dans ceux-ci, le composé décrit à l'exemple 3.f qui est le 2-[3-N-méthylamino-3-(4-trifluoro-méthylphényl)-pentan-1-yl]-2-(3,4,5-triméthoxyphényl)-1,3-dioxolane, et les composés de formule (I.C.3/O) dans lesquels W est carbonyle, et R3 R4, identiques ou différents, sont alkyle inférieur. Dans ceux-ci, on préfère la propanamine (I) décrite à l'exemple 9.b.2 qui est la 4-N,N-diméthylamino-4-(4-méthylphényl)-1-(3,4,5-triméthoxyphényl)-hexan-1-one.

L'invention vise également un procédé de préparation des propanamines (I) illustré aux schémas ci après qui consiste :

A - pour préparer une propanamine dans laquelle R3 et R4 sont l'hydrogène, et comme montré au schéma 1,

A.1 - à réduire le groupe =C=Q d'un précurseur nitré de formule (II)

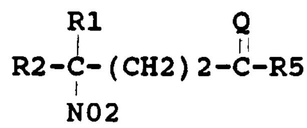

(II)

par un hydrure métallique ou organométallique (Hm.3) de formule générale (Hm)

M1(t) M2 H(r) Rx(s) (Hm)

dans laquelle

M1 est un métal alcalin tel que le lithium ou le sodium et dont l'indice (t) a pour valeur 0 ou 1,

M2 est un élément du groupe III de la classification périodique tel que l'aluminium ou le bore,

(r) est l'indice représentatif du nombre d'atomes d'hydrogène de l'hydrure et de valeurs 1, 2, 3 ou 4,

Rx est un groupe carbonitrile, un radical alkyle ou alkoxy inférieur et d'indice (s) représentatif de valeurs 0, 1, 2 ou 3, et dont les indices répondent à la relation :

$$(r) + (s) - (t) = 3$$

et pour lesquels dans les hydrures (Hm.3) on préfère que M2 soit le bore et, lorsque (s) a pour valeur 1 il soit un groupe carbonitrile,

pour obtenir un intermédiaire (III)

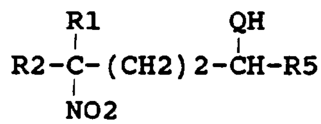

(III)

que l'on réduit par une hydrogénation catalysée par un métal du groupe VIII de la classification périodique ou un de leur oxyde ou encore un de leur sel et de façon préférée par le platine, le palladium ou le nickel et leurs dérivés précédemment mentionnés et, plus particulièrement pour le nickel, ses alliages avec l'aluminium comme dans l'alliage de Raney pour obtenir une propanamine (I.A.1)

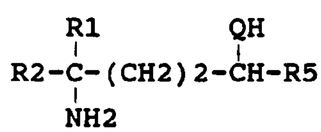

(I.A.1)

A.2 - à condenser un réactif bifonctionnel HQ-(CH2)n-QH dans lequel Q est l'oxygène ou le soufre et n a pour valeurs 2 ou 3 , avec le précurseur (II) précédemment décrit, pour obtenir un intermédiaire hétérocyclique (IV)

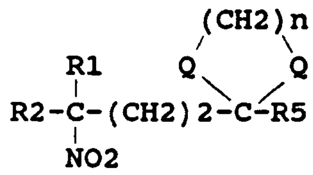

(IV)

que l'on réduit par une hydrogénation catalysée par les éléments du groupe VIII précédemment précisés en une propanamine (I.D.1)

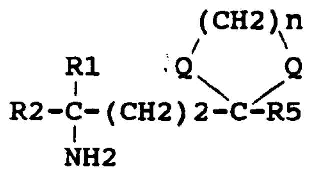

(I.D.1)

SCHEMA 1

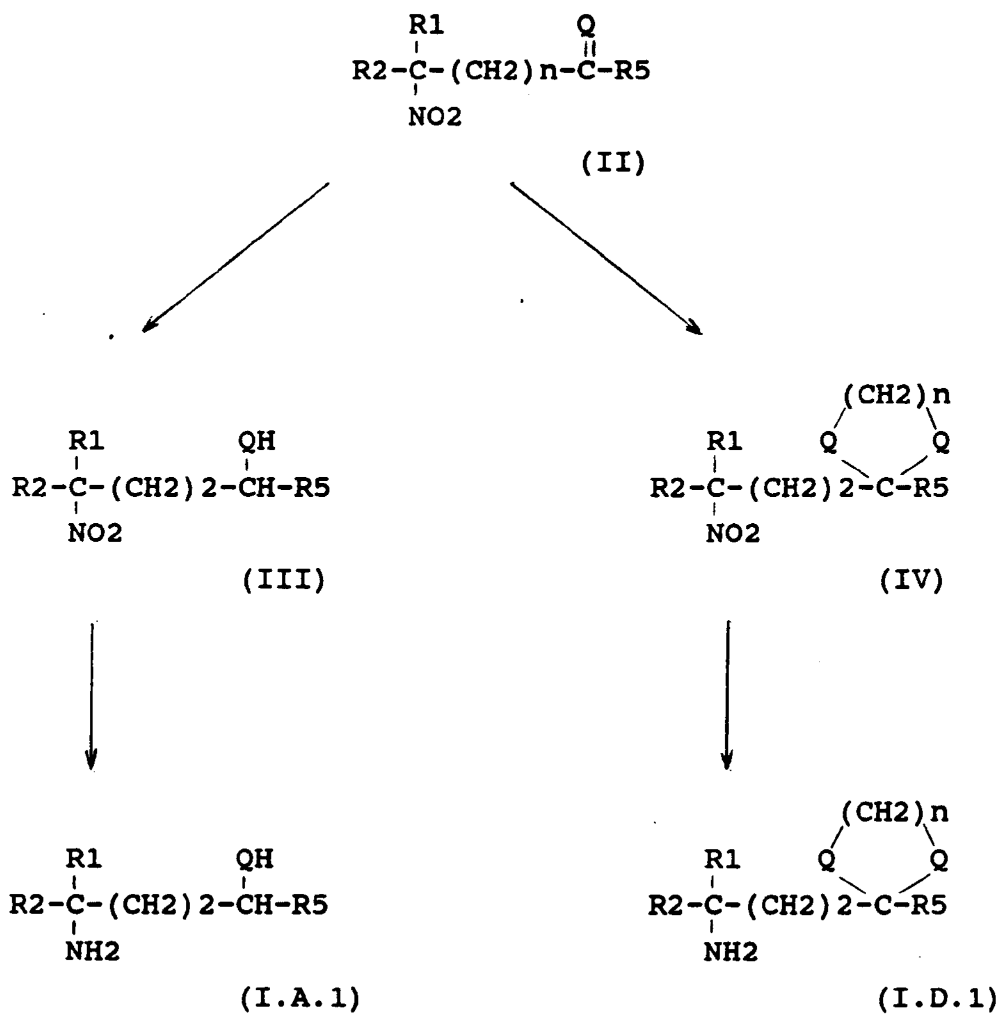

(II)

(III)

(IV)

(I.A.1)

(I.D.1)

B - et, pour préparer une propanamine dans laquelle R4 différent de R3 est l'hydrogène, le procédé consiste , tel que montré au schéma 2 :

B.1 - à alkyler une propanamine (I) de formule (I.A.1) ou de formule (I.D.1) par un réactif d'alkylation R3X1 dans lequel X1 est un atome d'halogène tel que le chlore, le brome ou l'iode, pour obtenir respectivement une propanamine (I.A.2) ou une propanamine (I.D.2)

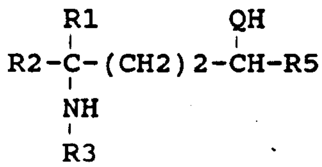

(I.A.2)

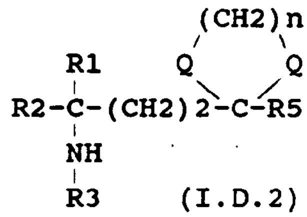

(I.D.2)

B.2 - à acyler une propanamine (I) de formule (I.A.1) ou de formule (I.D.1) par un réactif (R6CO)pX2 dans lequel R6 est l'hydrogène ou un radical homologue carboné inférieur d'un atome de carbone à R3 (R3 = CH2-R6), X2 est, lorsque p est égal à 1, un atome d'halogène comme le chlore, le brome ou un radical hydroxyle, et, lorsque p est égal à 2, est un atome d'oxygène, pour obtenir un intermédiaire N-carboxamide (V.A.2) ou un intermédiaire N-carboxamide (V.D.2)

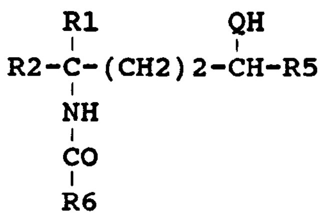

(V.A.2)

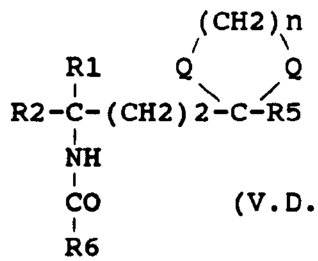

(V.D.2)

## SCHEMA 2

Groupe A : W = =C=Q;

Groupe D : W = =C[Q-(CH2)n-Q]

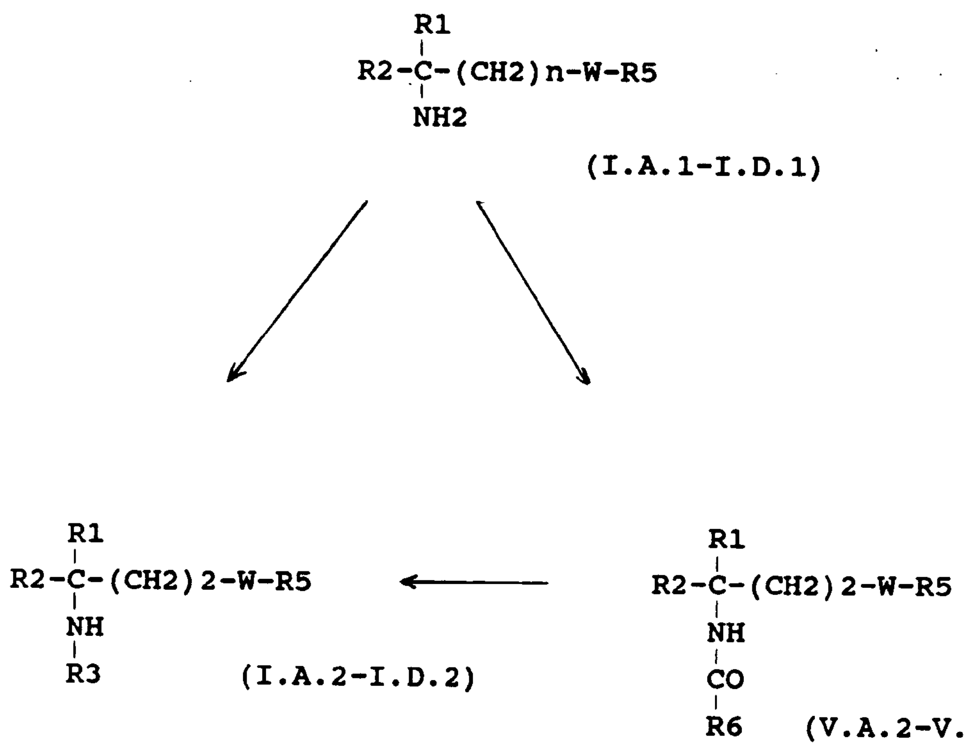

(I.A.1-I.D.1)

(I.A.2-I.D.2)

(V.A.2-V.D.2)

que l'on réduit par un hydrure métallique ou organométallique (Hm.2) de formule (Hm) précédemment définie et dans laquelle M2 est l'aluminium et lorsqu'il représente le bore (r) a pour valeur 3 et (t) a pour valeur O,

pour obtenir respectivement, une propanamine (I.A.2) ou une propanamine (I.D.2) précédemment décrite,

B.3 - et, ou bien à oxyder par un diatome ou un ion polyatomique dont le potentiel normal redox E° est supérieur à 0,60 V une propanamine (I.A.2) dans laquelle Q est l'oxygène,

ou bien à hydrolyser par une solution acide et/ou oxydante une propanamine (I.D.2),

pour obtenir une propanamine (I.C.2) dans laquelle Q est l'oxygène et de formule (I.C.2/O)

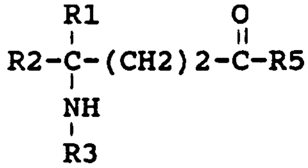

(I.C.2/O)

B.4 - et, pour préparer une propanamine dans laquelle Q est le soufre et de formule (I.C.2/S)

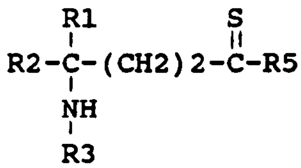

(I.C.2/S)

ou bien à faire réagir la propanamine (I.C.2/O) avec un réactif Rz-NH2 dans lequel Rz est l'hydrogène, une fonction amine primaire ou hydroxyle, ou encore un radical alkyle inférieur ou phényle pour obtenir un composé intermédiaire (VI.1)

SCHEMA 3

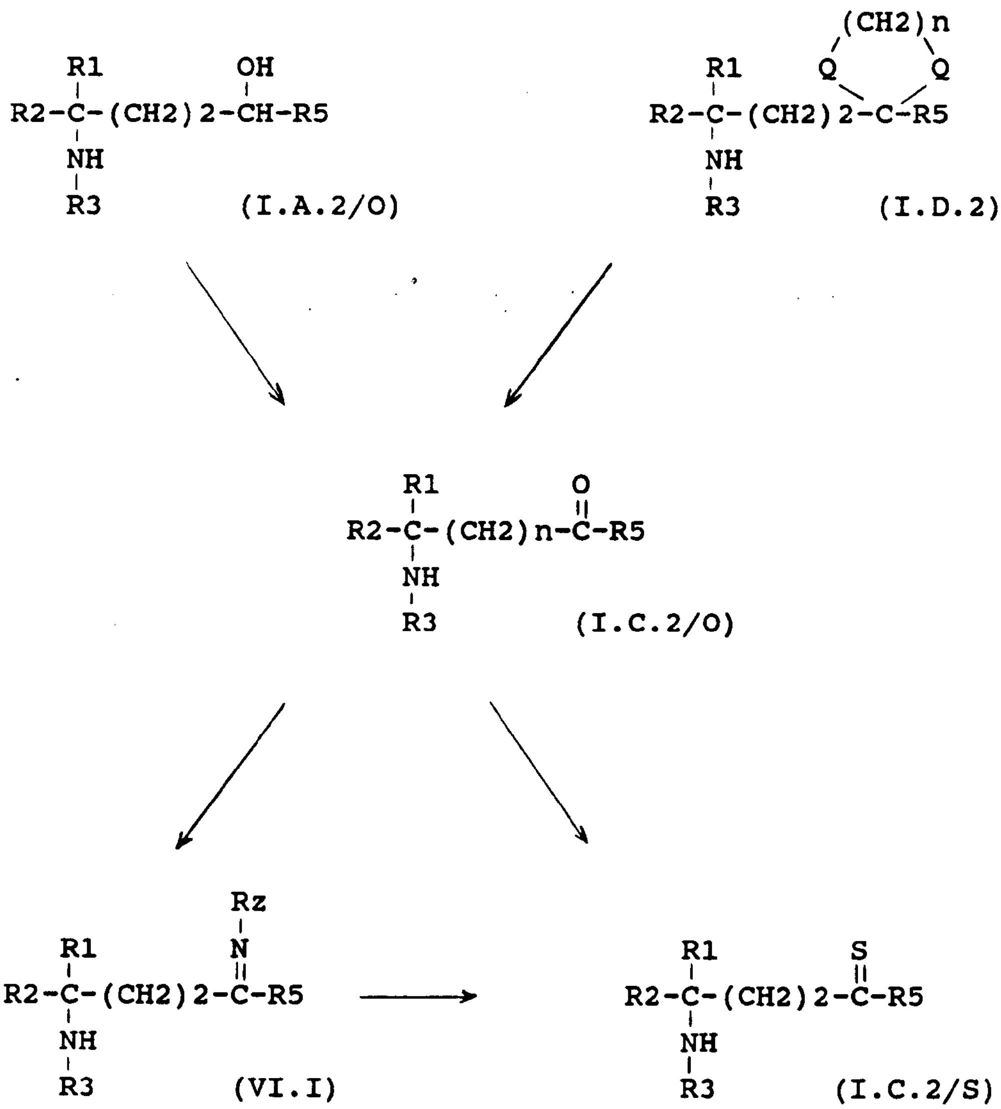

(I.A.2/O) (I.D.2)

(I.C.2/O)

(VI.I) (I.C.2/S)

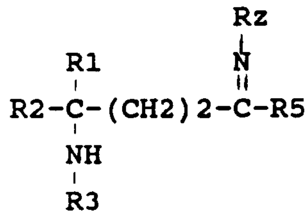

(VI.1)

que l'on engage dans une réaction de thioxo substitution avec l'hydrogène sulfuré ou le sulfure de carbone ou le monochlorure de soufre ou encore avec un acide S-alkylthioïque ou S-phénylthioïque,

ou bien à faire réagir la propanamine (I.C.2/O) avec le pentasulfure de phosphore pour obtenir le composé (I.C.2/S).

C - et, pour préparer une propanamine (I) dans laquelle R3 et R4 ne sont pas l'hydrogène,

C.1 - et, lorsque R3 et R4 identiques sont alkyle ou alkènyle inférieur,

à dialkyler une propanamine (I) de formule (I.A.1) ou de formule (I.D.1) par un aldéhyde R7-CHO dans lequel R7 est l'hydrogène ou un radical alkyle ou alkènyle, homologue carboné comprenant un atome de carbone de moins que R3 et R4 (R3 = R4 = -CH2-R7), et avec un agent de réduction qui est un hydrure métallique ou organo-métallique (Hm.3) précedemment défini ou bien l'acide formique ou un de ses sels pour obtenir selon la propanamine de départ engagée soit une propanamine (I.A.3) soit une propanamine (I.D.3) tel qu'il est montré au schéma 4,

SCHEMA 4

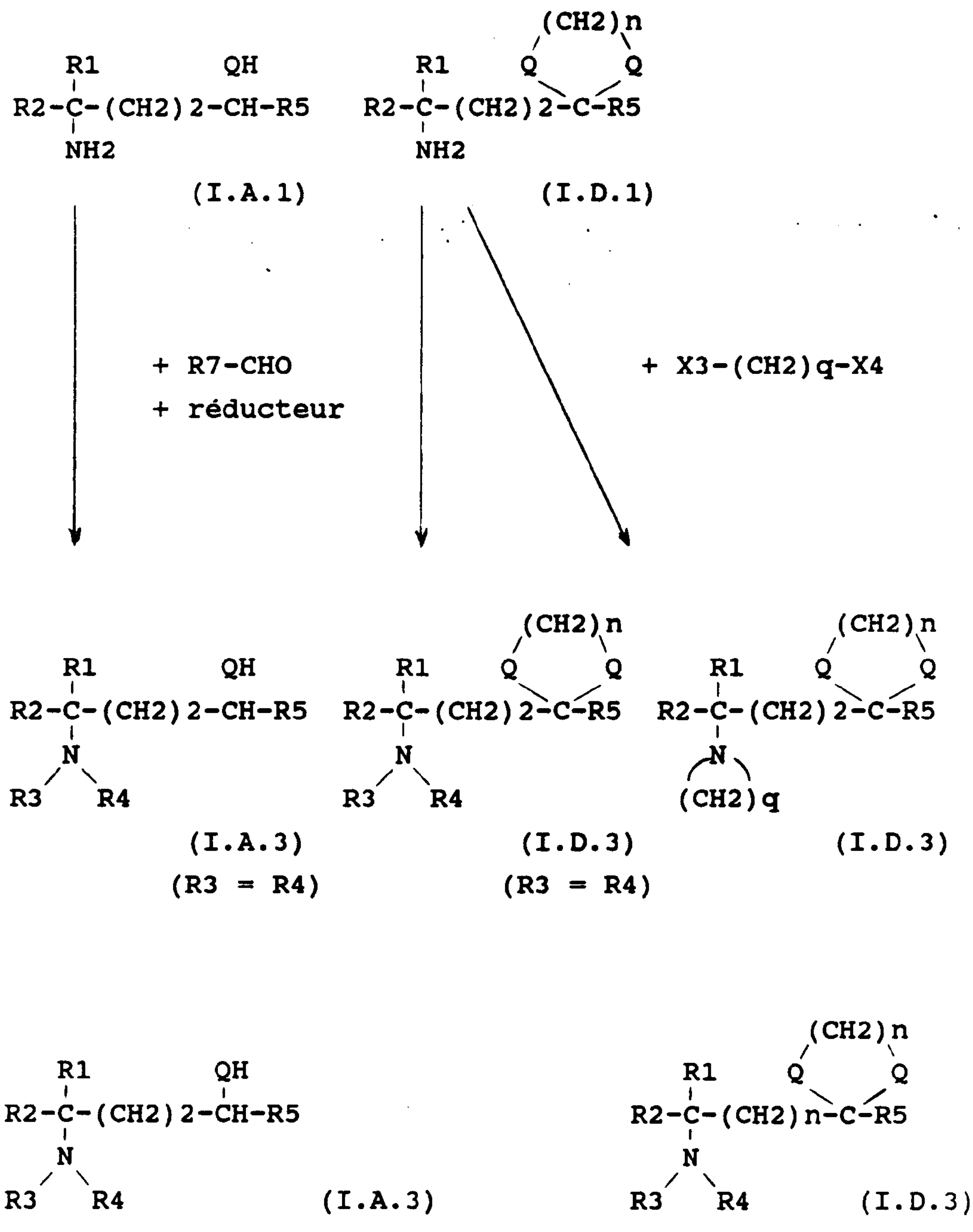


(I.A.1) (I.D.1)

(I.A.3) (R3 = R4) (I.D.3) (R3 = R4) (I.D.3)

(I.A.3) (I.D.3)

C.2 - et, lorsque R3 et R4 forment ensemble et avec l'atome d'azote auquel ils sont liés un hétérocycle saturé comprenant de 5 à 6 chainons, et comme montré au schéma 4,

à dialkyler une propanamine (I.D.1) par un réactif dihalogéné X3-(CH2)q-X4 dans lequel X3 et X4 identiques ou différents sont le chlore, le brome ou l'iode et q a pour valeur 4 ou 5

C.3 - et pour préparer une propanamine (I) dans laquelle R3 et R4 différents ne sont pas l'hydrogène,et

i) - de formule (I.D.3) ou de formule (I.C.3)

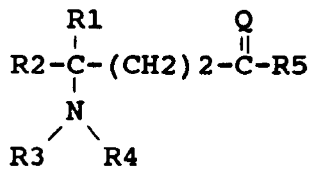

(I.C.3)

à alkyler une propanamine (I) de formule (I.D.2) ou de formule (I.C.2) dans laquelle R4 est l'hydrogène, par un réactif d'alkylation R4-X5 dans lequel

R4 est alkyle inférieur, alkényle inférieur ou cycloalkylalkyle inférieur,

X5 est un atome de chlore, de brome ou d'iode, et

ii) - de formule (I.A.3) ou de formule (I.D.3), à faire réagir une propanamine (I.A.2) ou une propanamine (I.D.2) avec un aldéhyde R7-CH0 précédemment défini et homologue inférieur d'un atome de carbone à R4 (R4 = -CH2-R7) et un agent de réduction tel précédemment défini, ou

iii) - à acyler une propanamine (I.A.2) ou une propanamine (I.D.2) avec un réactif d'acylation R9-COX6 dans lequel R9 est l'hydrogène ou un radical carboné homologue inférieur d'un atome de carbone à R4 (R4 = -CH2-R9) et X6 est un atome de chlore ou de brome ou encore un radical hydroxyle pour obtenir les N-carboxamides (V.A.3) et (V.D.3) correspondants

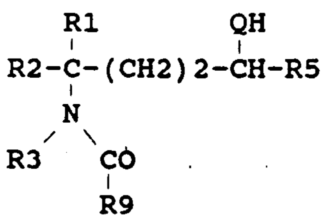

(V..A.3)

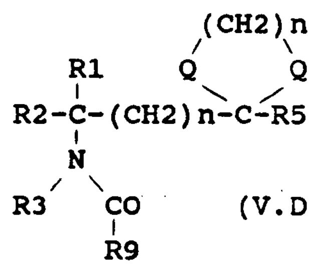

(V.D.3)

qui sont réduits par un hydrure métallique ou organométallique (Hm.2) précédemment défini en une propanamine de formule (I.A.3) ou de formule (I.D.3), et

C.4 - pour obtenir une propanamine (I) de formule (I.A.3) ou de formule (I.D.3),

à substituer le radical carbonitrile d'un amino nitrile de formule (VII.1) ou de formule (VII.2)

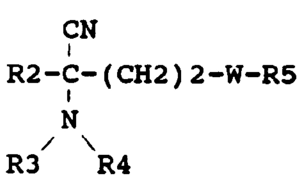

(VII.1)

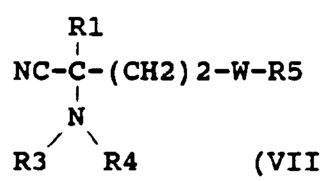

(VII.2)

dans lesquels W représente les groupes définis pour les propanamines (I) excepté le groupe C=Q, R3 et R4 étant tous deux différents de l'hydrogène,

par les radicaux carbonés R1 ou R2 des réactifs organométalliques R1-M-X7 et R2-M-X8 dans lesquels,

R1 représente les radicaux présentés pour les propanamines (I) excepté lorsqu'ils sont substitués par des atomes d'halogène,

R2 représente un radical identique à ceux présentés pour les propanamines (I),

M est un atome métallique bivalent compris parmi le magnésium, le cadmium ou le zinc,

X7 et X8 sont des atomes d'halogène choisis entre le chlore, le brome ou l'iode,

- et de formule (I.D.3),

à condenser une propanamine (I.C.3) avec un réactif bifonctionnel HQ-(CH2)n-QH dans lequel n a pour valeur 2 ou 3 , Q est le soufre ou l'oxygène,

## SCHEMA 5

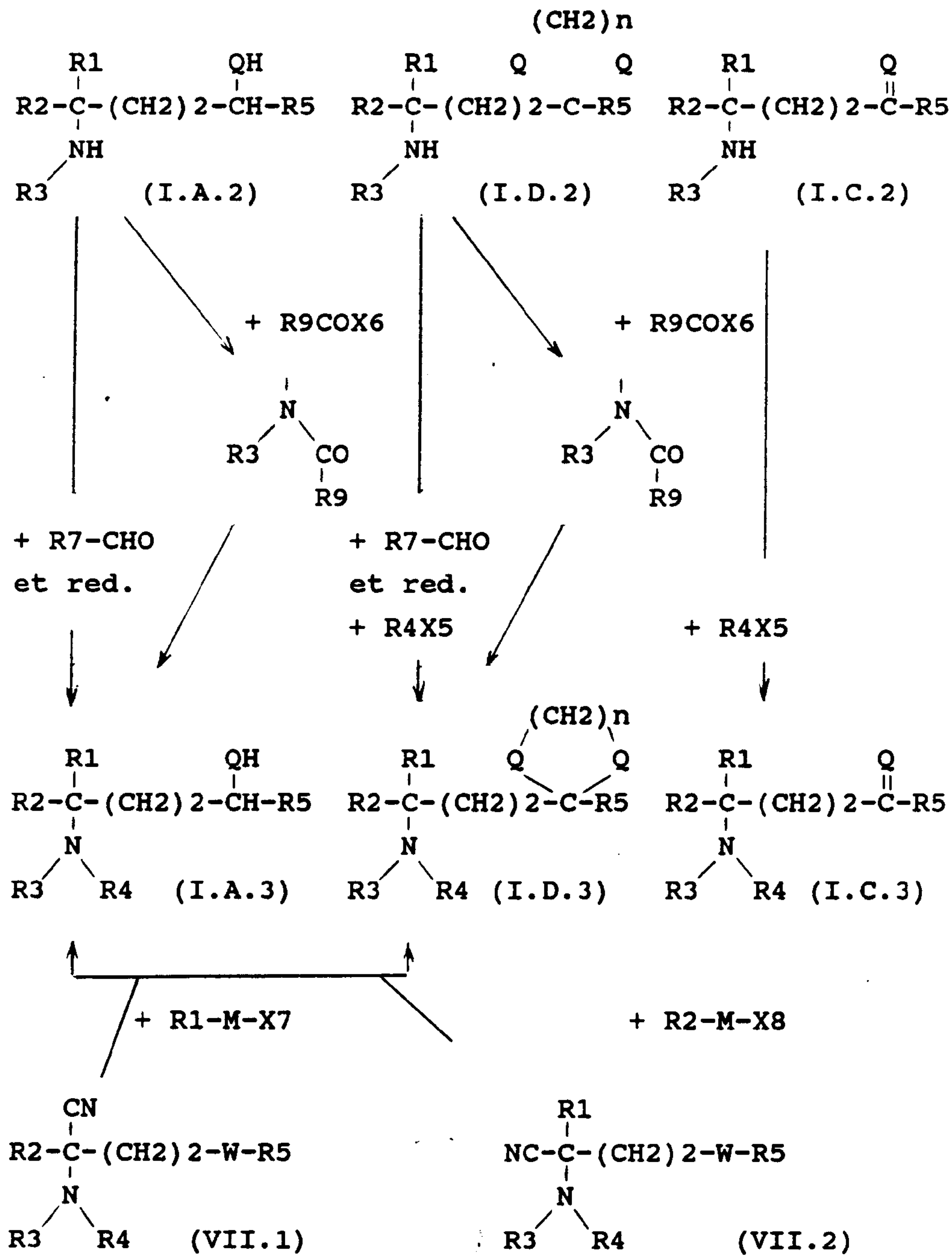


C.5 - comme il est montré au schéma 6,pour préparer des propanamines (I) dans lesquelles R3 et R4 ne sont pas l'hydrogène,

- et de formule (I.A.3) à réduire, par un hydrure métallique ou organométallique (Hm.3) précédemment défini une propanamine de formule (I.C.3)
- et de formule (I.C.3/O)

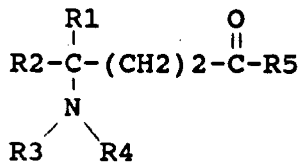

(I.C.3/O)

à oxyder, par des systèmes oxydo réducteurs ioniques mono ou polyatomiques dont le potentiel normal E° à 20°C est supérieur à 0,60 V une propanamine (I.A.3) dans laquelle la fonction QH est hydroxyle et de formule (I.A.3/O)

ou, à hydrolyser par une solution acide ou oxydante une propanamine (I.D.3),

- et de formule (I.C.3/S),

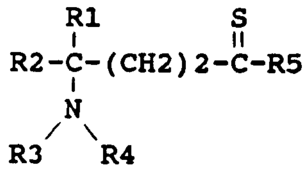

(I.C.3/S)

à faire réagir une propanamine (I.C.3/O) avec un réactif Rz-NH2 précédemment décrit pour obtenir un intermédiaire (VI.2) de formule

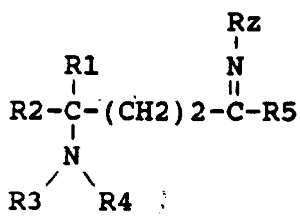

(VI.2)

qui est engagé dans une réaction de thioxo substitution avec les réactifs précédemment décrits,

## SCHEMA 6

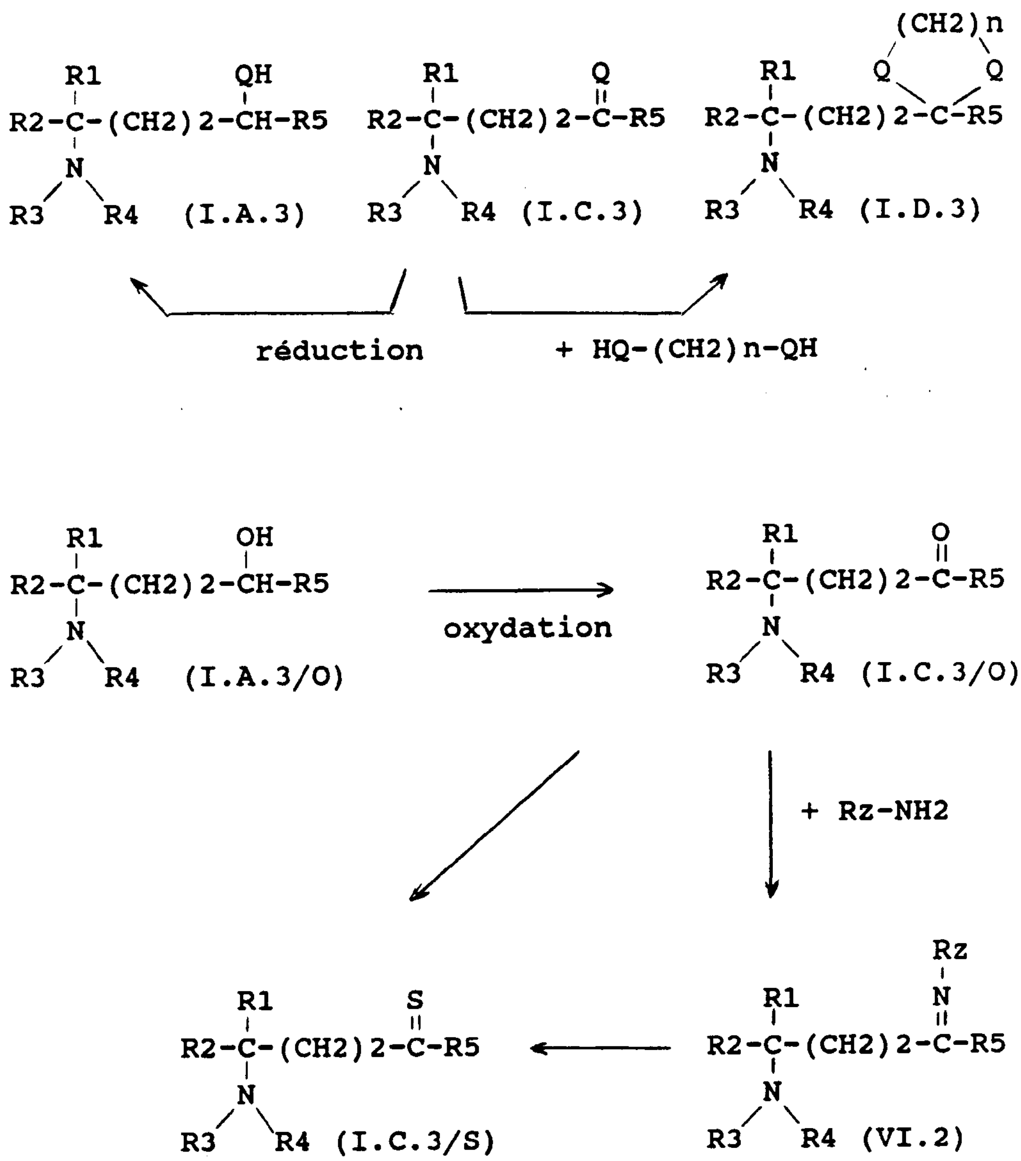


L'invention vise à titre d'intermédiaires les composés de formule (XX)

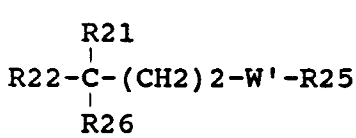

( XX)

dans laquelle :

R21 est un radical phényle, éventuellement mono di ou trisubstitué, de manière identique ou différente, par des atomes d'halogéne, des radicaux alkyle inférieur, haloalkyle inférieur, alkoxy inférieur,

ou est un radical hétéroaryle monocyclique de 5 ou 6 chainons dans lequel l'unique hétéroatome est l'azote, l'oxygène ou le soufre,

ou est un radical carbonitrile lorsque R22 est alkyle,

R22 est un radical alkyle inférieur ou un radical carbonitrile quand R21 est phényle éventuellement substitué ou hétéroaryle,

R25 est un radical cycloalkyle de 5 à 7 chainons, un radical phényle ou un radical hétéroaryle monocyclique de 5 ou 6 chainons dans lequel l'unique hétéroatome est l'azote, l'oxygène ou le soufre et qui sont éventuellement mono, di ou trisubstitués de façon identique ou différente par des atomes d'halogène, des radicaux alkyle inférieur, haloalkyle inférieur ou alkoxy inférieur,

R26 est un radical nitro lorsque R21 ou R22 ne sont pas carbonitrile,

ou, lorsque R21 ou R22 est carbonitrile, est groupe amino -N(R23)R24 dans lequel

R23 et R24 sont un radical alkyle inférieur, alkènyle inférieur, cycloalkylalkyle inférieur, différents ou identiques sans toutefois être tous deux cycloalkylalkyle

ou forment ensemble, avec l'atome d'azote auquel ils sont reliés, un hétérocycle saturé comprenant un seul hétéroatome et constitué de 5 à 6 chainons,

et W' représente

un groupe =CH-QH, ou un hétérocycle =C[Q-CH2)n-Q] quand R21 ou R22 sont carbonitrile,

ou un groupe =C=Q lorsque R26 représente un groupe nitro,

et dans lesquels :

Q est un atome d'oxygène ou de soufre,

n a pour valeur 2 ou 3.

L'invention vise également un procédé de préparation des intermédiaires (XX) de formules (II), (VII.1) et (VII.2) qui consiste :

- pour préparer un précurseur (II), à alkyler un dérivé nitré (VIII) de formule :

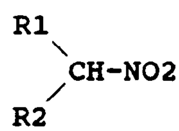

(VIII)

par un réactif (IX.a) de formule :

X9-(CH2)2-W-R5 (IX.a)

dans lequel X9 est un atome d'halogène, comme le chlore ou le brome ou encore représente une fonction -N(R10)R11 dans laquelle R10 et R11 sont alkyle inférieur et de préférence sont méthyle, et dans lequel W représente un groupe =C=Q,

- pour préparer un précurseur (VII.1) ou (VII.2) à alkyler un amino nitrile (X.1) ou (X.2) de formule

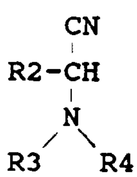

(X.1)

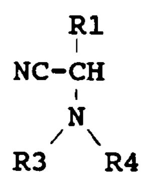

(X.2)

par un réactif (IX.b) de formule

X10-(CH2)2-W-R5 (IX.b)

dans lequel X10 est un atome d'halogène comme le chlore, le brome ou l'iode ou un radical arylsulfonyloxy ou alkylsulfonyloxy qui est préféré et W représente un groupe CH-QH ou un hétérocycle C[Q-(CH2)n-Q] qui est préféré.

Les préparations des composés (VIII), (IX.a), (IX.b), (X.1) et (X.2) utiles à la synthèse des propanamines (I) de l'invention sont présentées dans les exemples illustratifs de l'invention.

Explicitement, le procédé de préparation des composés (II), (VII.1) ou (VII.2), consiste à préparer l'anion d'un composé (VIII), (X.1) ou (X.2), puis à le faire réagir avec un carbocation généré par un agent d'alkylation (IX.a) ou (IX.b).

Ainsi, pour un intermédiaire (VIII), l'anion est favorablement préparé dans un alcool inférieur comprenant

jusqu'à 4 atomes de carbone, par réaction avec un alcoolate de métal alcalin comme le sodium ou le potassium, alors que pour un composé (X.1) ou un composé (X.2), la préparation de l'anion est réalisée dans un solvant aprotique comme le 1,2-diméthoxyéthane, le THF ou encore le DMF, sous l'action de bases appropriées dérivées de l'amidure de lithium telles que le N,N-diéthylamidure de lithium ou le N,N-diisopropyl amidure de lithium.

Pour illustrer ces pratiques, l'alkylation d'un composé (VIII) consiste, pour une mole de produit engagé, à préparer tout d'abord l'alcoolate nécessaire à la formation de l'anion, ce que l'on réalise en ajoutant 0,7 à 5 moles de métal alcalin dans 0,2 à 10 litres d'alcool inférieur approprié, puis à introduire d'abord le dérivé nitré pour en préparer l'anion correspondant et ajouter ensuite un agent d'alkylation (IX.a). Il est nécessaire, lorsque dans cet agent, X9 est dialkylamino inférieur, d'ajouter un excès d'iodure de méthyle pour préparer le dérivé d'ammonium quaternaire correspondant qui, in situ, génère le carbocation dans le milieu de la réaction.

Pratiquement, la préparation de l'alcoolate consiste à ajouter avec les précautions requises de 0,9 à 2,5 moles de sodium qui est le métal alcalin préféré dans 0,25 à 1 litre de méthanol ou d'éthanol à une température inférieure à 20°C, puis après formation de l'alcoolate de sodium, et à une température inférieure ou voisine de 0°C à ajouter une solution alcoolique du composé (VIII). Après formation de l'anion, on introduit de 0,8 à 1,25 moles d'agent d'alkylation (IX.a) en solution alcoolique, puis, si l'agent est une N,N-dialkylamine, il est également ajouté 1,2 à 1,9 moles d'iodure de méthyle. Le milieu réactionnel est maintenu de 2 à 24 h à une température comprise entre 10 et 60°C pour compléter l'alkylation puis traité de façon appropriée pour isoler et purifier le composé intermédiaire (II) obtenu.

Et, pour alkyler un composé (X.1) ou (X.2) en un composé (VII.1) ou (VII.2), la préparation préliminaire de l'anion peut être réalisée avec un produit issu du commerce ou bien un composé préparé "in situ". Cette dernière méthode est celle utilisée pour l'emploi du N,N-diisopropyl amidure de lithium qui est la base préférée pour la préparation d'anions avec des composés (X.1) ou (X.2).

Dans ce cas, pour exemple général, on opère favorablement pour une mole de composé avec 0,5 à 3 litres de THF dans lequel on a préparé préalablement de 0,75 à 1,25 mole de N,N diisopropyl amidure de lithium en mélangeant à une température inférieure à - 10°C des quantités équimoléculaires et correspondantes de n-butyllithium et de diisopropylamine.

L'addition du composé (X) et la formation de l'anion sont réalisées à une température inférieure à 0°C et, de préférence, comprise entre - 50 et - 80°C et dans une durée de 30 minutes à 5 heures. Après celà, on ajoute à la même température de 1,0 à 1,5 moles d'un agent d'alkylation (IX.b) et laisse la température revenir à environ 20°C. On laisse ensuite la réaction à cette température pendant 1 à 24 heures avant de traiter selon les pratiques appropriées pour isoler et purifier le composé (VII.1) ou (VII.2) correspondant ainsi obtenu.

Pour préparer les propanamines (I) de l'invention tel qu'il a été précédemment décrit, le procédé de l'invention fait appel à diverses réactions dont la mise en oeuvre est précisée dans ce qui suit et dans les exemples illustratifs de la partie expérimentale.

Essentiellement, ces réactions sont :

a) - des réactions de réduction réalisées :

a.1)par des hydrures métalliques ou organométalliques de formule générale (Hm) :

$$M1(t)\ M2\ H(r)\ Rx(s)$$

dans laquelle,

M1 est un métal alcalin comme le sodium ou le lithium,

M2 est un élément du groupe III de la classification périodique comme le bore ou l'aluminium,

(t) est égal à zéro ou à un,

(r) est l'indice représentatif du nombre d'atomes d'hydrogène et est égal à 1, 3 ou 4,

Rx est un radical alkyle ou alkoxy inférieur dont l'indice représentatif (s) est égal à 0, 1, 2 ou 3, et ces hydrures répondant à la relation : $r + s - t = 3$.

Les hydrures ainsi définis pouvent être utilisés sous forme de complexes avec des composés organiques tels que les complexes du borane avec le tétrahydro-furane ou le sulfure de diméthyle ou encore en association avec des halogènures métalliques comme le chlorure de lithium ou le chlorure d'aluminium, et qui permettent

a.1.1) de préparer des propanamines (I) ou leurs précurseurs dans lesquels W représente un groupe =CH-QH par réduction d'un composé correspondant dans lequel W est un groupe =C=Q au moyen d'un hydrure métallique ou organo-métallique tel que précédemment décrit et tel qu'il est pratiqué pour obtenir un composé (III) et pour obtenir une propanamine (I.A.3) à partir d'une propanamine (I.C.3).

La réduction des groupes C=Q est favorablement réalisée avec les hydrures dans lesquels M2 est le bore et parmi ceux ci on préfère ceux dans lequel M1 est le sodium et les indices t et s ont respectivement pour valeur 1 et 0.

Dans ce cas, leur mise en oeuvre en prenant pour exemple le borohydrure de sodium $NaBH_4$ est réalisée

dans des solvants protiques miscibles à l'eau tels que des alcools inférieurs comme le méthanol ou l'éthanol.

Ainsi, on procède à la réduction d'une mole de composé en le dissolvant dans 5 à 20 parties (poids/volume) d'alcool puis en ajoutant à la solution et sans dépasser 30°C, de 0,30 à 0,75 mole de NaBH4. Le mélange est maintenu de 1 à 5 heures à une température comprise entre 15°C et la température de reflux du solvant, puis les complexes sont décomposés par addition d'eau éventuellement acidifiée et l'alcool est éliminé par distillation. Le produit de réduction est isolé puis purifié par des méthodes appropriées comme la cristallisation, la distillation fractionnée sous vide poussé ou encore la chromatographie préparative.

a.1.2) de préparer des amines secondaires -NH-R3 telles que (I.A.2) et (I.D.2), ou des amines tertiaires -N(R3)R4 telles que (I.A.3) et (I.D.3), par réduction d'intermédiaires carboxamides (V.A.2/3) ou (V.D.2/3) correspondants.

Les hydrures utilisés à cet effet correspondent à la formule générale définie et plus particulièrement à celles dans lesquelles M2 est l'aluminium. Leur mise en oeuvre étant réalisée dans des solvants aprotiques anhydres comme les solvants aromatiques et les éthers oxydes.

On préfère parmi ceux ci les hydrures (Hm) qui sont solubles dans les hydrocarbures aromatiques comme le benzène ou le toluène, dans lesquels Rx dont l'indice s égal à 2 représente notamment le radical méthoxy-2-éthoxy ; également à titre préféré on utilise dans des éthers oxydes comme l'éther diéthylique et le THF, l'hydrure d'aluminium AlH3 qui est préparé dans le milieu de réduction par la réaction de trois moles d'hydrure de lithium aluminium avec une mole de chlorure d'aluminium.

La réduction d'une fonction carboxamide nécessite de 3 à 6 moles d'agent réducteur à une température comprise entre 0°C et celle du reflux du solvant.

Toutefois, dans des conditions préférées, on utilise de 3,5 à 5 moles d'agent réducteur par mole de composé (V.A) ou (V.D) en maintenant une température comprise entre 0 et 25°C lorsqu'on utilise l'hydrure d'aluminium, et la température du reflux du benzène ou du toluène lorsqu'on utilise l'hydrure organo-métallique, la durée, quel que soit le réducteur, étant comprise entre 1 et 6 heures, l'état d'avancement étant par ailleurs surveillé par des chromatographies sur couches minces de façon périodique pour stopper la réaction au moment opportun par une addition prudente d'eau qui décompose les complexes formés et les réactifs en excès. Les traitements habituels permettent d'isoler puis de purifier les produits issus de la réduction.

a.2) par des hydrogènations catalysées par les éléments métalliques du groupe VIII de la classification périodique des éléments, sous leur forme divisée ou sous leurs formes oxydées ou leurs formes salifiées, la dispersion de ces différents catalyseurs pouvant être réalisée sur des supports inertes divers comme pour exemples le charbon activé, l'alumine, le sulfate ou le carbonate de baryum.

Parmi les éléments du groupe VIII le platine, le palladium ou encore le nickel, leurs sels et leurs oxydes sont préférés, et, notamment pour le nickel on préfère la forme d'alliage avec l'aluminium dans le nickel de Raney.

Ces hydrogènations permettent de préparer, par réduction de dérivés nitrés, des propanamines (I) à fonction amine primaire telles que (I.D.1) et (I.A.1) à partir des composés (III) et (IV)

La mise en oeuvre de l'hydrogénation consiste à dissoudre le dérivé nitré dans 1 à 30 parties (p/v) d'un alcool aliphatique inférieur comme le méthanol ou l'éthanol puis à ajouter le catalyseur convenablement préparé, à raison de 10 à 100 g par mole de composé à hydrogéner dans le cas du nickel de Raney, puis à agiter énergiquement la suspension sous une atmosphère d'hydrogène, à une pression comprise entre $10^6$ et $5.10^6$ Pa et à une température de 20 à 60°C, jusqu'à réduction complète du dérivé, ce qui est obtenu à la fin de l'absorption d'hydrogène et qui se traduit, à volume constant et pour une température constante, par une stabilisation de la pression.

Dans la plupart des cas, sous une pression d'hydrogène comprise entre $5.10^5$ et $10^6$ Pa et à une température voisine de 25°C la réaction est complète après 10 à 24 heures, suite à quoi le catalyseur est filtré, l'alcool évaporé et le produit issu de l'hydrogénation est isolé puis purifié par les méthodes habituelles.

b) - des réactions d'acylation pour préparer tel que décrit précédemment en B.2 des intermédiaires (V.D.2) ou (V.A.2) et en C.2.2 des intermédiaires (V.D.3) ou (V.A.3) qui consistent

b.1) lorsque le réactif d'acylation (R6-CO)pX2 est un anhydride (p = 2 ; X2 = O) la réaction, lorsque le point d'ébullition de l'anhydride est inférieur à 140°C peut s'effectuer sans solvant, en faisant réagir une propanamine (I.A.1) ou (I.D.1) dans un large excès et à la température de reflux du réactif. La méthode préférée consiste cependant à réaliser la réaction en utilisant comme solvant la pyridine et en faisant réagir pour une mole de composé à acyler, de 1 à 5 mole d'anhydride.

Couramment, l'utilisation de 1,2 à 1,8 mole d'anhydride au reflux de la pyridine durant 1 à 3 heures conduit à des résultats convenables. Les intermédiaires (V.A.2) ou (V.D.2) obtenus sont, si leur état le nécessite, purifiés par les méthodes habituelles, ou engagés tels quels dans les réactions de réductions qui suivent pour préparer les propanamines correspondantes comme il est décrit précédemment en a.2).

b.2) Lorsque les réactifs d'acylation (R6-CO)pX2 ou R9COX6 sont des halogénures d'acyle (X2 ou X6

= Cl ou Br) les propanamines (I.A.1), (I.D.1), (I.A.2) et (I.D.2) sont solubilisées dans 3 à 20 volumes (p/v) d'un solvant organique apolaire halogéné comme le dichlorométhane, puis on ajoute à la solution une base organique telle que la triéthylamine ou tout autre base organique forte de pK comparable et non réactive, puis l'halogènure d'acyle à une température comprise entre - 20°C et 20°C et plus favorablement entre 0 et 15°C. Le milieu réactionnel est ensuite maintenu vers 20°C de 1 à 24 h pour compléter la réaction, puis dilué à l'eau et traité de façon convenable, comme par des opérations d'extractions pour isoler puis purifier les carboxamides (V) obtenus et,

b.3) Lorsque le réactif (R6-CO)pX2 ou le réactif R9-COX6 sont des acides carboxyliques (X2 ou X6 = OH) le procédé d'acylation consiste à préparer d'abord, dans un éther oxyde comme le THF, une solution équimoléculaire de l'acide et d'une base organique comme la N-méthylmorpholine, puis d'y ajouter à une température comprise entre - 50 et 0°C et de préférence entre - 20 et - 10°C une quantité équimoléculaire aux réactifs précédents de chloroformiate d'isobutyle puis la propanamine à acyler.

On laisse la réaction se développer durant 12 à 48 heures à une température comprise entre 0 et 20°C puis isole et purifie le carboxamide (V) obtenu et,

b.4) plus particulièrement avec l'acide formique, l'acylation est réalisée en présence de 1,1′-carbonyl diimidazole dans un solvant anhydre et apolaire comme le THF.

Pratiquement, à un mélange équimoléculaire d'acide formique et de 1,1′-carbonyldiimidazole on ajoute, à une température voisine de 0°C la propanamine à traiter à raison de 0,6 à 0,95 mole pour 1 mole d'acide formique. Le mélange est maintenu de 1 à 12 heures à une température comprise entre 0 et 20°C pour compléter la réaction, puis on traite de façon habituelle pour isoler et purifier le carboxamide (V) obtenu et,

c) - des réactions de condensation qui consistent comme par exemple pour préparer un intermédiaire (IV) à partir d'un précurseur (II) ou encore une propanamine (I.D.3) à partir d'une propanamine (I.C.3), à faire réagir un réactif bifonctionnel HQ-(CH2)n-QH sur un composé dans lequel W représente =C=Q.

A titre d'exemple, pour obtenir un hétérocycle W qui est un groupe 1,3-dioxolane-2,2 diyle à partir d'un composé dans lequel W est =C=Q dans lequel Q est l'oxygène, on peut utiliser les différentes techniques comme celles décrites au chapitre "Protection for The Carbonyl Group" in "Protective Groups in Organic Synthesis" T.W. Greene - Ed. Wiley 1981, p. 115-151. Un mode opératoire préféré consiste à dissoudre le dérivé carbonylé dans 3 à 50 volumes (p/v) et couramment dans 5 à 20 volumes d'un solvant organique aromatique inerte aux réactifs mis en oeuvre tel que le benzène ou le toluène,et capable d'entraîner l'eau par distillation azéotropique puis d'ajouter de 2 à 40 moles d'éthylène glycol et un catalyseur qui est choisi parmi les acides forts minéraux ou organiques comme les acides chlorhydrique, sulfurique, méthanesulfonique ou p. toluènesulfonique. Le mélange est porté à la température de reflux de l'azéotrope duquel, après condensation, on élimine en continu l'eau formée au cours de la réaction par un système comme celui de "Dean Stark". La fin de l'élimination d'eau est caractéristique de la fin de la réaction, ce qui nécessite de 30 minutes à 24 heures de reflux.

Dans des conditions usuelles, on effectue la condensation en ajoutant pour une mole de composé carbonylé de 3 à 10 moles d'éthylène glycol et de 2 à 10 ml d'acide sulfurique concentré (d = 1.84). La réaction est complète après 10 à 16 heures de reflux après quoi le composé issu de la condensation est isolé et purifié.

d) - des réactions d'hydrolyse pour obtenir des propanamines (I.C) dans lesquelles W est un groupe carbonyle (Q = oxygène) à partir de propanamines (I.D) dans lesquelles W est un hétérocycle pour préparer une propanamine (I.C.2/O) à partir d'une propanamine (I.D.2) ou pour préparer une propanamine (I.C.3/O) à partir d'une propanamine (I.D.3).

Le procédé utilisé peut consister en l'application d'une méthode décrite par exemple dans "Protection for the Carbonyl Group" (Référence déjà citée) et qui consiste lorsque dans l'hétérocycle Q représente l'oxygène, à pratiquer une hydrolyse en solution dans un alcool inférieur ou dans un mélange hydro alcoolique par un acide fort, minéral ou organique et, lorsque Q est le soufre à pratiquer cette hydrolyse par une solution oxydante telles que celles de sels hydrosolubles de mercure (II),d'argent (I) ou de cuivre (II) ou encore par des composés d'oxydation organiques comme la Chloramine B ou T.

Pour illustration générale, la méthode préférée pour hydrolyser un groupe 1,3-dioxolane-2,2-diyle consiste à dissoudre le composé à hydrolyser dans un solvant organique miscible à l'eau et choisi par exemple dans le groupe des cétones aliphatiques inférieures, à y ajouter une solution aqueuse d'un acide fort, de préférence l'acide chlorhydrique, puis à porter le mélange au reflux jusqu'à réaction d'hydrolyse complète ce qui peut être déterminé par des chromatographies sur couches minces réalisées périodiquement durant la réaction. Concrètement, une mole de composé à traiter est solubilisée dans 3 à 5 litres d'acétone puis on ajoute un volume équivalent d'une solution environ N d'acide chlorhydrique et porte au reflux de 30 minutes à 5 heures, l'avancement de l'hydrolyse étant suivi par C.C.M., puis l'acétone est éliminé par distillation et le résidu traité de manière appropriée pour isoler et purifier la propanamine (I) dans laquelle W est carbonyle.

e) - des réactions d'alkylation

e.1) par des halogénures d'alkyle qui consistent :

e.1.1. en une monoalkylation avec un réactif R3-X1 pour préparer des propanamines (I.A.2) ou (I.D.2) et avec un réactif R4-X5 pour préparer des propanamines (I.D.3) et (I.C.3).

La réaction est réalisée dans des conditions adaptées à favoriser la mono-alkylation de l'amine, ce que l'on réalise dans des solvants inertes aux réactifs comme par exemple le toluène et l'acétonitrile, et en faisant réagit une mole de propanamine avec de 0,5 à 6,0 mole d'halogénure R3-X1 ou R4-X5.

D'une manière préférée, on utilise de 0,8 à 1,2 mole de dérivé où l'halogène est le brome ou l'iode et optionnellement on ajoute une base, organique ou minérale, pour favoriser la réaction qui consiste à chauffer le milieu réactionnel à une température comprise entre 20 et 110°C et ce durant de 2 à 5 h, les produits étant ensuite isolés et purifiés par les méthodes habituelles, notamment par chromatographie.

e.1.2 en une dialkylation d'une propanamine (I.D.1) par un dihalogénure X3-(CH2)q-X4 qui consiste à réaliser la réaction comme indiqué ci-dessus mais en utilisant de façon préférée de 1,2 à 2,0 mole de dérivé halogéné et en réalisant la réaction à une température comprise entre 60 et 110°C durant 4 à 24 heures pour obtenir une propanamine N-hétérocyclique (I.D.3).

e.2) par des aldéhydes R7-CH0 et des agents de réduction et qui consistent

e.2.1 en une dialkylation pour obtenir, à partir de propanamines (I.A.1) ou (I.D.1) des propanamines (I.A.3) ou (I.D.3) dans lesquelles R3 et R4 sont identiques et que l'on réalise en faisant réagir la propanamine à traiter avec de 2 à 5 mole et plus favorablement de 2,2 à 3 mole d'aldéhyde puis à faire réagir un agent de réduction qui peut être ou bien l'acide formique ou un de ses sels selon la réaction d'Eischweiler-Clarke (J. Am. Chem. Soc. 55, 4571, 1933) ou bien un hydrure d'un métal du groupe III de la classification périodique des éléments comme le bore et plus particulièrement parmi ceux ci le cyano borohydrure de sodium NaBH3CN en utilisant une adaptation de la méthode présentée dans J. Med. Chem. 25, n° 4, 446, (1982).

Pour exemple d'une N,N-diméthylation d'une propanamine (I.A.1) ou (I.D.1) par la réaction d'Eischweiler - Clarke, on mélange intimement dans un premier temps 1 mole d'amine primaire avec 2 à 5 mole et plus favorablement 2,2 à 3,0 mole de solution aqueuse de formaldéhyde à environ 37 % (p/v) puis on introduit entre 0 et 10°C 2 à 6 mole d'acide formique pur et, de préférence, de 2,5 à 3,3 mole puis on chauffe progressivement le mélange jusqu'à 100°C jusqu'à fin de dégagement gazeux ce qui nécessite de 15 minutes à 4 heures et, plus fréquemment de 45 minutes à 2 heures. On traite ensuite notamment par des extractions pour isoler la propanamine (I.A.3) ou (I.D.3) qui est purifiée par les méthodes usuelles.

e.2.2 en une monoalkylation pour préparer une propanamine (I.A.3) ou (I.D.3) par alkylation réductrice d'une propanamine correspondante (I.A.2) ou (I.D.2) et qui consiste à pratiquer une réaction telle que l'une de celles décrites ci-dessus en utilisant toutefois quantitativement la moitié des réactifs indiqués.

f) - des réactions de substitution de radicaux carbonitriles de précurseurs (VII.1) ou (VII.2) par des radicaux R1 ou R2 appartenant à des réactifs halogénométalliques pour préparer des propanamines (I.D.3) ou (I.A.3).

On préfère pratiquer selon une réaction décrite par N.J. Léonard et coll., J. Am. Chem. Soc., 1956, 78, p. 1986 et 1957, 79, p. 5279, dans laquelle la substitution du radical carbonitrile du composé (VII.1) ou (VII.2) par un radical R1 ou R2 est réalisée à l'aide d'un dérivé organo magnésien dans des éthers comme l'éther diéthylique, le méthyl-t.butyl ether, les ethers di-isopropylique ou dibutylique ou encore le tétrahydrofurane qui est le solvant préféré, et consiste pour une mole de précurseur à faire réagir 1,5 à 6 mole de dérivé organo magnésien à une température comprise entre 5 et 50°C et ce durant 30 minutes à 12 heures.

La méthode préférée consiste à ajouter à une température comprise entre 10 et 20°C, 1 mole de précurseur (VII.1) ou (VII.2) en solution dans le THF, à 4 à 5 mole du composé organomagnésien également en solution dans le THF. La réaction est poursuivie durant de 2 à 5 heures à la même température puis le complexe obtenu est décomposé par addition de solution aqueuse de chlorure d'ammonium. Après traitement, la propanamine (I.D.3) ou (I.A.3) obtenue est isolée et purifiée par les méthodes déjà citées.

g) - des réactions d'oxydation, pour obtenir des propanamines dans lesquelles W est un carbonyle =C=O à partir de propanamines correspondantes dans lesquelles W est hydroxyméthylène =CH-OH, telles que pour obtenir (I.C.2/O) à partir d'une propanamine hydroxylée (I.A.2) ou pour obtenir (I.C.3/O) à partir d'un dérivé hydroxylé (I.A.3).

La réaction peut être réalisée à l'aide de divers réactifs aptes à oxyder une fonction alcool secondaire en fonction cétone et qui sont recapitulés par exemple dans "Advanced Organic Chemistry" - J. March - 3ème Ed. Wiley, p. 1057-1060. On utilise pour ces réactions des systèmes oxydo réducteurs ioniques mono ou polyatomiques dont le potentiel normal E° à 20°C est supérieur à 0,60 volts.

Et, plus particulièrement, on utilise les ions polyatomiques dérivés d'oxydation du chrome, du manganèse ou de l'azote dont le potentiel E° est voisin ou supérieur à 1 volt comme l'ion permanganate MnO4- ou l'ion dichromate Cr2O7-- qui est préféré et que l'on obtient à partir de dichromate de potassium ou de sodium en présence d'acide sulfurique.

La réaction d'oxydation couramment pratiquée consiste à préparer une solution contenant de 0,3 à 0,5 mole de dichromate avec 0,6 à 1,0 mole d'acide sulfurique puis d'y ajouter 0,6 mole d'hydroxypropanamine à oxyder.La température est maintenue entre 40 et 60°C durant 2 à 6 heures puis le mélange est traité notamment par des extractions en milieu alcalin pour obtenir le composé résultant de l'oxydation qui est isolé et purifié par les méthodes habituelles.

h) - des réactions de sulfuration pour obtenir des propanamines (I) dans lesquelles W représente un groupe C=S à partir de propanamines (I) correspondantes dans lesquelles W représente un groupe C=O comme par exemples pour obtenir un composé (I.C.2/S) ou un composé (I.C.3/S).

On préfère la réaction qui consiste à préparer dans un premier temps un intermédiaire avec un réactif Rz-NH2 dans lequel plus particulièrement Rz est Rz'-NH-dans lequel Rz' est l'hydrogène, alkyle inférieur ou phényle pour obtenir une hydrazone correspondante intermédiaire (VI.1) ou (VI.2) qui en solution dans le benzène est traité par le monochlorure de soufre pour obtenir une propanamine (I.C.2/S) ou bien (I.C.3/S) précédemment présentées.

La mise en oeuvre de ces réactions est illustrée par la description dans la partie expérimentale des produits (I) de l'invention et de leurs intermédiaires.

Sauf mention particulière, dans ces exemples, les procédures d'isolement et de purification font appel aux méthodes habituelles telles que la cristallisation, la distillation fractionnée (notamment sous vide poussé) et des méthodes de chromatographies préparatives comme la technique de chromatographie rapide effectuée sur gel de silice (produit Merck réf. 4063) selon Still W.C. et coll., J. Org. Chem. 43, 2923, 1978 ou la technique de chromatographie sur colonne de silice obtenue après compression axiale avec un appareil de marque Jobin-Yvon.

Les déterminations de pureté et d'identité des produits sont effectuées par les méthodes habituelles qui sont rappelées brièvement :

- détermination du point de fusion par la méthode au tube capillaire et dont la valeur obtenue n'est pas corrigée,
- détermination de pureté par chromatographies sur couches minces de silice 60 F 254 de 0,25 mm d'épaisseur (produit Merck Réf. 5714), les chromatogrammes étant examinés après développement sous lumière ultra violette de 254 nm et/ou après pulvérisation de réactif de Dragendorff ou de réactif à la toluidine,
- détermination d'identité par :

  i) spectre de résonnance magnétique nucléaire du proton, réalisée avec un appareil JEOL FX-90 P (90 MHz) le tétraméthylsilane (TMS) étant utilisé comme référence interne ; les spectres des composés étant décrits, dans les exemples, par le déplacement chimique des signaux (en p.p.m. par rapport au TMS), leur multiplicité, leur intégration et le cas échéant leur échange après addition d'oxyde de deutérium étant signalé,

  ii) spectrographie infra rouge des composés en pastilles de KBr ou en films ou encore en solution dans le CCl4 ou bien en suspension dans le Nujol (R) sur un appareil Schimadzu IR-435. Les absorbtions les plus importantes et caractéristiques sont reportées dans les exemples par leur longueur d'onde en cm-1.

Les analyses élémentaires, notamment celles des composés (I) ont été effectuées. Sauf exceptions (solvants par exemple) leurs résultats, conformes aux normes admises ne sont pas reportés, toutefois les éléments analysés sont mentionnés. Enfin, des abréviations couramment utilisées (par exemple THF pour tétrahydrofurane) peuvent être employées dans la description de ces exemples.

En ce qui concerne les CCM les solvants d'élution utilisés sont indiqués dans la partie descriptive des composés de l'invention par leur sigle S.(solvant) indicatif des mélanges indiqués ci dessous et dans lesquels les proportions indiquées sont en v/v.

| | | |
|---|---|---|
| A.1 | - acétate d'éthyle | |
| A.2 | - " " / éthanol | 3/1 |
| B. | - benzène/éthanol | 85/15 |
| C.1 | - chlorure de méthylène | |
| C.2 | - " " /méthanol | 19/1 |
| H.1 | - hexane/acétate d'éthyle | 1/1 |
| H.2 | - " " " | 1,5/1 |
| H.3 | - " " " | 2/1 |
| H.4 | - " " " | 3/1 |
| H.5 | - " " " | 4/1 |
| H.6 | - " " " | 6/1 |
| H.7 | - " /chlorure de méthylène | 7/3 |

PREPARATION DES COMPOSES INTERMEDIAIRES INTERMEDIAIRES IX - IX.a : X9 = (CH3)2N- ; W = CO

Intermédiaire IX.a.1 : 3-diméthylamino-1-(3,4,5-triméthoxyphényl)-propan-1-one

[IX.a ; X9 = (CH3)2N-, W = CO, R5 = 3,4,5(CH3O)3-C6H2]

Dans un réacteur, on introduit 55 ml d'éthanol, 9,3 g (0,31 mol) de paraformaldéhyde, 50,0 g (0,24 mol) de 3,4,5-triméthoxy acétophénone, 25,2 g (0,31 mol) de chlorhydrate de diméthylamine et 1,0 ml d'acide chlorhydrique concentré (d = 1,18). Le mélange est chauffé et porté au reflux deux heures, puis après refroidissement on ajoute 400 ml d'acétone et chauffe à nouveau au reflux durant 25 minutes.

Le milieu est alors refroidi et maintenu deux heures en glaciaire.

Le chlorhydrate cristallisé est filtré puis séché sous vide.

Poids = 41,0 g Rdt = 57 % F = 175°C

Pour obtenir le produit sous forme de base le composé précédent est traité par un mélange de solution saturée en carbonate de sodium et de dichlorométhane. La phase organique est séparée puis la phase aqueuse réextraite au dichlorométhane. Les phases organiques réunies sont lavées à l'eau puis séchées sur Na2SO4.

La solution concentrée permet d'obtenir un produit résiduel huileux de couleur orange (34,2 g) qui est purifié par recristallisation dans l'hexane.

Poids 31,0 g. Rdt = 49 % F = 47°C

Intermédiaire IX.a.2 : 3-diméthylamino-1-(3,4-dichloro phényl)-propan-1-one

[IX.a ; X9 = (CH3)2N-, W = CO, R5 = 3,4(Cl)2-C6H3]

Selon un procédé semblable à l'exemple précédent et à partir de 3,4-dichloroacétophénone on obtient le produit purifié par cristallisation. F = 51°C.

Intermédiaire IX.b.1 : 2-méthylsulfonyloxyéthyl-2-phényl-1,3-dioxolane

[IX.b , X10 = CH3-SO3-, W = C(OCH2CH2O), R5 = C6H5]

i) un mélange de 185,6 g (0,97 mol) de β-oxobenzène propanoate d'éthyle, 350 ml (6,3 mol) d'éthylène glycol, 1,9 ml d'acide sulfurique concentré (d = 1,88) dans 1,1 l. de benzène est porté au reflux sous agitation en éliminant l'eau formée par un système séparateur de Dean Stark.

Après 24 heures, on ajoute à nouveau 2,0 ml d'acide et porte encore au reflux durant 22 heures. On refroidit, ajoute une solution diluée de bicarbonate de sodium. La phase organique est séparée, lavée à l'eau, séchée sur Na2SO4 puis concentrée sous vide. On obtient un résidu huileux de 2-éthyloxycarbonylméthyl-2-phényl-1,3-dioxolane.

Poids = 199,0 g Rdt = 87 %

ii) Dans un réacteur anhydre et sous atmosphère d'azote on introduit dans 600 ml d'éther déshydraté sur

tamis moléculaire, 16,04 g (0,425 mol) d'hydrure de lithium-aluminium.

On ajoute ensuite 98,1 g (0,415 mol) de l'ester i) précédent en solution dans 20 ml d'éther en une période de 30 minutes environ. Le mélange est agité 30 minutes à température du laboratoire puis trois heures au reflux après quoi il est refoidi et on y ajoute au goutte à goutte 25,5 ml d'une solution d'hydroxyde de sodium à 10 % p/v. On ajoute ensuite 250 ml d'éther et 32 ml d'eau puis le mélange, après agitation durant 16 heures, est filtré et concentré sous vide pour obtenir le 2-hydroxyéthyl-2-phényl-1,3-dioxolane brut sous forme d'une huile incolore.

Poids = 73,7 g Rdt = 91 %

iii) Dans 250 ml de dichlorométhane anhydre on introduit 15,6 g (80 mmol) du dérivé préparé au stade ii) précédent et 17,0 ml de triéthylamine. Sous atmosphère d'azote et à -10°C on ajoute goutte à goutte 6,85 ml (88,5 mmol) de chlorure de méthanesulfonyle. Après agitation 15 minutes à la même température le mélange est extrait successivement par l'eau, une solution diluée de bicarbonate de sodium, une solution d'acide chlorhydrique puis finalement est déshydraté sur Na2SO4. Le solvant est éliminé par distillation sous vide et le résidu est cristallisé dans un mélange hexanes/acétate d'éthyle. Après filtration et séchage on obtient 20,6 g de produit.

Rdt = 94 % F = 50°C

CCM : 0,35 - 0,40 ; S. H.3

Intermédiaire IX.b.2 : <u>2-méthyloxysulfonyléthyl-2-(3,4,5-triméthoxyphényl)-1,3-dioxolane</u>

[IX.b ;X10 =CH3-SO3-, W = C(OCH2CH2O), R5 = 3,4,5 (CH3O)3-C6H2]

Le composé est préparé comme il est décrit au mode opératoire précédent IX.b.1. à partir du 3,4,5-triméthoxy-β-oxo benzènepropanoate de méthyle (préparé selon R.E Strube "Org. Synthesis", coll. vol. IV, Wiley, New York, 1963, p. 417) et en obtenant successivement les composés suivants :

i) 2-acétyloxyéthyl-2-(3,4,5-triméthoxyphényl) -1,3-dioxolane.

Rdt = 78 % ; F = 76°C (hexanes) ; CCM : 0,30 ; S. H.2

ii) 2-hydroxyéthyl-2-(3,4,5-triméthoxyphényl) -1,3-dioxolane.

Rdt = 65 % ; F = 68°C (hexanes) ; CCM : 0,30 ; S. H.1

iii) composé intermédiaire IX.b.2 Rdt = 57 % ; F = 94°C (hexanes) ; CCM : 0,45 ; S. H.1

Intermédiaire IX.b.3 : <u>2-bromoéthyl-2-cyclohexyl-1,3-dioxolane</u>

[IX.b , X10 = Br, W = C(OCH2CH2O), R5 = CH(CH2)5]

Selon le procédé décrit pour les composés IX.b on prépare le 2-méthylsulfonyloxyéthyl-2-cyclohexyl-1,3-dioxolane. On mélange 19,5 g (70 mmol) du produit avec 45,1 g (140 mmol) de bromure de tétrabutyl-ammonium, 15,0 ml (140 mmol) de 2,2'- diméthyl-1,3- dioxolane dans 250 ml de 1-bromopropane. Le mélange est porté au reflux 14 heures puis refroidi, concentré sous vide et le résidu est additionné d'eau, d'éther et d'hexanes. La phase aqueuse est décantée et extraite à nouveau par un mélange hexanes/éther. Les phases organiques sont réunies, lavées à l'eau saturée par du bicarbonate de sodium puis séchées sur Na2SO4. Le solvant est éliminé et le composé est obtenu sous la forme d'un liquide incolore. Poids = 17,9 g ; Rdt = 97 %

<u>INTERMEDIAIRES VIII</u>

Mode opératoire général :

i.1) Dans un réacteur sous atmosphère d'azote on introduit 50 ml d'éther déshydraté sur tamis moléculaire puis 1,9 g (50 mmol) d'hydrure de lithium aluminium. La suspension est refroidie vers 5°C puis on ajoute en solution dans 50 ml d'éther 100 mmol d'une cétone R1-CO-R2 en 30 minutes environ et à une température comprise entre 0 et 5°C. La suspension est maintenue 30 minutes à la température ambiante puis on ajoute avec précautions 30 ml d'une solution à 10% (p/v) d'hydroxyde de sodium puis 10ml d'eau. L'insoluble est filtré et le filtrat évaporé. L'alcool brut résiduel R1-CHOH-R2 est purifié par distillation sous vide poussé.

i.2) Ou bien, dans un réacteur anhydre et sous atmosphère d'azote on introduit 50 ml (100 mmol) d'une solution dans le tétrahydrofurane d'un dérivé organo magnésien R2-MgBr. Après refroidissement à une température comprise entre 0 et 5°C on introduit 100 mmol d'aldéhyde R1-CHO en solution dans 30 ml de tétrahydrofurane, en une heure et à une température inférieure à 10°C. Le mélange est agité 30 minutes puis précipité dans 125 ml d'eau glacée. On ajoute ensuite 75 ml de solution d'acide sulfurique à 15 % (p/p) pour dissoudre le précipité puis on extrait à l'éther. Les phases organiques sont lavées, séchées sur Na2SO4 puis concentrées sous vide. L'alcool R1-CHOH-R2 est purifié par distillation sous vide poussé.

ii) Dans un réacteur approprié on introduit 50 ml de toluène et 100 mmol d'alcool R1-CHOH-R2. On ajoute ensuite en 30 minutes environ et sans dépasser 35°C 36 mmol de tribromure de phosphore.

Le mélange est chauffé au reflux 45 minutes puis refroidi. La phase toluènique est lavée par une solution saturée en bicarbonate de sodium puis par une solution saturée en NaCl et enfin séchée sur Na2SO4. Les solvants sont évaporés sous vide puis le résidu bromé résiduel R1-CH(Br)-R2 est purifié par distillation sous vide poussé.

iii) Dans un réacteur on ajoute 200 mmol de nitrite de sodium à 75 ml de diméthylsulfoxyde. On agite et ajoute sans dépasser 10°C 100 mmol de dérivé bromé préparé en ii).

Le mélange est agité 2 heures 30 puis précipité dans 200 ml d'eau glacée; il est ensuite extrait à plusieurs reprises par l'hexane.

Les phases organiques réunies sont séchées sur Na2SO4 puis le solvant est éliminé par distillation sous vide. Le dérivé nitré R1-CH(NO2)-R2 est purifié par distillation sous vide poussé, en prenant des précautions contre tout risque de décomposition brutale.

Les composés intermédiaires VIII.1 à VIII.5 décrits ci dessous sont préparés selon ce protocole.

Intermédiaire VIII.1 : 1-nitro-propylbenzène.

[VIII ; R1 = C6H5, R2 = C2H5]
Préparé à partir de 1-phényl-propan-1-ol
ii) 1-bromo-propylbenzène : Rdt = 90% ; Eb/2600Pa = 101-110°C
iii)1-nitro-propylbenzène : Rdt = 66% ; Eb/65Pa = 68 - 78°C

Intermédiaire VIII.2: 1-(4-chlorophényl)-1-nitro-propane

[VIII ; R1 = p.Cl-C6H4, R2 = C2H5]
Préparé à partir de 4-chloropropiophénone.
i.1) 1-(4-chlorophényl)-propan-1-ol
Rdt = 82 % Eb/65Pa = 92°C
ii) 1-(4-chlorophényl)-1-bromo-propane
Rdt = 81 % Eb/20Pa = 70°C
iii) 1-(4-chlorophényl)-1-nitro-propane
Rdt = 50 % ; Eb/40Pa = 95 - 115°C ; CCM : 0,85 ; S. C.1

Intermédiaire VIII.3: 1-(4-méthylphényl)-1-nitro-propane

[VIII , R1 = p.CH3-C6H4, R2 = C2H5]

Préparé à partir de 4-méthylpropiophénone.
i.1) 1-(4-méthylphényl)-propan-1-ol
Rdt = 91 % Eb/7Pa = 70 - 85°C
ii) 1-(4-méthylphényl)-1-bromo-propane
Rdt = 90 % Eb/13Pa = 60 - 65°C
iii) 1-(4-méthylphényl)-1-nitro-propane
Rdt = 62 % ; Eb/25Pa = 76 - 81°C ; CCM : 0,80 ; S. C.1

Intermédiaire VIII.4 : 1-(4-trifluorométhylphényl)-1-nitro-propane.

[VIII ; R1 = p.F3C-C6H4, R2 = C2H5]
Préparé à partir de 4-trifluorométhylbenzaldéhyde.
i.2) 1-(4-trifluorométhylphényl)-propan-1-ol
Rdt = 92 % Eb/60Pa = 60 - 65°C
ii) 1-(4-trifluorométhylphényl)-1-bromo-propane
Rdt = 69 % Eb/130Pa = 57 - 70°C
iii) 1-(4-trifluorométhylphényl)-1-nitro-propane
Rdt = 45 % (chromatographie) ; CCM : 0,50 ; S. H.7

Intermédiaire VIII.5 : 1-(3,4-dichlorophényl)-1-nitropropane.

[VIII ; R1 = 3,4(Cl)2-C6H4, R2 = C2H5]
Préparé à partir de 3,4-dichlorobenzaldéhyde.
i.2) 1-(3,4-dichlorophényl)-propan-1-ol
Rdt = 93 % Eb/25 Pa = 110°C
ii) 1-(3,4-dichlorophényl)-1-bromo-propane
Rdt = 89 % Eb/25Pa = 88 - 98°C
iii) 1-(3,4-dichlorophényl)-1-nitro-propane
Rdt = 55 % (chromatographie) ; CCM : 0,90 ; S. C.1

INTERMEDIAIRE X : α-diméthylamino-phénylacétonitrile.

[R1 = C6H5, R3 = R4 = CH3]

Une solution de 20,3 ml (200 mmol) de benzaldéhyde dans 20 ml de méthanol est ajoutée goutte à goutte dans une solution de 11,8 g (240 mmol) de cyanure de sodium et de 19,6 g (240 mmol) de chlorhydrate de diméthylamine dans 40 ml d'eau et en une durée de 75 minutes à une température de 30 à 40°C. L'agitation est poursuivie 4 heures à 30°C; on ajoute 150 ml d'eau et extrait par 4 fois 200 ml d'éther, les phases éthérées réunies sont lavées à l'eau puis par une solution à 25 % (p/v) de bisulfite de sodium et à nouveau lavées à l'eau. Après séchage sur Na2SO4, l'éther est éliminé par distillation et le résidu purifié par distillation fractionnée.

Poids = 30,4 g Rdt = 95 % Eb/100Pa = 75°C

INTERMEDIAIRES VII

Mode opératoire général : Dans un réacteur protégé de l'humidité et maintenu sous atmosphère d'azote on introduit goutte à goutte à - 10°C 100 mmol de n. butyl lithium en solution 2 M dans l'hexane dans une solution de 105 mmol de diisopropylamine dans 100 ml de tétrahydrofurane. La solution est maintenue 15 minutes à - 10°C. On refroidit à - 78°C puis ajoute 100 mmol d'intermédiaire amino-nitrile (X) en solution dans 100 ml de THF, goutte à goutte et en 15 minutes à -78°C. La solution est ensuite agitée 2 heures à cette température puis on ajoute 105 mmol d'intermédiaire IX.b (X10 = halogène, W = =C(O-CH2-CH2-O)], laisse agiter 15 minutes à - 78°C puis une heure et demie à température ambiante. On ajoute alors 300 ml d'une solution à 10 % (p/v) de chlorure d'ammonium et extrait le mélange à l'éther.

Les phases éthérées réunies sont lavées et séchées sur Na2SO4. L'éther est évaporé et le produit résiduel brut dont la pureté est appréciée par CCM est utilisé tel quel dans la réaction qui suit.

Les composés (VII).2 a, b et c sont préparés selon ce protocole par réaction de l'intermédiaire (X) avec les agents d'alkylation décrits aux intermédiaires (IX.b).

Intermédiaire VII.2.a : 2-(1-cyano-1)-N,N-diméthylamino-1-phényl-prop-3-yl)-2-phényl-1,3-dioxolane.

[VII.2 ; R1 = R5 = C6H5, R3 = R4 = CH3]
Préparé à partir des intermédiaires X.2 et IX.b.1
Rdt = 84 % CCM : 0,50 ; S. H.1

Intermédiaire VII.2.b : 2-(1-cyano-1-N,N-diméthylamino-1-phényl-prop-3-yl)-2-(3,4,5-triméthoxyphényl)-1,3-dioxolane.

[VII.2 ; R1 = C6H5, R3 = R4 = CH3, R5 = (CH3O)3-C6H2]
Préparé à partir des intermédiaires X.2 et IX.b.2
Rdt = 57 % CCM : 0,60 ; S. H.3

Intermédiaire VII.2.c : 2-(1-cyano-1-N,N-diméthylamino-1-phényl-prop-3-yl)-2-cyclohexyl-1,3-dioxolane.

[VII.2 ; R1 = C6H5, R3 = R4 = CH3, R5 = CH(CH2)5]
Préparé à partir des intermédiaires X.2 et IX.b.3 Rdt = 74 % CCM : 0,20 ; S. H.6

INTERMEDIAIRES (II)

Mode opératoire général : Dans un réacteur anhydre et sous atmosphère d'azote on ajoute dans 80 ml de méthanol 4,83 g (209 mmol) de sodium. La température du milieu progresse jusqu'à 70°C. Après dissolution on refroidit à 0°C puis introduit goutte à goutte en 15 minutes environ une solution de 100 mmol d'un intermédiaire nitré (VIII) en solution dans 20 ml de méthanol. L'agitation est maintenue 15 minutes puis on introduit 100 mmol d'une cétone (XI.a) en solution dans 50 ml de méthanol.

Le mélange est agité 15 minutes puis on y ajoute 10,05 ml (160 mmol) d'iodure de méthyle. Le mélange est à nouveau agité 30 minutes à 0°C puis une nuit à température ambiante.

Le précipité est filtré et recristallisé dans l'isopropanol pour purification.

Les intermédiaires II.a à II.g décrits ci-dessous sont préparés selon ce mode opératoire appliqué aux intermédiaires (VIII) et (IX.a) décrits précédemment.

Intermédiaire II.a : 1-(3,4-dichlorophényl)-4-nitro-4-phényl-hexan-1-one.

[II ; R1 = C6H5, R2 = C2H5, R5 = 3,4(Cl)2-C6H3]
Préparé à partir de VIII.1 et de IX.a.2
Rdt = 57 %   F = 130°C (hexanes - acét. éthyle)
CCM : 0,50 ; S. H.4

Intermédiaire II.b : 1,4-diphényl-4-nitro-hexan-1-one.

[II ; R1 = R5 = C6H5, R2 = C2H5]
Préparé à partir de VIII.1 et de chlorhydrate de 3-diméthylamino propiophénone.
Rdt = 65 %   F = 96°C (hexanes - acét. éthyle)
CCM : 0,40 ; S. H.3

Intermédiaire II.c : 4-nitro-4-phényl-1-(3,4,5-triméthoxyphényl)-hexan-1-one.

[II ; R1 = C6H5, R2 = C2H5, R5 = 3,4,5(CH3O)3-C6H2]
Préparé à partir de VIII.1 et de IX.a.1
Rdt = 47 %   F = 86°C (méthanol)
CCM : 0,50 ; S. H.3

Intermédiaire II.d : 4-(4-chlorophényl)-4-nitro-1-(3,4,5-triméthoxyphényl)-hexan-1-one.

[II ; R1 = p.Cl-C6H4, R2 = C2H5, R5 = 3,4,5(CH3O)3-C6H2]
Préparé à partir de VIII.2 et de IX.a.1
Rdt = 67 %   F = 80°C (isopropanol)
CCM : 0,30 ; S. C.1

Intermédiaire II.e : 4-nitro-4-(4-méthylphényl)-1-(3,4,5-triméthoxyphényl)-hexan-1-one.

[II ; R1 = p.CH3-C6H4, R2 = C2H5, R5 = 3,4,5(CH3O) 3-C6H2] Préparé à partir de VIII.3 et de IX.a.1
Rdt = 61 %   F = 63°C (isopropanol)
CCM : 0,25 ; S. C.1

Intermédiaire II.f : 4-nitro-4-(4-trifluorométhylphényl) - 1-(3,4,5-triméthoxyphényl)-hexan-1-one.

[II ; R1 = p.F3C-C6H4, R2 = C2H5, R5 = 3,4,5(CH3O)3-C6H2]
Préparé à partir de VIII.4 et de IX.a.1
Rdt = 82 %   F = 130°C (isopropanol)
CCM : 0,40 ; S. C.1

Intermédiaire II.g : 4-(3,4-dichlorophényl)-4-nitro-1-(3,4,5-triméthoxyphényl)-hexan-1-one.

[II ; R1 = 3,4(Cl)2-C6H3, R2 = C2H5, R5 = 3,4,5(CH3O)3-C6H2]
Préparé à partir de VIII.5 et de IX.a.1

Rdt = 77 % F = 127°C ( isopropanol)
CCM : 0,30 ; S. C.1

PREPARATION DES COMPOSES DE L'INVENTION : EXEMPLES

EXEMPLES 1

Mode opératoire général :

- stade a : (préparation de l'intermédiaire nitro-dioxolane IV à partir de nitro-cétone II).

Dans un réacteur équipé avec un système d'agitation et un réfrigérant en position de reflux muni d'un système de séparation d'eau de type "Dean Stark" on introduit 100 mmol d'une nitro-cétone (II), 350 ml de benzène et 34 ml (450 mmol) d'éthylène glycol. Le mélange hétérogène est agité, on y ajoute 0,7 ml d'acide sulfurique concentré (95/97 % ; d = 1,84) puis chauffe et maintient au reflux 16 heures sous agitation afin d'éliminer au fur et à mesure l'eau formée par la réaction.

Après avoir été refroidi à la température ambiante le mélange est précipité dans 300 ml d'une solution saturée en bicarbonate de sodium. La phase aqueuse est séparée et extraite par deux fois 150 ml d'éther. Les phases organiques réunies sont lavées à l'eau et déshydratées sur $Na_2SO_4$. Les solvants sont éliminés par distillation sous pression réduite et le résidu est purifié si nécessaire par une méthode appropriée

- stade b (composé de l'invention I.D.1 à partir d'intermédiaires IV).

Dans un réacteur étanche éprouvé à une pression d'environ $1,2.10^6$ Pa et équipé d'un système d'agitation énergique on introduit 100 mmol d'un intermédiaire nitro-dioxolane (IV) et 350 ml d'éthanol. Sous atmosphère d'azote on ajoute 7,0 +/- 1,0 g de nickel de Raney activé (type W 2) en suspension dans environ 50 ml d'éthanol.

Le réacteur est hermétiquement fermé puis il est procédé à une succession de purges qui consiste à introduire alternativement à trois reprises et sans agiter de l'azote puis de l'hydrogène sous une pression de $5.10^5$Pa environ. Cette procédure terminée on introduit de l'hydrogène sous une pression de $8.10^5$ Pa et on agite violemment la suspension durant 16 heures, période après laquelle la pression interne est stable et l'hydrogénation est terminée.

Le catalyseur est filtré sur un buchner muni d'un filtre et garni d'un lit de terre d'infusoires et le filtrat est concentré sous pression réduite.

Le résidu est éventuellement purifié par les méthodes habituelles à l'exclusion toutefois des traitements aqueux en milieu acide ou alcalin.

Les composés I.D.1 de l'invention des exemples 1.a à 1.g sont préparés selon ce protocole à partir des nitro cétones II.a à II.g précédemment décrites.

Exemple 1.a : 2-(3-amino-3-phényl-pentan-1-yl)-2-(3,4-dichlorophényl)-1,3-dioxolane.

[I ; R1 = C6H5, R2 = C2H5, R3 = R4 = H, R5 = 3,4(Cl)2-C6H3, W = C(O-CH2-CH2-O)]

a) Préparé à partir de la nitro cétone II.a le produit brut (Rdt = 75%) est engagé tel quel dans le stade suivant.

b) Rdt = 73% brut (huile) F = 63°C (hexanes)
CCM : 0,60 - 0,80 ; S. H.3
RMN : 0,60 (t,3H), 1,25-1,95 (m,8H), 3,55-4,05 (m,4H), 7,10-7,50(m,8H).
IR (KBr) : 3360, 3295, 3070, 3050, 3005, 2950, 2930, 2885, 1590, 1482, 1470, 1452, 1372, 1340, 1280, 1245, 1200, 1135, 1120, 1040, 1020, 1000, 980, 960, 932, 910, 900, 885, 850, 825, 810, 760, 735, 695, 665 cm-1.
- Chlorhydrate :
Rdt = 68 % F = 225°C (éthanol)
Anal.(C20H23Cl2NO2.HCl) C, H, Cl, N, O

Exemple 1.b : 2-(3-amino-3-phényl-pentan-1-yl)-2-phényl-1,3-dioxolane.

[I ; R1 = R5 = C6H5, R2 = C2H5, R3 = R4 = H, W = C(O-CH2-CH2-O)]

a) Préparé à partir de la nitro cétone II.b
Rdt = 79 % F = 77°C (hexanes)
CCM : 0,55 ; S. H.5

b) Rdt = 68 % F = 64°C (hexanes)
CCM : 0,25 ; S. H.5
Anal.($C_{20}H_{25}NO_2$) C, H, N, O
RMN : 0,60 (t,3H), 1,25-1,95 (m,8H), 3,55-4,10 (m,4H), 7,00-7,60 (m,10H)

Exemple 1.c : <u>2-(3-amino-3-phényl-pentan-1-yl)-2-(3,4,5-triméthoxyphényl)-1,3-dioxolane.</u>

[I ; R1 = C6H5, R2 = C2H5, R3 = R4 = H, R5 = 3,4,5 (CH3O)3-C6H2, W = C(O-CH2-CH2-O)]
a) Préparé à partir de la nitro cétone II.c
Rdt = 83 % F = 112°C (hexanes)
CCM : 0,45 ; S. H.3
b) Rdt = 58 % F = 87°C (hexanes - éther)
CCM : 0,15 ; S. H.5
Anal.($C_{23}H_{31}NO_5$) C, H, N, O
RMN : 0,60 (t, 3H), 1,25-1,95 (m,8H), 2,30 (s,9H), 3,55-4,10 (m,4H), 7,00-7,60 (m,7H)

Exemple 1.d : <u>2-[3-amino-3-(4-chlorophényl)-pentan-1-yl]-2-(3,4,5-triméthoxyphényl)-1,3-dioxolane.</u>

[I ; R1 = p.Cl-C6H4, R2 = C2H5, R3 = R4 = H, R5 = 3,4,5 (CH3O)3-C6H2, W = C(O-CH2-CH2-O)]
a) Préparé à partir de la nitro cétone II.d
Rdt = 95 % F = 67°C (hexanes)
CCM : 0,55 , S. H.3
b) Rdt = 35 % chromatographie F = 87°C (hexanes)
CCM : 0,30 - 0,40 ; S. C.2
Anal.($C_{23}H_{30}ClNO_5$) C, H, Cl, N, O
RMN : 0,70 (t, 3H), 1,40 (s, 2H), 1,40-2,00 (m,6H), 3,75- 4,15 (m,13H), 6,60 (s,2H), 7,25 (s,4H)

Exemple 1.e : <u>2-[3-amino-3-(4-méthylphényl)-pentan-1-yl]-2-(3,4,5-triméthoxyphényl)-1,3-dioxolane.</u>

[I ; R1 = p.CH3-C6H4, R2 = C2H5, R3 = R4 = H, R5 = 3,4,5 (CH3O)3-C6H2, W = C(O-CH2-CH2-O)]
a) Préparé à partir de la nitro cétone II.e
Rdt = 91% F = 58°C (hexanes)
CCM : 0,60 ; S. H.3
b) Rdt = 35% chromatographie (huile)
CCM : 0,10 - 0,25 ; S. C.2
Anal.($C_{24}H_{33}NO_5$) C, H, N, O
RMN : 0,70 (t, 3H), 1,50 (s, 2H), 1,45-2,00 (m,6H), 2,30 (s,3H), 3,55-4,05 (m,13H), 6,55 (s,2H), 6,95-7,30 (m,4H)

Exemple 1.f : <u>2-[3-amino-3-(4-trifluorométhylphényl)-pentan-1-yl]-2-(3,4,5-triméthoxyphényl)-1,3-dioxolane.</u>

[I ; R1 = p.F3C-C6H4, R2 = C2H5, R3 = R4 = H, R5 = 3,4,5 (CH3O)3-C6H2, W = C(O-CH2-CH2-O)]
a) Préparé à partir de la nitro cétone II.f
Rdt = 89% F = 75°C (hexanes)
CCM : 0,35 ; S. C.1
b) Rdt = 95% (huile)
CCM : 0,55 ; S. C.2
Anal.($C_{24}H_{30}F_3NO_5$) C, H, F, N, O
RMN : 0,70 (t, 3H), 1,55 (s, 2H), 1,60-1,95 (m,6H), 3,55- 4,25 (m,13H), 6,60 (s,2H), 7,30-7,65 (m,4H)

Exemple 1.g : <u>2-[3-amino-3-(3,4-dichlorophényl)-pentan-1-yl]-2-(3,4,5-triméthoxyphényl)-1,3-dioxolane.</u>

[I ; R1 = 3,4(Cl)2-C6H3, R2 = C2H5, R3 = R4 = H, R5 = 3,4,5(CH3O)3-C6H2, W = C(O-CH2-CH2-O)]
a) Préparé à partir de la nitro cétone II.g
Rdt = 82 % F = 59°C (hexanes)
CCM : 0,25 ; S. C.1
b) Rdt = 92 % (huile)
CCM : 0,40 ; S. C.2

Anal.($C_{23}H_{29}Cl_2NO_5$) C, H, Cl, N, O
RMN : 0,70 (t, 3H), 1,35-2,00 (m, 8H), 3,60-4,10 (m,13H), 6,60 (s,2H), 7,00-7,50 (m,3H)

Exemple 2 : 4-amino-4-phényl-1-(3,4,5-triméthoxyphényl)-hexan-1-ol.

[I ; R1 = $C_6H_5$, R2 = $C_2H_5$, R3 = R4 = H, R5 = 3,4,5 $(CH_3O)_3$-$C_6H_2$, W = CHOH]

Dans un réacteur anhydre et sous atmosphère d'azote on introduit une solution toluènique contenant 200 mmol de bis (2-méthoxy éthoxy) hydrure d'aluminium et de sodium, puis, sous agitation on ajoute goutte à goutte, à température ambiante, 19,2 g (49,5 mmol) de 4-nitro-4-phényl-1-(3,4,5-triméthoxyphényl)-hexan-1-one (décrite en II.c) en solution dans 100 ml de toluène.

Le mélange est chauffé 4 heures au reflux puis refroidi par un bain d'eau glacée; on y ajoute alors avec précautions 11,7 ml de solution d'hydroxyde de sodium à 10% (p/v) puis 14,5 ml d'eau. La suspension est agitée durant 16 heures puis on ajoute 500 ml d'eau et extrait par 3 fois 200 ml d'éther. Les phases éthérées réunies sont lavées à l'eau puis séchées sur $Na_2SO_4$. L'éther est éliminé par distillation sous pression réduite ; le résidu qui est l'intermédiaire III (Q = O) correspondant au composé II de départ est engagé tel quel dans la réaction d'hydrogénation qui suit.

Dans un réacteur étanche à une pression interne d'environ $1,2.10^6$ Pa on introduit 9,1 g (23 mmol) du 4-nitro-4-phényl-1-(3,4,5-triméthoxyphényl)-hexan-1-ol obtenu précédemment avec 250 ml d'éthanol et 4,0 +/- 1,0 g de nickel de Raney activé de type W 2 en suspension dans environ 70 ml d'éthanol. Après avoir effectué les purges convenables comme indiquées à l'exemple 1, on introduit de l'hydrogène à raison d'une pression de $10^6$ Pa puis agite la suspension à température ambiante durant 72 heures.

Le catalyseur est ensuite filtré et le filtrat concentré sous pression réduite, le résidu est dissout dans 100 ml d'éther et extrait par 100 ml d'une solution d'acide chlorhydrique 3 N puis par 2 fois 75 ml d'acide N.

Les phases acides sont réunies et alcalinisées jusqu'à pH 12 à une température inférieure à 25°C par une solution concentrée d'hydroxyde de sodium (d = 1,33), on extrait ensuite par 3 fois 100 ml d'éther. Les phases éthérées réunies sont lavées à l'eau, séchées sur $Na_2SO_4$; l'éther est ensuite éliminé par distillation sous pression réduite pour obtenir finalement le produit sous forme d'une gomme.

Poids = 7,6 g    Rdt = 90%
CCM : 0,20 et 0,30 (isomères) ; S. A.2
RMN : 0,90 (m,3H), 1,20 (t,2H), 1,40-2,50 (m,3H), 3,00 (m,1H), 3,50 (q,1H), 3,80 (m,9H), 4,60 (m,1H), 6,55 (s,2H), 7,40 (s,5H), 9,00 (m,2H).
- Chlorhydrate :
Rdt = 75 %    F = 85 à 125°C (éther)
Anal.($C_{21}H_{29}NO_4$.HCl.0,2$C_2H_5$-O-$C_2H_5$) C, H, Cl, N, O IR (KBr): 3310, 2950, 1598, 1513, 1468, 1427, 1335, 1243,1135, 1012, 848, 770, 707 cm-1.

EXEMPLES 3

Mode opératoire général :

- stade a : (intermédiaire N-formamide V.D.2 à partir de composés de l'invention de formule I.D.1)

Dans un réacteur protégé de l'humidité et sous atmosphère d'azote on introduit 250 ml de tétrahydrofurane déshydraté sur tamis moléculaire, 3,9 ml d'acide formique à 99 % (d = 1,22 - 104 mmol) et 16,5 g (102 mmol) de 1,1'-carbonyldiimidazole.

La solution est agitée une heure à température ambiante puis on y ajoute goutte à goutte 100 mmol du composé I.D.1 en solution dans 100 ml de tétrahydrofurane. L'agitation est maintenue durant 4 heures à température ambiante puis le solvant est éliminé par distillation sous pression réduite.

Le résidu est traité par 250 ml de solution d'acide chlorhydrique 0,5 N et 200 ml d'éther. La phase aqueuse acide est décantée et extraite à nouveau par deux fois 100 ml d'éther.

Les phases éthérées réunies sont lavées à l'eau puis séchées sur $Na_2SO_4$. L'éther est éliminé par distillation sous pression réduite et le produit résiduel obtenu est utilisé tel quel ou purifié si nécessaire par cristallisation.

- stade b : (composés I de structures I.D.2 dans lesquels R3 = $CH_3$ à partir de N-formamides V.D.2)

Dans un réacteur anhydre et sous atmosphère d'azote on introduit 400 mmol de bis (2-éthoxy-méthoxy) hydrure d'aluminium et de sodium en solution dans le toluène, puis sous agitation et à température ambiante

on ajoute goutte à goutte 100 mmol du composé N -formamide V.D.2 en solution dans 400 ml de toluène.

Le mélange est agité à environ 20°C durant une période de 2 à 8 heures selon le composé engagé, l'avancement de la réaction étant déterminé par CCM. Au moment opportun on refroidit le mélange à une température inférieure à 10°C et introduit goutte à goutte 23 ml de solution à 10% (p/v) d'hydroxyde de sodium puis 28 ml d'eau. Après une heure d'agitation l'insoluble est filtré et lavé à l'éther sur un buchner garni d'un lit de terre d'infusoires, les filtrats réunis sont lavés à l'eau, séchés sur Na2SO4 puis les solvants sont éliminés par distillation sous pression réduite. Le résidu est purifié par les méthodes appropriées habituelles, à l'exception, toutefois, des traitements aqueux acides ou alcalins.

Exemple 3.a :<u>2-(3-N-méthylamino-3-phényl-pentan-1-yl)-2-(3,4-dichlorophényl)-1,3-dioxolane.</u>

[I ; R1 = C6H5, R2 = C2H5, R3 = CH3, R4 = H, R5 = 3,4 (Cl)2-C6H3, W = C(O-CH2-CH2-O)]

a) Préparé à partir du composé de l'exemple 1.a
Rdt = 75 % brut (huile)
CCM : 0,30 ; S. H.3
le produit est utilisé sans purifications.
b) Rdt = 55 % chromatographie F = 58 - 70°C (hexanes)
CCM : 0,70 ; S. H.3
Anal.(C21H25Cl2NO2) C, H, Cl, N, O
RMN : 0,55 (t,3H), 1,25 (s,1H), 1,45-1,85 (q,9H), 2,05 (d,3H), 3,60-4,10 (m,4H), 7,10-7,60 (m,8H).
IR (KBr): 3340, 2980, 2750, 2420, 1585, 1500, 1470, 1415, 1380, 1345, 1305, 1225, 1190, 1130, 1090, 1035, 952, 890, 830, 760, 700cm-1.

Exemple 3.b : <u>2-(3-N-méthylamino-3-phényl-pentan-1-yl)-2-phényl-1,3-dioxolane.</u>

[I ; R1 = R5 = C6H5, R2 = C2H5, R3 = CH3, R4 = H, W = C(O-CH2-CH2-O)]

a) Préparé à partir du composé de l'exemple 1.b
Rdt = 65 % brut (huile)
CCM : 0,65 ; S. H.3
le produit est utilisé sans purifications.
b) Rdt = 89 %    F = 58 - 70°C (hexanes)
CCM : 0,50 ; S. H.3
Anal.(C21H27NO2) C, H, N, O
RMN : 0,60 (t,3H), 1,30 (s,1H), 1,55-1,95 (m,6H), 2,10 (s,3H), 3,65-4,15 (m,4H), 7,15-7,55 (m,10H).
IR (film) : 3350, 3090, 3060, 3030, 2970, 2890, 1950, 1890, 1600, 1580, 1490, 1465, 1400, 1380, 1295, 1220, 1190, 1145, 1045, 955, 875, 765, 700 cm-1.

Exemple 3.c :<u>2-(3-N-méthylamino-3-phényl-pentan-1-yl)-2-(3,5-diméthoxyphényl)-1,3-dioxolane.</u>

[I ; R1 = C6H5, R2 = C2H5, R3 = CH3, R4 = H, R5 = 3,5 (CH3O)2-C6H3, W = C(O-CH2-CH2-O)]

a) Préparé à partir du composé de l'exemple 1.c on obtient l'intermédiaire V.D.2 [R5 = 3,4,5(CH3O)3-C6H2]
Rdt = 82 % brut (huile)
CCM : 0,20 ; S. H.1
le produit est utilisé sans purifications.
b) La réaction s'accompagne de la déméthoxylation en position 4 du radical R5 du composé.
Rdt = 48%    F = 71°C (hexanes - éther)
CCM : 0,35 ; S. A.1
Anal.(C23H31NO4) C, H, N, O
RMN : 0,60 (t,3H), 1,30 (s,1H), 1,50-1,90 (m,6H), 2,05 (s,3H), 3,80 (s,6H), 3,60-4,10 (m,4H), 6,60 (d,3H), 7,10-7,30 (m,5H).
IR (KBr): 3220, 1590, 1450, 1418, 1315, 1233, 1188, 1148, 1054, 1032, 847, 750, 690 cm-1.

Exemple 3.d : <u>2-[3-N-méthylamino-3-(4-chlorophényl)-pentan-1-yl]-2-(3,4,5-triméthoxyphényl)-1,3-dioxolane.</u>

[I ; R1 = p.Cl-C6H4, R2 = C2H5, R3 = CH3, R4 = H, R5 = 3,4,5 (CH3O)3-C6H2, W = C(O-CH2-CH2-O)]

a) Préparé à partir du composé de l'exemple 1.d

Rdt = 65 % F = 94°C (hexanes)
CCM : 0,50 ; 0,60 ; S. C.2
b) Rdt = 58 % F = 94°C (éther)
CCM : 0,50 ; S. C.2
Anal.(C24H32ClNO5) C, H, Cl, N, O
RMN : 0,60 (t,3H), 1,10-1,35 (m,H), 1,45-1,85 (m,6H), 2,05 (s,3H), 3,50-4,05 (m,13H), 6,60 (s,2H), 7,25 (s,4H) IR (KBr): 3000, 1580, 1420, 1400, 1380, 1220, 1120, 1000, 820 cm-1

Exemple 3.e.1 :2-[3-N-méthylamino-3-(4-méthylphényl)-pentan-1-yl]-2-(3,4,5-triméthoxyphényl)-1,3-dioxolane.

[I ; R1 = p.CH3-C6H4, R2 = C2H5, R3 = CH3, R4 = H, R5 = 3,4,5 (CH3O)3-C6H2, W = C(O-CH2-CH2-O)]
a) Préparé à partir du composé de l'exemple 1.e
Rdt = 85 % F = 118°C (hexanes)
CCM : 0,40 ; S. C.2
b) Rdt = 52 % F = 95°C (hexanes)
CCM : 0,30 ; S. C.2
Anal. (C25H35NO5) C, H, N, O
RMN : 0,60 (t,3H), 1,20-1,85 (m,7H), 2,05 (s,3H), 2,30 (s,3H),3,650-4,10 (m,13H), 6,60 (s,2H), 7,00-7,30 (m,4H)
IR (KBr) : 3000, 1590, 1500,1450, 1400, 1320, 1220, 1100, 1000, 820 cm-1

Exemple 3.e.2 :2-[3-N-méthylamino-3-(4-méthylphényl)-pentan-1-yl]-2-(3,5-diméthoxyphényl)-1,3-dioxolane.

[I ; R1 = p.CH3-C6H4, R2 = C2H5, R3 = CH3, R4 = H, R5 = 3,5 (CH3O)2-C6H3, W = C(O-CH2-CH2-O)]
Sous produit obtenu au stade de réduction b) dans la préparation du produit de l'exemple précédent.
CCM : 0,45 ; S. C.2
Anal. (C24H33NO4) CHNO
RMN : 0,60 (t,3H), 1,20-1,85 (m,7H), 2,05 (s,3H), 2,30 (s,3H), 3,65-4,10 (m,10H), 6,60 (m,3H), 7,00-7,30 (m,4H)

Exemple 3.f :2-(3-N-méthylamino-3-(4-trifluorométhyl phényl)-pentan-1-yl]-2-(3,4,5-triméthoxyphényl)-1,3-dioxolane.

[I ; R1 = p.F3C-C6H4, R2 = C2H5, R3 = CH3, R4 = H, R5 = 3,4,5 (CH3O)3-C6H2, W = C(O-CH2-CH2-O)]
a) Préparé à partir du composé de l'exemple 1.f
Rdt = 64 % F = 144°C (éther)
CCM : 0,80 ; S. C.2
b) Rdt = 78 % F = 85°C (hexanes)
CCM : 0,60 - 0,70 ; S. C.2
Anal. (C25H32F3NO5) C, H, F, N, O
RMN : 0,60 (t,3H), 1,30 (s,1H), 1,50-1,80 (m,6H),2,10 (s,3H), 3,70-4,10 (m,13H), 6,60 (s,2H), 7,35-7,60 (m,4H) IR (KBr) : 2990, 1580, 1460, 1410, 1320, 1220, 1110, 930 cm-1

Exemple 4 :4-N-méthylamino-4-phényl-1-(3,4,5-triméthoxy) phényl-hexan-1-ol.

[I ; R1 = C6H5, R2 = C2H5, R3 = CH3, R4 = H, R5 = 3,4,5 (CH3O)3-C6H2, W = CHOH]

La réaction du stade a) de l'exemple 3 précédent est appliquée à 21,5 g (60 mmol) du 4-amino hexanol de l'exemple 2.

On obtient après traitements 18,4 g (46 mmol) d'un mélange du N-formamide hexanol correspondant et de son formate (Rdt = 77 %)

Tel quel, ce mélange en solution dans 100 ml de tétrahydrofurane anhydre, est introduit goutte à goutte à la température ambiante dans une solution de 9,9 g (260 mmol) d'hydrure de lithium aluminium dans 250 ml de tétrahydrofurane.

Le mélange est chauffé durant 5 heures au reflux, refroidi à 20°C par un bain d'eau glacée puis dilué par addition de 200 ml d'éther anhydre. On ajoute ensuite goutte à goutte, avec précautions, 16,0 ml de solution d'hydroxyde de sodium à 10 % (p/v) puis 20,0 ml d'eau. Le mélange est agité durant 16 heures à la température

ambiante puis l'insoluble est filtré. Le filtrat est concentré par distillation sous pression réduite. On obtient le mélange d'isomères du produit sous la forme d'une huile visqueuse de couleur orange.
Poids = 8,1 g  Rdt = 47 %
CCM : 0,50 et 0,90 (isomères) ; S. A.2
RMN : 0,90 (m,3H), 1,70 (m,2H), 2,25 (m,4H), 3,20 (s,1H), 3,75-3,90 (m,12H), 4,55-5,10 (m,1H), 6,60 (s,2H), 7,30 (s,5H), 7,75 (m,1H).
- Chlorhydrate
Rdt = 85 %  F = 112°C (benzène - $CH_2Cl_2$)
Anal. ($C_{22}H_{31}NO_4.HCl$) C, H, Cl, N, O
IR (KBr) : 3420, 2940, 1590, 1503, 1460, 1423, 1328, 1240, 1130, 1010, 840, 764, 700 cm-1.

EXEMPLES 5

Mode opératoire général : Dans un réacteur anhydre et maintenu sous atmosphère d'azote, on introduit goutte à goutte 100 mmol d'un intermédiaire α-dialkylamino butyronitrile VII dans 150 ml d'éther déshydraté sur tamis moléculaire dans une solution de 500 mmol de bromure d'éthyl magnésium dans 350 ml d'éther.

L'introduction est réalisée en 30 minutes environ puis le mélange est chauffé au reflux durant 2 heures et refroidi par un bain d'eau glacée avant d'être précipité dans 400 ml de solution saturée en chlorure d'ammonium.

La phase aqueuse est décantée et écartée, la phase éthérée est extraite à deux reprises par 200 ml de solution d'acide chlorhydrique N. Les phases acides réunies sont alcalinisées à une température inférieure à 20°C jusqu'à pH 12 par une solution d'hydroxyde de sodium (d = 1,33) puis extraites à trois reprises par 250 ml d'éther. Les phases réunies sont lavées par extraction avec une solution saturée en chlorure de sodium puis séchées sur $Na_2SO_4$. L'éther est éliminé par distillation sous vide et le produit résiduel brut est, après examen chromatographique de sa pureté, soit utilisé tel quel, soit purifié par une méthode appropriée.

Les composés I.D.3 des exemples 5.a à 5.c sont préparés selon ce protocole à partir de butyronitriles VII dont la préparation est précédemment décrite.

Exemple 5.a : 2-(3-N,N-diméthylamino-3-phényl-pentan-1-yl)-2-phényl-1,3-dioxolane

[I ; R1 = R5 = $C_6H_5$, R2 = $C_2H_5$, R3 = R4 = $CH_3$, W = C(O-$CH_2$-$CH_2$-O)]
Préparé à partir du composé VII.a
Rdt = 75 % brut (huile)
CCM : 0,20 - 0,30 ; S. H.5
Anal. ($C_{22}H_{29}NO_2$) C, H, N, O
RMN : 0,60 (t,3H), 1,60-1,90 (m,6H), 2,10 (s,6H), 3,60-4,20 (m,4H), 7,00-7,60 (m,10H)

Exemple 5.b : 2-(3-N,N-diméthylamino-3-phényl-pentan-1-yl)-2-(3,4,5-triméthoxyphényl)-1,3-dioxolane

[I ; R1 = $C_6H_5$, R2 = $C_2H_5$, R3 = R4 = $CH_3$, R5 = 3,4,5 ($CH_3O$)-$C_6H_2$, W = C(O-$CH_2$-$CH_2$-O)]
Préparé à partir du composé VII.b
Rdt = 48 % brut (huile)
CCM : 0,30 ; S. H.1
Anal.($C_{25}H_{35}NO_5$) C, H, N, O
RMN :0,60 (t,3H), 1,60-1,90 (m,6H), 2,10 (s,6H), 3,60-4,20 (m,4H), 6,60 (s,2H), 7,00-7,60 (m,5H)

Exemple 5.c : 2-(3-N,N-diméthylamino-3-phényl-pentan-1-yl)-2-cyclohexyl-1,3-dioxolane

[I ; R1 = $C_6H_5$, R2 = $C_2H_5$, R3 = R4 = $CH_3$, R5 = CH $(CH_2)_5$, W = C(O-$CH_2$-$CH_2$-O)]
Préparé à partir du composé VII.c
Rdt = 59 % brut (huile)
CCM : 0,30 - 0,40 ; S. H.3
Anal. ($C_{22}H_{35}NO_2$) C, H, N, O
RMN : 0,60 (t,3H), 1,60-1,90 (m,6H), 2,10 (s,6H), 3,60-4,20 (m,14H), 5,90 (m,H), 7,00-7,60 (m,5H)

Exemple 6 : 2-[3-(N-cyclopropylméthyl-N-méthylamino)-3-(4-méthylphényl)-pentan-1-yl]-2-(3,4,5-triméthoxy phényl)-1,3-dioxolane.

[I ; R1 = p. CH3-C6H4, R2 = C2H5, R3 = CH2-CH(CH2)2, R4 = CH3, R5 = 3,4,5(CH3O)3-C6H2,W = C(O-CH2-CH2-O)]

Stade 1 : Dans un réacteur anhydre et sous atmosphère d'azote on introduit 45 ml de chlorure de méthylène déshydraté sur tamis moléculaire puis successivement 5,00 g (11,6 mmol) du composé préparé à l'exemple 3.e.1, et 2,0 ml (1,46 g - 14,4 mmol) de triéthylamine.

La solution est refroidie dans un bain de glace puis on introduit goutte à goutte et à une température inférieure à 10°C, 1,2 ml (1,38 g - 13,2 mmol) de chlorure d'acide cyclopropylcarboxylique.

Le mélange est ensuite agité durant 2 heures 30 à la température ambiante , on ajoute alors 50 ml de chlorure de méthylène puis extrait la solution successivement et à chaque reprise par 50 ml d'une solution aqueuse à 10 % d'ammoniaque, d'eau, d'une solution à 10 % d'acide chlorhydrique, d'eau, d'une solution saturée en bicarbonate de sodium et finalement par de l'eau.

La phase organique ainsi traitée est déshydratée sur Na2SO4 puis le chlorure de méthylène est évaporé par distillation sous vide.

On obtient le dérivé N-cyclopropylcarboxamide intermédiaire de formule V.D.3 sous forme d'huile visqueuse dont l'état de pureté à l'état brut est satisfaisant pour que le produit soit engagé tel quel au stade suivant.

Poids = 5,50 g Rdt = 95 %

CCM : 0,65 - 0,75 , S. C.2

Anal. (C29H39NO6) C, H, N, O

RMN : 0,40-0,90 (m,6H), 1,60-2,40 (m,11H), 3,10 (s,3H), 3,60-4,05 (m,13H), 6,60 (s,2H), 6,90-7,20 (m,4H)

Stade 2 : Dans un réacteur anhydre et sous atmosphère d'azote on introduit 13,4 ml (d = 1,03 -48 mmol) d'une solution toluènique à 70% p/p de bis (2-méthoxy éthoxy) hydrure d'aluminium et de sodium et 40 ml de toluène déshydraté sur tamis moléculaire. En 30 minutes environ, goutte à goutte et à une température inférieure à 25°C on introduit 5,9 g (12 mmol) du N-carboxamide en solution dans 40 ml de toluène déshydraté. Le mélange est chauffé et maintenu au reflux durant 40 minutes puis refroidi rapidement à 10°C environ par un bain d'eau glacée.

On ajoute ensuite goutte à goutte et à une température inférieure à 20°C, 2,8 ml d'une solution d'hydroxyde de sodium à 10 % p/v puis 3,5 ml d'eau. Après agitation durant une heure le mélange est filtré sur un buchner garni d'une couche de terre d'infusoires.

Le filtrat est évaporé sous vide ; on obtient le produit brut à l'état d'huile (5,2 g) qui est purifiée par chromatographie.

Poids = 4,3 g (huile) Rdt = 74 %

CCM : 0,15 - 0,30 ; S. C.2

Anal. (C29H41NO5) C, H, N, O

RMN : 0,00 (m,2H), 0,30-0,50 (m,2H), 0,60-0,85 (m,4H), 1,60-2,40 (m,14H), 3,70-4,10 (m,13H), 6,65 (s,2H), 7,00-7,35 (m,4H)

Exemple 7 : 2-(3-N,N-diméthylamino-3-(4-méthylphényl)-pentan-1-yl]-2-(3,5-diméthoxyphényl)-1,3-dioxolane.

[I ; R1 = p. CH3-C6H4, R2 = C2H5, R3 = R4 = CH3, R5 = 3,5(CH3O)2-C6H3, W = C(O-CH2-CH2-O)]

Dans un réacteur muni d'un système d'agitation on introduit 4,6 g (11,5 mmol) de la N-méthyl-α-tolylpropanamine décrite à l'exemple 3.e.2 et 1,95 ml (26 mmol) de solution de formaldéhyde à 37 % (d = 1,08), sous agitation violente le mélange est faiblement exothermique.

Dans l'émulsion formée on ajoute ensuite goutte à goutte sans dépasser 25°C, 1,35 ml (36 mmol) d'acide formique à 99 % (d = 1,22). La solution alors obtenue est chauffée sur bain marie durant 30 minutes jusqu'à fin de dégagement gazeux, puis elle est refroidie et diluée avec 50 ml d'eau et acidifiée jusqu'à pH 1 par addition d'acide chlorhydrique concentré (d = 1,18).

Le mélange est extrait par 2 fois 25 ml d'éther; les phases éthérées sont écartées et la phase aqueuse acide est alcalinisée à une température inférieure à 25°C, jusqu'à pH 12 par addition d'une solution concentrée d'hydroxyde de sodium (d = 1,33). Le mélange est extrait par 3 fois 40 ml d'éther; les phases éthérées réunies sont lavées par une solution saturée en chlorure de sodium puis déshydratées sur Na2SO4. L'éther est éliminé par distillation. Le produit est obtenu brut avec un état de pureté satisfaisant.

Poids = 3,4 g Rdt = 71 %

CCM : 0,50 - 0,65 , S. C.2

Anal. (C25H35NO4) C, H, N, O RMN : 0,75 (t,3H), 1,65-2,25 (m,12H), 3,70-4,10 (m,13H), 6,65 (s,2H), 7,15-7,50 (m,5H)

EXEMPLES 8

Mode opératoire général : Dans un réacteur on introduit 100 mmol d'un composé de l'invention I de structure I.D.1 à N,N diméthyler. Sous agitation énergique et à température ambiante il est ajouté rapidement 17,5 ml (233 mmol) de solution de formaldéhyde à 37% (d = 1,08).

La réaction est exothermique, il se forme une émulsion visqueuse qui est refroidie par un bain de glace. Sans dépasser 15°C, on ajoute goutte à goutte 11,4 ml d'acide formique à 99% (d = 1,22 - 302 mmol). La solution est ensuite chauffée durant une heure et demie sur un bain marie bouillant juqu'à fin complète de dégagement gazeux. On ajoute ensuite 150 ml d'eau, refroidit par un bain de glace, puis, sans dépasser 20°C alcalinise jusqu'à pH 12 par une solution concentrée d'hydroxyde de sodium (d = 1,33). Le mélange est extrait par 3 fois 150 ml d'éther. les phases éthérées réunies sont lavées par une solution saturée en NaCl puis séchées sur Na2SO4. L'éther est ensuite éliminé par distillation sous vide et le résidu est soit utilisé tel quel, soit purifié par les méthodes appropriées.

Les composés I de structure I.D.3 des exemples 8.a à 8.e qui sont décrits dans ce qui suit, sont préparés suivant ce protocole expérimental à partir des composés décrits aux exemples 1.

Exemple 8.a : 2-[3-(4-chlorophényl)-3-N,N-diméthylamino-pentan-1-yl]-2-(3,4,5-triméthoxyphényl)-1,3-dioxolane.

[I ; R1 = p.Cl-C6H4, R2 = C2H5, R3 = R4 = CH3, R5 = 3,4,5(CH3O)-C6H2, W = C(O-CH2-CH2-O)]
Préparé à partir du composé I.D.1 de l'exemple 1.d
Rdt = 93 % brut (huile)
CCM : 0,35 - 0,45 , S. C.2
Anal. (C25H34ClNO5) C, H, N, O
RMN : 0,75 (t,3H), 1,65-2,25 (m,12H), 3,70-4,10 (m,13H), 6,65 (s,2H), 7,15-7,40 (m,4H)

Exemple 8.b : 2-[3-N,N-diméthylamino-3-(4-méthylphényl)-pentan-1-yl]-2-(3,4,5-triméthoxyphényl)-1,3-dioxolane.

[I ; R1 = p.CH3-C6H4, R2 = C2H5, R3 = R4 = CH3, R5 = 3,4,5(CH3O)-C6H2, W = C(O-CH2-CH2-O)]
Préparé à partir du composé I.D.1 de l'exemple 1.e
Rdt = 84 % brut (huile)
CCM : 0,40 ; S. C.2
Anal. (C26H37NO5) C, H, N, O
RMN : 0,75 (t,3H), 1,70-2,20 (m,12H), 2,30 (s,3H), 3,65-4,15 (m,13H), 6,65 (s,2H), 7,00-7,30 (m,4H)

Exemple 8.c : 2-[3-N,N-diméthylamino-3-(4-trifluoro méthylphényl)-pentan-1-yl]-2-(3,4,5-triméthoxyphényl)-1,3-dioxolane.

[I ; R1 = p.CF3-C6H4, R2 = C2H5, R3 = R4 = CH3, R5 = 3,4,5(CH3O)-C6H2, W = C(O-CH2-CH2-O)]
Préparé à partir du composé I.D.1 de l'exemple 1.f
Rdt = 82 % brut (huile)
CCM : 0,50 - 0,60 ; S. C.2
Anal. (C25H34F3NO5) C, H, N, O
RMN : 0,75 (t,3H), 1,65-2,25 (m,12H), 3,70-4,10 (m,13H), 6,65 (s,2H), 7,15-7,40 (m,4H)

Exemple 8.d : 2-[3-(3,4-dichlorophényl)-3-N,N-diméthyl amino-pentan-1-yl]-2-(3,4,5-triméthoxyphényl)-1,3-dioxolane.

[I ; R1 = 3,4(Cl)2-C6H3, R2 = C2H5, R3 = R4 = CH3, R5 = 3,4,5(CH3O)-C6H2, W = C(O-CH2-CH2-O)]
Préparé à partir du composé I.D.1 de l'exemple 1.g
Rdt = 84 % brut (huile)
CCM : 0,80 , S. C.2
Anal. (C25H33C12NO5) C, H, Cl, N, O
RMN : 0,75 (t,3H), 1,65-2,25 (m,12H), 3,70-4,10 (m,13H), 6,65 (s,2H), 7,10-7,40 (m,3H)

Exemple 8.e : 2-(3-N,N-diméthylamino-3-phényl-pentan-1-yl)-2-(3,4-dichlorophényl)-1,3-dioxolane

[I ; R1 = C6H5, R2 = C2H5, R3 = R4 = CH3, R5 = 3,4(Cl)2-C6H3 , W = C(O-CH2-CH2-O)]
Préparé à partir du composé I.D.1 de l'exemple 1.a
Rdt = 77 % brut (huile)
CCM : 0,60 - 0,80 , S. C.2
Anal. (C22H27Cl2NO2) C, H, N, O
RMN : 0,75 (t,3H), 1,65-2,25 (m,12H), 3,70-4,10 (m,13H), 6,65-7,40 (m,8H)

EXEMPLES 9

Mode opératoire général : Dans un réacteur équipé d'un réfrigérant en position de reflux et d'un système d'agitation on introduit 100 mmol de composé 1,3-dioxolane-2-propanamine (I.D.3) à traiter en solution dans 540 ml d'acétone.

Sous agitation, on ajoute ensuite 500 ml d'eau déminéralisée puis 42,5 ml d'acide chlorhydrique concentré (d = 1,18). Le mélange est porté sur bain marie et chauffé 30 minutes au reflux puis refroidi à la température ambiante , l'acétone est ensuite éliminée par distillation sous vide.

Au résidu de concentration on ajoute 625 ml d'eau glacée puis extrait par 3 fois 200 ml d'éther diéthylique. Les phases éthérées sont écartées, la phase acide est alcalinisée jusqu'à pH 12 par une solution d'hydroxyde de sodium concentrée (d = 1,33). Le mélange est extrait par 3 fois 200 ml d'éther, les phases éthérées réunies sont lavées par une solution saturée en chlorure de sodium puis séchées sur Na2SO4. Après filtration de l'agent déshydratant, l'éther est éliminé par distillation sous vide.

Selon son état de pureté qui est déterminé par CCM, le produit brut obtenu est soit purifié par des méthodes appropriées comme la distillation sous vide poussé, la cristallisation ou encore la chromatographie, soit engagé tel quel dans la préparation d'un sel d'addition qui est le plus souvent un chlorhydrate.

Les composés de l'invention décrits aux exemples 9.a à 9.i qui suivent sont obtenus selon ce protocole à partir des composés de l'invention I de structure I.D.3 préparés aux exemples 5, 6, 7 et 8 précédents.

Exemple 9.a : 4-(4-chlorophényl)-4-N,N-diméthylamino-1(3,4,5-triméthoxyphényl)-hexan-1-one.

[I ; R1 = p.Cl-C6H4, R2 = C2H5, R3 = R4 = CH3, R5 = 3,4,5(CH3O)3-C6H2, W = CO]
Préparation à partir du composé I.D.3 de l'exemple 8.a
Rdt = 84 % brut (huile)
CCM : 0,50 - 0,60 ; S. C.2
RMN : 0,85 (t,3H), 1,95-2,40 (m,10H), 2,70-3,10 (m,2H), 3,90 (s,9H), 7,20 (s,2H), 7,35 (s,4H)
- Chlorhydrate
Rdt = 78% F = 203°C (éthanol)
Anal. (C23H30ClNO4.HCl) C, H, Cl, N, O
IR (KBr) : 3400, 3000, 2500, 2400, 1680, 1580, 1460, 1410, 1320, 1120, 990, 820 cm-1

Exemple 9.b.1 : 1-(3,5-diméthoxyphényl)-4-N,N-diméthyl amino- 4-(4-méthylphényl)-hexan-1-one.

[I ; R1 = p.CH3 - C6H4, R2 = C2H5, R3 = R4 = CH3, R5 = 3,5 (CH3O)2-C6H3, W = CO]
A partir du composé I.D.3 de l'exemple 7.
Rdt = 79 % chromatographie (huile)
CCM : 0,55 - 0,65 ; S. C.2
RMN : 0,85 (t,3H), 1,75-2,50 (m,13H), 2,80-3,10 (m,2H), 3,85 (s,6H), 6,60-7,40 (m,7H)
- Chlorhydrate
Rdt = 72 % F = 159°C (CH2Cl2 - éther)
Anal. (C23H31NO3.HCl) C, H, Cl, N, O
IR (KBr) : 3400, 2900, 2400, 1680, 1580, 1480, 1440, 1320, 1200, 1140, 1060, 1000, 800 cm-1

Exemple 9.b.2 : 4-N,N-diméthylamino-4-(4-méthylphényl)-1-(3,4,5-triméthoxyphényl)-hexan-1-one.

[I ; R1 = p.CH3 - C6H4, R2 = C2H5, R3 = R4 = CH3, R5 = 3,4,5 (CH3O)3-C6H2, W = CO]
A partir du composé I.D.3 de l'exemple 8.b
Rdt = 70 % cristallisé F = 81°C (hexanes)
CCM : 0,30 - 0,50 ; S. C.2

RMN : 0,85 (t,3H), 1,85-2,45 (m,13H), 2,75-3,10 (m,2H), 3,90 (s,9H), 7,10-7,40 (m,6H)
- Chlorhydrate
Rdt = 85 % F = 188°C (CH2Cl2 - éther)
Anal. (C24H33NO4.HCl) C, H, Cl, N, O
IR (KBr) : 3400, 3000, 2700, 1670, 1580, 1540, 1500, 1420, 1120, 980, 800 cm-1

Exemple 9.c : 4-N,N-diméthylamino-4-(4-trifluorométhyl phényl)-1-(3,4,5-triméthoxyphényl)-hexan-1-one.

[I ; R1 = p.CF3 - C6H4, R2 = C2H5, R3 = R4 = CH3, R5 = 3,4,5 (CH3O)3-C6H2, W = CO]
A partir du composé I.D.3 de l'exemple 8.c
Rdt = 92 % brut (huile)
CCM : 0,70 - 0,80 ; S. C.2
RMN : 0,85 (t,3H), 1,70-2,45 (m,10H), 2,75-3,10 (m,2H), 3,95 (s,9H), 7,210 (s,2H),7,60 (m,4H)
- Chlorhydrate
Rdt = 65 % F = 195°C (éthanol)
Anal. (C24H30F3NO4.HCl) C, H, Cl, F, N, O
IR (KBr) : 3400, 3000, 2900, 1680, 1580, 1540, 1460, 1410, 1320, 1110, 1000, 820 cm-1

Exemple 9.d : 4-(3,4-dichlorophényl)-4-N,N-diméthylamino-1-(3,4,5-triméthoxyphényl)-hexan-1-one.

[I ; R1 = 3,4 (Cl)2-C6H4, R2 = C2H5, R3 = R4 = CH3, R5 = 3,4,5 (CH3O)3-C6H2, W = CO]
A partir du composé I.D.3 de l'exemple 8.d
Rdt = 86 % brut (huile)
CCM : 0,80 - 0,90 , S. C.2
RMN : 0,85 (t,3H), 1,70-2,40 (m,10H), 2,70-3,00 (m,2H), 3,90 (s,9H), 7,10 -7,60 (m,5H)
- Chlorhydrate
Rdt = 70 % F = 153°C (éthanol)
Anal. (C23H29Cl2NO4.HCl) C, H, Cl, N, O
IR (KBr) : 3400, 2900, 2600, 1680, 1560, 1460, 1410, 1380, 1120, 990 cm-1

Exemple 9.e : 4-N,N-diméthylamino-1,4-diphényl-hexan-1-one.

[I ; R1 = R5 = C6H5, R2 = C2H5, R3 = R4 = CH3, W = CO]
A partir du composé I.D.3 de l'exemple 5.a
Rdt = 38 % Eb / 7Pa = 163 - 185°C
CCM : 0,25 ; S. H.3
Anal. (C20H25NO) C, H, N, O
RMN : 0,80(t,3H), 1,65-2,60(m,10H), 2,70-3,20(m,2H), 6,95-7,65(m,8H), 7,75-8,10(m,2H)
IR (film) : 3065, 2980, 2945, 2885, 2835, 2795, 1682, 1597, 1578, 1447, 1317, 1290, 1233, 1208, 1180, 1001, 981, 759, 741, 700, 691.

Exemple 9.f : 4-N,N-diméthylamino-4-phényl-1-(3,4,5-triméthoxyphényl)-hexan-1-one.

[I.C.3 ; R1 = C6H5, R2 = C2H5, R3 = R4 = CH3, R5 = 3,4,5(CH3O)3-C6H2, W = CO]
A partir du composé I.D.3 de l'exemple 5.b
Rdt = 55 % chromatographié (huile)
CCM : 0,30 ; S. B
Anal. (C23H31NO4) C, H, N, O
RMN : 0,90 (t,3H), 1,80-3,40 (m,6H), 2,20 (s,6H), 3,85 (s,9H), 7,20 (s,2H), 7,35 (s,5H)
IR (film) : 3100-2780, 1677, 1584, 1502, 1459, 1412, 1330, 1231, 1188, 1154, 1128, 1005, 855, 763, 703, 680 cm-1

Exemple 9.g : 1-(3,4-dichlorophényl)-4-N,N-diméthylamino-4-phényl-hexan-1-one.

[I ; R1 = C6H5, R2 = C2H5, R3 = R4 = CH3, R5 = 3,4 (Cl) 2-C6H3, W = CO]
A partir du composé I.D.3 de l'exemple 8.e
Rdt = 96 % brut (huile)
CCM : 0,60 ; S. A.1

RMN : 0,80 (t,3H), 1,60-3,20 (m,12H), 7,20-8,10 (m,8H)
- Chlorhydrate
Rdt = 85 % F = 160°C (méthanol - éther)
Anal. (C20H23Cl2NO.HCl) C, H, Cl, N, O
IR (KBr) : 3650, 3405, 3060, 3010, 2900, 2660, 2600, 2570, 2450, 1670, 1640, 1610, 1580, 1550, 1515, 1425, 1405, 1385, 1350, 1340, 1320, 1292, 1278, 1252, 1200, 1155, 1135, 1080, 1043, 1030, 1020, 1000, 915, 900, 845, 825, 810, 765, 730, 705, 670 cm-1.

Exemple 9.h : 1-cyclohexyl-4-N,N-diméthylamino-4-phényl-hexan-1-one.

[I ; R1 = C6H5, R2 = C2H5, R3 = R4 = CH3, R5 = CH (CH2)5, W = CO]
A partir du composé I.D.3 de l'exemple 5.c
Rdt = 26 % distillé (huile) Eb/13Pa = 153 - 164°C
CCM : 0,30 ; S. H.5
Anal. (C20H31NO) C, H, O, N
RMN : 0,80 (t,3H), 0,90-2,50 (m,17H), 2,15 (s,6H), 7,40 (m,5H)
IR (film) : 1707, 1445, 1145, 1000, 758, 700 cm-1

Exemple 9.i : N-cyclopropylméthyl-4-N-méthylamino-4-(4-méthylphényl)-1-(3,4,5-triméthoxy)phényl-hexan-1-one.

[I ; R1 = p.CH3-C6H4, R2 = C2H5, R3 = CH3, R4 = CH2-CH (CH2)2, R5 = 3,4,5(CH3O)3-C6H2, W = CO]
A partir du composé I.D.3 de l'exemple 6
Rdt = 92 % brut (huile)
CCM : 0,60 - 0,75 ; S. C.2
RMN : 0,00 (m,2H), 0,30-0,60 (m,2H), 0,70-1,00 (m,4H), 1,70-2,45 (m,14H), 2,65-3,00 (m,2H), 3,90 (d,9H), 7,00-7,50 (m,6H)
- Chlorhydrate
Rdt = 75 % F = 112°C (méthanol)
Anal. (C27H37NO4.HCl) C, H, Cl, N, O
IR (KBr) : 3400, 2950, 2150, 1660, 1580, 1500, 1450, 1410, 1330, 1100, 950, 810 cm-1

Exemple 10 : N-allyl-1,4-diphényl-4-N-méthylamino-hexan-1-one

[I ; R1 = R5 = C6H5, R2 = C2H5, R3 = CH3, R4 = CH2-CH=CH2, W=CO]

Stade 1 : 2-(3-N-allylamino-3-phényl-pentan-1-yl)-2-phényl-1,3-dioxolane [I ; R1 = R5 = C6H5, R2 = C2H5, R3 = H, R4 = CH2-CH=CH2, W=C(0-CH2-CH2-0)]

Dans un réacteur de 250 ml protégé de l'humidité à 10,0 g (32 mmol) du composé I de l'exemple 1.b en solution dans 160 ml d'acétonitrile deshydraté on ajoute 2,73 ml (32 mmol) de bromure d'allyle (d=1,398)

La solution est chauffée durant 24 h au reflux, puis l'acétonitrile est éliminé par distillation sous vide. Le résidu brut est purifié par chromatographie sur colonne de silice en éluant par un mélange chlorure de méthylène-méthanol.
Poids : 9,5 g (huile jaune) Rdt = 84 %
CCM : 0,9 , S. C.2
RMN : 0,65 (t,3H), 1,20 (s,1H éch.), 1,50-1,85 (m,6H),
2,70-2,90 (m,2H), 3,60-4,05 (m,4H), 4,90-5,30 (m,2H), 5,60-6,10 (m,1H), 7,00-7,50 (m,10H)

Stade 2 : 2-(N-allylamino-3-N-méthylamino-3-phényl-pentan-1-yl)-2-phényl-1,3-dioxolane [I; R1 = R5 = C6H5, R2 = C2H5, R3 = CH3, R4 = CH2-CH=CH2, W=C(0-CH2-CH2-0)]

Dans un ballon de 100 ml, on introduit 8,9 g (25 mmol) de la propanamine du stade précédent, que l'on mélange intimement avec 4,4 ml de solution de formaldéhyde à 37 % (d=1,08).

On ajoute ensuite à l'émulsion obtenue, 2,92 ml d'acide formique à 99 % (d=1,22). La solution jaune orangé est chauffée 45 minutes sur bain-marie bouillant puis précipitée dans 100 ml d'eau glacée. Le mélange est acidifié jusqu'à pH 1 par une solution d'acide chlorhydrique concentré, puis extraite à deux reprises par 75 ml d'éther.

Les phases éthérées sont écartées, la phase aqueuse acide est alcalinisée jusqu'à pH 12, sans dépasser 20°C, par une solution concentrée d'hydroxyde de sodium. Le mélange est extrait par 3 fois 70 ml d'éther. Les phases éthérées réunies sont lavées par une solution saturée en chlorure de sodium puis séchées sur sulfate de sodium.

L'éther est évaporé. L'huile résiduelle jaune-orangé (7,0 g) est engagée telle quelle dans le stade suivant.

Stade 3 : N-allyl-1,4-diphényl-4-N-méthylamino-hexan-1-one

[I; R1 = R5 = C6H5, R2 = C2H5, R3 = CH3, R4 = CH2-CH=CH2, W=C0]

Dans un ballon de 500 ml, on introduit 7,0 g (19,2 mmol) d'allylamine du stade précédent, 8,2 ml d'acide chlorhydrique concentré (d=1,18) dans 200 ml de mélange acétone-eau 1-1 (v/v). La solution est chauffée et maintenue 30 minutes au reflux puis l'acétone est éliminée par distillation sous vide.

La phase aqueuse résiduelle est extraite à deux reprises par 100 ml d'éther. Les phases éthérées sont écartées, la phase aqueuse acide est alcalinisée par une solution concentrée d'hydroxyde de sodium puis extraite par 3 fois 70 ml d'éther. Les phases éthérées réunies sont lavées par une solution saturée en chlorure de sodium puis séchées sur sulfate de sodium.

Après élimination de l'éther par distillation on obtient 6,1 g d'huile orangée résiduelle qui est purifiée par chromatographie. L'élution par un mélange chlorure de méthylène-acétone permet d'obtenir le produit pur sous forme d'huile de couleur jaune pâle.

Poids : 5,7 g Rdt = 92,5 %

CCM : 0,70 ; S. C.2

RMN : 0,85 (t,3H), 1,80-2,40 (m,4H), 2,30 (s,3H), 2,80-3,20 (m,4H), 4,90-5,30 (m,2H), 5,60-6,00 (m,1H), 7,10-8,00 (m,10H)

IR (film) : 3000, 1680, 1440, 1280, 1200, 990, 905, 760, 740, 700 cm-1

Exemple 11 : N-allyl-1,4-diphényl-4-N-méthylamino-hexan-1-ol

[I , R1 = R5 = C6H5, R2 = C2H5, R3 = CH3, R4 = CH2-CH=CH2, W=CHOH]

Dans un réacteur protégé de l'humidité on introduit 150 ml de méthanol anhydre puis 5,0 g (15,5 mmole) de l'hexanone préparée à l'exemple 10 précédent.

A la solution jaune orangé obtenue on ajoute 0,75 g (19,7 mmol) de borohydrure de sodium sous agitation et à température ambiante. Après dégagement gazeux, la solution jaune pâle est maintenue sous agitation à 20°C durant une heure puis on y ajoute 15,5 ml d'eau.

Le mélange est agité 20 minutes puis le méthanol est éliminé par distillation sous vide. Après addition de 100 ml d'eau au résidu de distillation, il est extrait par deux fois 75 ml d'éther.

Les phases éthérées réunies sont lavées par une solution saturée en chlorure de sodium puis déshydratées sur Na2SO4.

L'éther est éliminé par distillation. Le résidu brut huileux (4,9 g) est purifié par chromatographie. L'élution par du chlorure de méthylène progressivement enrichi en méthanol permet, avec le mélange CH2Cl2-CH3OH 95-5 (v/v) d'obtenir les isomères du composé sous forme d'une huile visqueuse incolore.

Poids : 4,30 g Rdt = 86 %

CCM : 0,80 ; S. C.2

Anal. (C22H29NO) C, H, N, O

RMN : 0,60-0,90 (m,3H), 1,60-2,20 (m,9H), 2,80-3,10 (m,2H), 3,40-3,70 (m,1H ech.), 4,50-4,70 (m,1H), 4,90-5,20 (m,2H), 5,50-6,00 (m,1H), 7,10-7,50 (m,10H) IR (film) : 3400, 3000, 1490, 1450, 990, 910, 740, 700 cm-1

Exemple 12 : 2-(3-N,N-diméthylamino-3-phényl-pentan-1-yl)-2-phényl-1,3-dithiolane

[I , R1 = R5 = C6H5, R2 = C2H5, R3 = R4 = CH3, W = C (S-CH2-CH2-S)]

Dans un ballon de 25 ml protégé de l'humidité et sous atmosphère d'azote, on introduit 5,0 g (17 mmol) de 4-dimethylamino-1,4-diphényl-hexanone préparée à l'exemple 9.e et 8,3 ml (d=1,233 - 109 mmol) d'éthanedithiol.

Sous agitation, on ajoute à la solution orangée 8,3 ml (d=1,154 - 67 mmol) d'éthérate de trifluorure de bore. La solution noirâtre est agitée durant 24 heures à la température du laboratoire puis précipitée dans 100 ml d'eau glacée, alcalinisée à pH 12 par une solution concentrée d'hydroxyde de sodium, puis extraite par deux fois 100 ml d'éther.

Les phases éthérées réunies sont lavées par une solution saturée en chlorure de sodium puis déshydra-

tées sur $Na_2SO_4$.

L'éther est éliminé par distillation sous vide et le résidu brut (8,0 g) est purifié par chromatographie.

L'élution par un mélange chlorure de méthylène -méthanol permet d'obtenir 6,0 g de produit pur sous forme d'une huile de couleur orangée.

Rdt = 95 % CCM : 0,50-0,70 ; S. C.2

RMN : 0,70 (t,3H), 1,70-2,50 (m,6H), 2,00 (s,6H), 3,05-3,50 (m,4H), 7,10-7,80 (m,10H)

IR (film) : 2900, 2750, 1480, 1440, 1280, 1000, 760, 740, 700 $cm^{-1}$

Chlorhydrate

Dans un ballon de 50 ml protégé de l'humidité, on ajoute 3,0 g (8 mmol) du composé précédemment obtenu dans un excès d'éthanol chlorhydrique. Après deux heures de contact sous agitation à la température du laboratoire les solvants sont éliminés par distillation sous vide et le résidu amorphe est repris par 10 ml d'acétate d'éthyle. L'insoluble est filtré, séché sous vide jusqu'à poids constant.

Poids : 3,0 g Rdt : 92 % F : 204°C

Anal. ($C_{22}H_{29}NS_2$, HCl) C,H, Cl, N, S

ETUDES PHARMACOLOGIQUES

Les méthodes pratiquées et les résultats obtenus qui permettent de mettre en évidence les propriétés des composés de l'invention sont reportés dans ce qui suit. Ce sont essentiellement :

- l'activité inhibitrice chez le rat des diarrhées provoquées par l'huile de ricin,
- l'étude de l'activité des composés sur le transit gastrointestinal chez le rat,
- l'étude chez la souris de l'aptitude des composés à provoquer des comportements dits de dépendance.

1 - Activité antidiarrhéïque chez le rat.

Le test est pratiqué en étudiant l'activité des composés à l'essai sur le phénomène diarrhéïque provoqué chez l'animal par l'administration d'huile de ricin.

La méthode consiste à administrer le produit à tester en solution par voie orale et ce, une heure avant l'administration, également par voie orale, de 1,0 ml d'huile de ricin par animal.

Les animaux sont ensuite observés et le nombre de rats qui émettent des selles liquides est déterminé en tout ou rien chaque heure et ce durant les sept heures qui suivent l'administration de l'agent diarrhéïque.

Tel que pratiqué les produits de l'invention ont été administrés à plusieurs doses de façon à pouvoir déterminer leur $DE_{50}$ après 3 heures qui représente la dose efficace en mg/kg de produit permettant d'inhiber à ce temps l'effet de l'huile de ricin chez 50 % des animaux traités. Les résultats sont présentés au tableau 1 qui suit, le Lopéramide (DCI) et la morphine étant étudiés aux titres de produit de comparaison et de produit de référence.

Tableau 1 : Activité antidiarrhéïque des composés.

| Composé | DE 50 mg/Kg à 3 h. |
|---|---|
| Ex. 3.c | 16,0 |
| Ex. 3.f | 6,0 |
| Ex. 9.a | 6,0 |
| Ex. 9.b.2 | 2,0 |
| Ex. 9.c | 15,0 |
| Ex. 9.g | 3,0 |
| Ex. 10 | 24,2 |
| Ex. 12 | 23,1 |
| Lopéramide (DCI) | 0,20 |
| Morphine | 0,83 |

2 - Activité sur le transit intestinal.

L'essai est réalisé selon la technique décrite par Green A.F., J. Pharmacol., 1959, 14, 26-34. Il consiste à déterminer chez le rat la distance parcourue dans l'intestin de l'animal par un repas semi solide après un temps déterminé.

Les produits à l'essai sont administrés par voie orale une heure avant le gavage de l'animal par un repas semi solide contenant comme traceur du charbon végétal. Quinze minutes après l'administration, l'animal est sacrifié et la distance parcourue dans l'intestin par le repas est rapportée à la longueur totale.

Ce rapport, pour les animaux traités, est comparé à celui constaté chez des animaux témoins n'ayant reçu que le repas.

Dans cet essai les produits de l'invention ont été administrés à raison de 30 mg/kg et les résultats exprimés en pourcentages de variation par rapport aux témoins, rapport qui est positif quand le transit est accéléré et négatif lorsqu'il est ralenti ; ils sont présentés au tableau 2 dans lequel pour la morphine et le Lopéramide (DCI) qui sont engagés comme produits de comparaison, les résultats sont exprimés différemment, à savoir par leur DE50 qui est en mg/kg la dose efficace capable de ralentir de 50 % le transit chez l'animal.

Tableau 2 :

Activité sur le transit intestinal chez le rat

| Composé | Dose | Activité |
| --- | --- | --- |
| Ex. 3.c | 30 mg/kg | - 3 % |
| Ex. 3.f | 30 mg/kg | - 17 % |
| Ex. 9.a | 30 mg/kg | - 15 % |
| Ex. 9.b.2 | 30 mg/kg | - 14 % |
| Ex. 9.c | 30 mg/kg | + 2 % |
| Ex. 9.g | 30 mg/kg | - 25 % |
| Ex. 10 | 30 mg/kg | - 6 % |
| Ex. 12 | 30 mg/kg | - 12 % |
| Lopéramide | 1,79 mg/kg | - 50 % |
| Morphine | 10 mg/kg | - 50 % |

Ces résultats montrent que l'effet sur le transit provoqué par les composés de l'invention peut être considéré comme insignifiant ou même nul comme par exemple pour les produits des exemples 3.c et 9.c .

3 - Etude de l'état de "dépendance" provoqué par les composés.

Ce test est réalisé chez la souris et consiste à administrer les composés à l'étude à l'animal par voie orale et en solution sous un volume de 0,4 ml pour 20 g de poids corporel d'animal.

L'étude des composés de l'invention consiste en leur administration par doses successives et croissantes durant deux jours pour atteindre un total de 360 mg/kg, en respectant les séquences suivantes :
t 0 - 10 mg/kg ; t 1 h et t 2 h - 25 mg/kg ; t 4 h et
6 h - 50 mg/kg ; t 24 h et 26 h - 100 mg/kg .

A t 28 h, les animaux recoivent par voie intrapéritonéale 100 mg/kg de naloxone. Ils sont ensuite placés dans des cages et observés durant 30 minutes , ceux qui effectuent des sauts sont considérés être en état d'assuétude et sont comptabilisés.

Les résultats sont exprimés par le nombre d'animaux qui présentent cet état par rapport au nombre total d'animaux du lot d'essai.

Le Lopéramide (DCI) et la morphine essayés dans ce test sont trop toxiques aux doses d'administration précédemment décrites. Des doses inférieures ont donc été administrées pour définir leur DE50 qui est la dose efficace totale en mg/kg sur 26 heures qui provoque un phénomène de dépendance de la moitié des animaux engagés dans l'essai. Ces résultats sont présentés au tableau 3.

Tableau 3 :

Etude du phénomène de dépendance chez la souris

| Composé | Dose | % dépendance |
|---|---|---|
| Ex. 3.f | 360 mg/kg | 0 % |
| Ex. 9.b.2 | 360 mg/kg | 10 % |
| Lopéramide | 75 mg/kg | 50 % |
| Morphine | 53 mg/kg | 50 % |

Cette étude est probante de l'inocuité des composés de l'invention. Ainsi les composés préférés de l'invention ont une activité pratiquement nulle à des doses 5 à 7 fois supérieures à celles ou le Lopéramide (DCI) et la morphine provoquent des phénomènes de dépendance chez 50 % des animaux traités. Ils sont donc particulièrement appropriés à traiter les états diarrhèïques sans pour autant perturber le transit intestinal ni, lors de traitements renouvelés, provoquer d'effets de dépendance.

Les propanamines de l'invention sont présentés sous des formes pharmaceutiques dont les doses unitaires sont habituellement comprises entre 1 et 500 mg et plus particulièrement entre 5 et 200 mg selon l'intensité de l'affection à traiter. Les doses thérapeutiques journalières peuvent être réparties en plusieurs prises dont le total est compris entre 5 et 2000 mg de produit par jour. Cependant une posologie journalière de 50 à 500 mg de produit répartis en deux à quatre prises est généralement satisfaisante.

Les formes pharmaceutiques peuvent être, des comprimés, dragées, capsules, poudres, solutions, suspensions, gels ou suppositoires. Ces formes sont préparées avec les propanamines sous leur forme de base ou bien sous leur forme salifiée.

Généralement dans les formes "solides", le principe actif représente de 5 à 90% en poids du total de la forme terminée alors que les excipients pharmaceutiquement appropriés représentent de 95 à 10%. Pour les formes liquides ou considérées comme telles, la quantité de principe actif est comprise entre 0,1 et 10% en poids de la forme terminée alors que les excipients pharmaceutiquement appropriés en représentent de 99,9 à 90%.

Il est présenté des exemples de formulation et leur mise en oeuvre pour l'administration des produits de l'invention sous des formes pharmaceutiques.

Soluté isotonique injectable

- Formule :

| | |
|---|---|
| Composé de l'exemple 9 b.2 (chlorhydrate) | 10 mg |
| Chlorure de sodium | 9 mg |
| Eau distillée en quantité suffisante pour | 1,0 ml |

- Préparation : Le soluté isotonique est réparti en ampoules de volume approprié qui sont, après scellement, stérilisées par des moyens thermiques connus en soi ou bien le soluté est stérilisé par filtration, réparti en ampoules qui sont ensuite scellées, l'ensemble de ces opérations étant réalisé sous atmosphère stérile.

Dans le dernier cas, on préfère ajouter dans la formule décrite 1 % d'alcool benzylique comme agent bactériostatique soit 10 mg de cet alcool par ml de soluté.

Comprimés

- Formule

| | |
|---|---|
| Substance active de l'exemple 3.f | 10,0 à 50,0 mg |
| Polyvinylpyrrolidone | 20,0 mg |
| Carboxyméthylamidon | 8,0 mg |
| Stéarate de magnésium | 2,0 mg |
| Silice colloïdale | 0,4 mg |
| Lactose (en quantité suffisante pour) | 200,0 mg |

- Préparation : Le principe actif est mélangé au lactose puis granulé avec la polyvinylpyrrolidone en solution. Les grains sont séchés et tamisés sur une grille d'ouverture de 1 mm. Le carboxyméthylamidon est mélangé à la silice colloïdale puis ajouté aux granulés. On mélange ensuite intimement avec le stéarate de magnésium puis comprime à raison de 200,0 mg par unité.

Suspension gélifiée buvable

- Formule

| | |
|---|---|
| Substance active de l'exemple 9.b.2 (chlorhydrate) | 0,20 à 0,50 mg |
| Hydroxypropylcellulose | 2,00 g |
| Saccharinate de sodium | 0,01 g |
| Sirop de sorbitol à 70 % (p/v) | 25,00 g |
| Arôme naturel de fraise | 0,50 g |
| Conservateur | 0,10 g |
| Eau purifiée en quantité suffisante pour | 100,00 g |

- Préparation

Les conservateurs et le saccharinate de sodium sont dissous dans l'eau, puis, sous agitation, on ajoute en la dispersant l'hydroxypropylcellulose. L'agitation est maintenue jusqu'à l'obtention d'un gel auquel, toujours sous agitation, on ajoute le sirop de sorbitol puis finalement l'arôme.

## Revendications

**Revendications pour les Etats contractants suivants : AT, BE, CH, DE, DK, FR, GB, IT, LI, LU, NL, SE**

**1.** Propanamines de formule générale (I) :

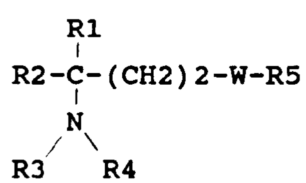

(I)

dans laquelle :

R1 est un radical phényle, éventuellement mono, di ou trisubstitué de manière identique ou différente par des atomes d'halogène, des radicaux alkyle de $C_1$ à $C_4$, haloalkyle de $C_1$ à $C_4$, alkoxy de $C_1$ à $C_4$,
ou est un radical hétéroaryle monocyclique de 5 ou 6 chaînons dans lequel l'unique hétéroatome est l'azote, l'oxygène ou le soufre,

R2 est un radical alkyle de $C_1$ à $C_4$,

R3 et R4 sont un atone d'hydrogène, un radical alkyle de $C_1$ à $C_4$, alkényl de $C_1$ à $C_4$, cycloalkylalkyle (avec cycloalkyle de $C_3$ à $C_7$ et alkyle de $C_1$ à $C_4$), et sont différents ou identiques sans toutefois être tous deux cycloalkylalkyle,
ou forment ensemble, avec l'atome d'azote auquel ils sont reliés, un hétérocycle saturé comprenant un seul hétéroatome et constitué de 5 ou 6 chaînons,

R5 est un radical cycloalkyle de 5 à 7 chaînons, un radical phényle ou un radical hétéroaryle monocyclique de 5 ou 6 chaînons dans lequel l'unique hétéroatome est l'azote, l'oxygène ou le soufre et qui sont éventuellement mono, di ou trisubstitués de façon identique ou différente par des atomes d'halogène, des radicaux alkyle de $C_1$ à $C_4$, haloalkyle de $C_1$ à $C_4$ ou alkoxy $C_1$ à $C_4$,
et W représente un groupe =CH-QH,
ou un hétérocycle =C[Q-(CH2)n-Q]
ou un groupe =C=Q,
dans lesquels
Q est un atome d'oxygène ou de soufre,
n a pour valeur 2 ou 3,
W n'étant =C=Q que lorsque R3 et R4 ne sont pas tous deux l'hydrogène,
et leurs sels d'addition avec des acides.

2. Propanamines selon la revendication 1, caractérisées en ce que R1 et R5 sont des radicaux phényle éventuellement mono, di ou trisubstitués.

3. Propanamines selon la revendication 1 ou 2, caractérisées en ce que W représente un groupe =C[Q-(CH2)n-Q] avec Q représentant un atome d'oxygène.

4. Propanamines selon la revendication 3, caractérisées en ce que n est égal à 2.

5. Propanamines selon la revendication 2, caractérisées en ce que W représente un groupe carbonyle.

6. Propanamines selon l'une des revendications 1 à 5, caractérisées en ce que R3 est méthyle et R4 est l'hydrogène.

7. Propanamines selon l'une des revendications 1 à 5, caractérisées en ce que R3 et R4 sont chacun un radical méthyle.

8. Le2-[3-N-méthylamino-3-(4-trifluorométhylphényl)-pentan-1-yl]-2-(3,4,5-triméthoxyphényl)-1,3,dioxolane, et ses sels d'addition avec des acides.

9. Le 4-N,N-diméthylamino-4-(4-méthylphényl)-1-(3,4,5-triméthoxyphényl)-hexan-1-one, et ses sels d'addition avec des acides.

10. Procédé de préparation des propanamines de formule (I) telle que définie à la revendication 1, caractérisé en ce qu'il consiste A. pour préparer une propanamine dans laquelle R3 et R4 sont l'hydrogène,
a) quand W représente le groupe =CH-QH ce qui correspond à une propanamine de formule (I.A.1)

```
    R1          QH
    |           |
R2-C-(CH2)2-CH-R5          (I.A.1)
    |
    NH2
```

à réduire un composé nitré (III)

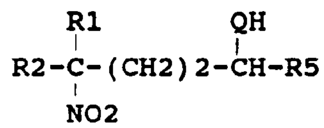

(III)

par hydrogénation catalysée par un élément du groupe VIII de la table périodique des éléments et

b) quand W représente le groupe =C[Q-(CH2)n-Q] ce qui correspond à une propanamine de formule (I.D.1)

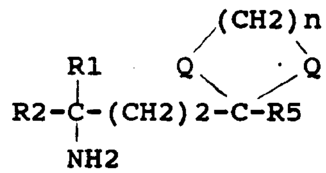

(I.D.1)

à réduire un composé nitré (IV)

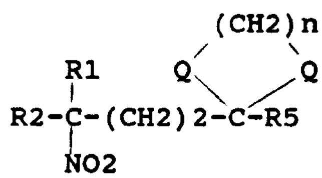

(IV)

par hydrogénation catalysée par un élément du groupe VIII de la table périodique des éléments,

B. pour préparer une propanamine dans laquelle R4 est l'hydrogène et est différent de R3,

a) quand W représente un groupe =CH-QH, ce qui correspond à une propanamine (I.A.2),

à alkyler une propanamine de formule (I.A.1) par un réactif d'alkylation R3-X1 dans lequel X1 est un atome d'halogène tel que le chlore, le brome ou l'iode, ou bien, à réduire par un hydrure métallique ou organométallique (Hm) : M1(t)M2H(r)RX(s) dans lequel M1 est un métal alcalin, M2 est un élément du groupe III de la classification périodique, RX est un groupe carbonitrile ou un radical alkyle ou alkoxy de $C_1$ à $C_4$, (t) est égal à zéro ou un, (r) est égal à un, trois ou quatre et (s) est égal à zéro, un, trois ou quatre et dans lequel (r) + (s) - (t) = 3, et de formule (Hm.2) dans laquelle M2 est l'aluminium ou, lorsque M2 est le bore (r) vaut trois, (s) et (t) ayant pour valeur zéro, un N-carboxamide de formule (V.A.2.) de formule :

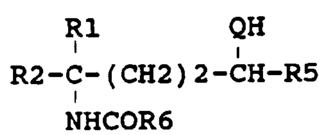

(V.A.2.)

dans laquelle R6 est l'hydrogène ou un radical homologue inférieur d'un atome de carbone à R3 (R3 = -CH2-R6),

b) quand W représente le groupe =C[Q-(CH2)n-Q], ce qui correspond à une propanamine (I.D.2), à alkyler une propanamine de formule (I.D.1) par un réactif d'alkylation R3-X1 dans lequel X1 est un atome d'halogène tel que le chlore, le brome ou l'iode,

ou bien à réduire par un hydrure métallique ou organométallique (Hm.2) précédemment défini un N-carboxamide de formule (V.D.2)

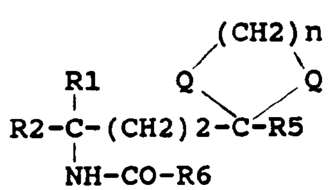

(V.D.2)

c) quand W représente le groupe =C=Q lorsque Q représente l'oxygène, ce qui correspond à une propanamine (I.C.2/O) de formule

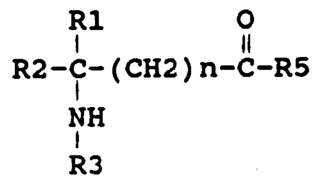

(I.C.2/O)

à oxyder par un diatome ou un ion polyatomique dont le potentiel normal redox E° est supérieur à 0,60V une propanamine (I.A.2) dans laquelle Q est l'oxygène, ou bien à hydrolyser par une solution acide lorsque dans le groupe -Q(CH2)nQ- Q est l'oxygène et par une solution oxydante lorsque dans le groupe -Q(CH2)nQ- Q est le soufre, une propanamine (I.D.2),

d) quand W représente le groupe =C=Q lorsque Q représente le soufre, ce qui correspond à une propanamine (I.C.2/S) de formule

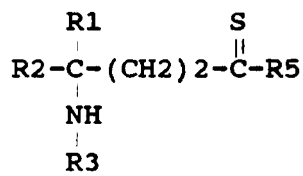

(I.C.2/S)

à faire réagir une propanamine (I.C.2/O) avec le pentasulfure de phosphore,
ou bien à effectuer une réaction de thio-oxo substitution entre un composé (VI.1) de formule

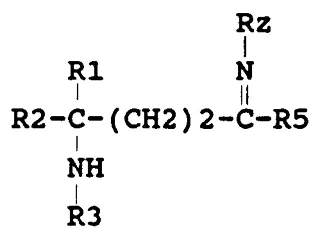

(VI.1)

dans laquelle Rz est l'hydrogène, un radical amine (-NH2), hydroxyle (-OH), un radical alkyle inférieur ou un radical phényle,
et l'hydrogène sulfuré, le sulfure de carbone, le monochlorure de soufre, un acide S-alkylthioïque dans lequel le radical alkyle comprend de un à cinq atomes de carbone en chaîne linéaire ou ramifiée ou un acide S-phénylthioïque comme l'acide S-thiobenzoïque, C. pour préparer une propanamine (I) dans laquelle R3 et R4 ne sont pas l'hydrogène,
C.1. et, lorsque R3 et R4 sont identiques et représentent des radicaux alkyle ou alkényle de $C_1$ à $C_4$,
a) lorsque W représente un groupe =CH-QH, ce qui correspond à une propanamine (I.A.3) ou un groupe =C[Q-(CH2)n-Q] dans une propanamine (I.D.3),

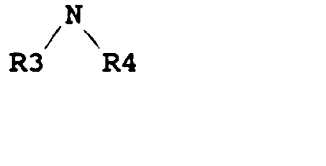

(I.A.3)

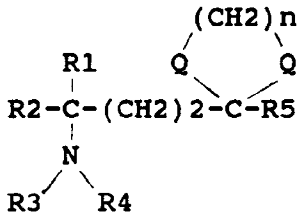

(I.D.3)

à dialkyler une propanamine de formule (I.A.1) ou de formule (I.D.1) par un aldéhyle R7-CHO dans lequel R7 est l'hydrogène ou un radical alkyle ou alkényle, homologue comprenant un atome de carbone de moins que R3 et R4 (R3=R3=CH2-R7), et avec un agent de réduction qui est un hydrure organométallique (Hm) pour lequel on préfère que M2 soit le bore et Rx est un groupe carbonitrile (Hm.3) ou l'acide formique ou un de ses sels,
b) lorsque W représente un groupe =C=O, ce qui correspond à une propanamine (I.C.3/O)

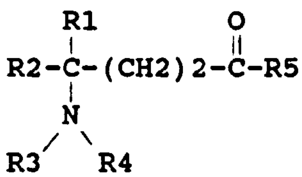

(I.C.3/O)

à oxyder une propanamine correspondante dans laquelle W est un groupe =CH-OH, par un diatome ou un ion polyatomique dont le potentiel normal redox E° est supérieur à 0,60V,
ou bien à hydrolyser une propanamine (I.D.3) par une solution acide lorsque dans le groupe [Q-(CH2)n-Q] Q est l'oxygène et par une solution oxydante, lorsque dans le groupe [Q-(CH2)n-Q] Q est le soufre, une propanamine (I.D.2),
c) lorsque W représente un groupe =C=S, ce qui correspond à une propanamine (I.C.3/S)

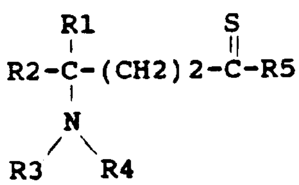

(I.C.3/S)

à faire réagir une propanamine (I.C.3/O) avec le pentasulfure de phosphore,
ou bien à effectuer une réaction de thio-oxo substitution entre un composé (VI.2) de formule

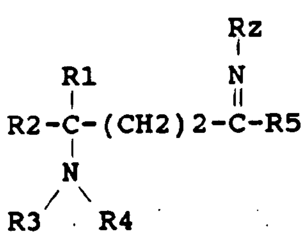

(VI.2)

dans laquelle Rz est l'hydrogène, un radical amine (-NH2), hydroxyle (-OH), un radical alkyle $C_1$ à $C_4$ ou un radical phényle,
et l'hydrogène sulfuré, le sulfure de carbone, le mono chlorure de soufre, un acide S-alkylthioïque dans lequel le radical alkyle comprend de un à cinq atomes de carbone en chaîne linéaire ou ramifiée ou un acide S-phénylthioïque comme l'acide S-thiobenzoïque,
C.2. et, pour préparer une propanamine (I) dans laquelle R3 et R4 forment ensemble et avec l'atome d'azote auquel ils sont liés un hétérocycle saturé comprenant de 5 à 6 chaînons,
à dialkyler une propanamine (I.D.1) par un réactif dihalogéné X3-(CH2)q-X4 dans lequel X3 et X4 identiques ou différents sont le chlore, le brome ou l'iode et q a pour valeur 4 ou 5, pour obtenir une propanamine (I.D.3) de formule

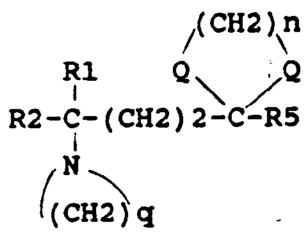

(I.D.3)

et à préparer une propanamine (I.C.3/O) correspondantes en hydrolysant par un acide ou un oxydant dans les condition précédemment définies une propanamine (I.D.3),
et à préparer une propanamine correspondante (I.C.3/S) en faisant réagir une propanamine (I.C.3/O) avec le pentasulfure de phosphore ou bien en préparant un composé intermédiaire (VI.2) que l'on engage dans une réaction de thio oxo substitution avec les réactifs précédemment cités,
et à préparer une propanamine (I.A.3/O) par réduction d'une propanamine (I.C.3/O), et une propanamine (I.A.3/S) par réduction d'une propanamine (I.C.3/S) avec un hydrure métallique-ou organométallique (Hm) dans lequel on préfère que M2 soit le bore et M1 le sodium,
C.3. pour préparer une propanamine (I) dans laquelle R3 et R4, différents l'un de l'autre, ne sont pas l'hydrogène et,
a) lorsque W représente un groupe =C=Q, ce qui correspond à une propanamine (I.C.3) ou un groupe =C[Q-(CH2)n-Q], ce qui correspond à une propanamine (I.D.3)

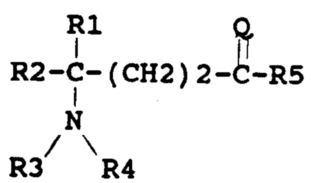

(I.C.3)

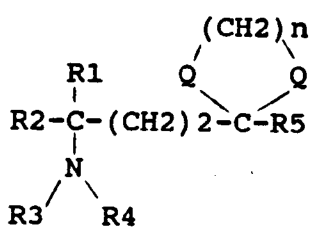

(I.D.3)

à alkyler une propanamine de formule (I.D.2) ou de formule (I.C.2) dans laquelle R4 est l'hydrogène par un réactif d'alkylation R4X5 dans lequel X5 est un atome de chlore, de brome ou d'iode et,
b) lorsque W représente un groupe =CH-QH) ce qui correspond à une propanamine (I.A.3) ou un groupe =C[Q-(CH2)n-Q] ce qui correspond à une propanamine (I.D.3), à faire réagir une propanamine (I.A.2) ou une propanamine (I.D.2) avec un aldéhyde R7-CHO homologue inférieur d'un atome de carbone à R4 (R4=-CH2-R7) et un hydrure réducteur (Hm) de formule (Hm.3) précédemment défini,
ou bien à réduire par un hydrure métallique ou organo nétallique (Hm), de formule (Hm.2) précédemment définie un N-carboxamide de formule (V.A.3) ou un carboxamide de formule (V.D.3)

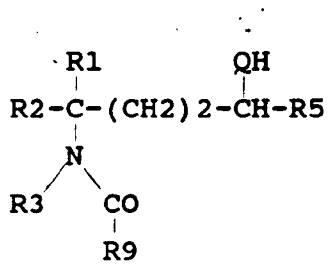

(V.A.3)

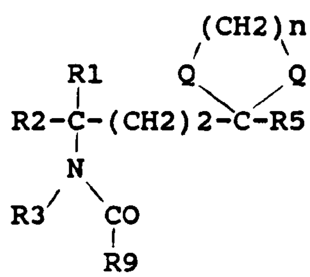

(V.D.3)

dans lequel R9 est l'hydrogène ou un radical carboné homologue inférieur d'un atome de carbone à R4 (R4=-CH2-R9), ou bien à substituer le radical carbonitrile d'un aminonitrile de formule (VII.1) ou de formule (VII.2)

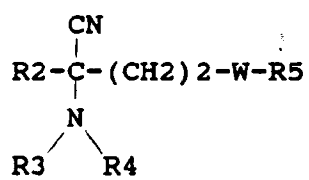

(VII.1)

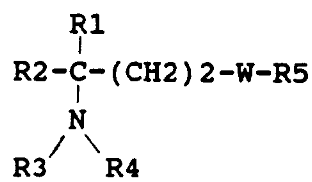

(VII.2)

par les radicaux carbonés R1 et R2 des réactifs organométalliques R1-M-X7 et R2-M-X8 dans lesquels
R1 a les significations indiquées pour les propanamines (I) à l'exception de radicaux halogénés,
R2 est alkyle de $C_1$ à $C_4$,
M est un atome métallique bivalent choisi parmi le magnésium, le cadmium et le zinc,
X7 et X8 sont des atomes d'halogène choisis entre le chlore, le brome ou l'iode, et
C.4. pour préparer des propanamines (I) dans lesquelles ni R3 ni R4 ne sont de l'hydrogène,
a) quand W est =CH-Q, ce qui correspond à une propanamine de formule (I.A.3)

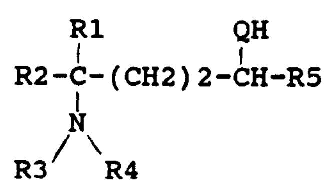

(I.A.3)

à réduire, par un hydrure métallique ou organo métallique (Hm) dans lequel M2 est de préférence le bore et M1 le sodium, une propanamine de formule (I.C.3),

b) et, lorsque W est =C=O ce qui correspond à une propanamine de formule (I.C.3/O), à oxyder, par des systèmes oxydo-réducteurs ioniques mono ou polyatomiques et dont le potentiel normal E° à 20°C est supérieur à 0,60 V, une propanamine (I.A.3/O), ou bien, à hydrolyser, par une solution acide le groupe W lorsque Q est l'oxygène ou par une solution oxydante le groupe W lorsque Q est le soufre, une propanamine (I.D.3),

c) et, lorsque W est =C=S, ce qui correspond à une propanamine (I.C.3/S)

à effectuer une réaction de thioxo substitution avec un intermédiaire (VI.2) de formule

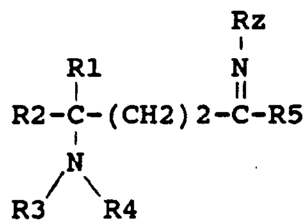

(VI.2)

d) et lorsque W est un groupe =C[Q-(CH2)n-Q], ce qui correspond à une propanamine de formule (I.D.3)

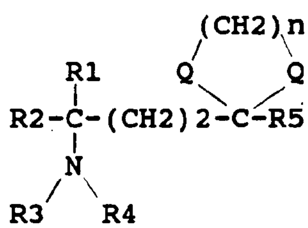

(I.D.3)

à condenser une propanamine (I.C.3) avec une réactif bifonctionnel HQ-(CH2)n-QH dans lequel n a pour valeur 2 ou 3, Q étant le soufre ou l'oxygène.

**11.** Médicament caractérisé en ce qu'il comprend une propanamine (I) telle que définie à l'une des revendications 1 à 9.

**12.** Utilisation d'une propanamine (I), telle que définie à l'une des revendications 1 à 9, pour l'obtention d'un médicament destiné au traitement des états diarrhéïques.

**13.** Intermédiaires de formule (XX)

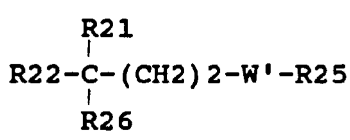

(XX)

dans laquelle

R21 est un radical phényle éventuellement mono, di ou trisubstitué de manière identique ou différente par des atomes d'halogène, des radicaux alkyle de $C_1$ à $C_4$, haloalkyle de $C_1$ à $C_4$, alkoxy de $C_1$ à $C_4$, ou est un radical hétéroaryle monocyclique de 5 ou 6 chaînons dans lequel l'unique hétéroatome est l'azote, l'oxygène ou le soufre, ou est un radical carbonitrile lorsque R22 est alkyle de $C_1$ à $C_4$,

R22 est, quand R21 est un radical phényle ou hétéroaryle comme défini ci-dessus, un radical alkyle de $C_1$ à $C_4$ ou un radical carbonitrile,

R25 est un radical cycloalkyle de 5 à 7 chaînons, un radical phényle ou un radical hétéroaryle monocyclique de 5 à 6 chaînons dans lequel l'unique hétéroatome est l'azote, l'oxygène ou le soufre

et qui sont éventuellement mono, di ou trisubstitués de façon identique ou différente par des atomes d'halogène, des radicaux alkyle de $C_1$ à $C_4$, haloalkyle de $C_1$ à $C_4$ ou alkoxy de $C_1$ à $C_4$,

R26 est un radical nitro lorsque ni R21 ni R22 ne sont carbonitrile, ou lorsque l'un de R21 ou de R22 est carbonitrile, est un groupe -N(R23)R24 dans lequel R23 et R24 sont un radical alkyle de $C_1$ à $C_4$, alkényle de $C_1$ à $C_4$, cycloalkylalkyle (avec cycloalkyle de $C_3$ à $C_7$ et alkyle de $C_1$ à $C_4$), différents ou identiques sans toutefois être tous deux cycloalkylalkyle, ou R23 et R24 forment ensemble, avec l'atome d'azote auquel ils sont reliés, un hétérocycle saturé comprenant un seul hétéroatome et constitué de 5 à 6 chaînons, et

W' représente un groupe =CH-QH ou un hétérocycle =C[Q-(CH2)n-Q] quand l'un de R21 ou R22 sont carbonitrile, et dans lesquels Q est un atome d'oxygène ou de soufre, ou un groupe =C=Q lorsque R26 représente un groupe nitro, Q étant alors un atome d'oxygène, de soufre ou un groupe N-Ry dans lequel Ry est l'hydrogène ou un radical alkyle de $C_1$ à $C_4$, n a pour valeur 2 ou 3.

**Revendications pour l'Etat contractant suivant : ES**

**1.** Procédé de préparation de propanamines de formule générale (I)

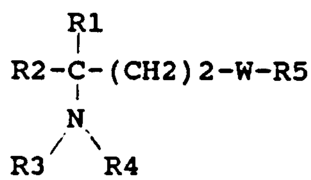

(I)

dans laquelle:

R1 est un radical phényle, éventuellement mono, di ou trisubstitué de manière identique ou différente par des atomes d'halogène, des radicaux alkyle de $C_1$ à $C_4$, haloalkyle de $C_1$ à $C_4$, alkoxy de $C_1$ à $C_4$, ou est un radical hétéroaryle monocyclique de 5 ou 6 chaînons dans lequel l'unique hétéroatome est l'azote, l'oxygène ou le soufre,

R2 est un radical alkyle de $C_1$ à $C_4$,

R3 et R4 sont un atome d'hydrogène, un radical alkyle de $C_1$ à $C_4$, alkényl de $C_1$ à $C_4$, cycloalkylalkyle (avec cycloalkyle de $C_3$ à $C_7$ et alkyle de $C_1$ à $C_4$), et sont différents ou identiques sans toutefois être tous deux cycloalkylalkyle, ou forment ensemble, avec l'atome d'azote auquel ils sont reliés, un hétérocycle saturé comprenant un seul hétéroatome et constitué de 5 ou 6 chaînons,

R5 est un radical cycloalkyle de 5 à 7 chaînons, un radical phényle ou un radical hétéroaryle monocyclique de 5 ou 6 chaînons dans lequel l'unique hétéroatome est l'azote, l'oxygène ou le soufre et qui sont éventuellement mono, di ou trisubstitués de façon identique ou différente par des atomes d'halogène, des radicaux alkyle de $C_1$ à $C_4$, haloalkyle de $C_1$ à $C_4$ ou alkoxy de $C_1$ à $C_4$,
et W représente un groupe =CH-QH, (groupe I.A)
ou un hétérocycle =C[Q-(CH2)n-Q] (groupe I.D)
ou un groupe =C=Q (groupe I.C),
dans lesquels
Q est un atome d'oxygène ou de soufre,
n a pour valeur 2 ou 3,
W n'étant =C=Q que lorsque R3 et R4 ne sont pas tous deux l'hydrogène,
et leurs sels d'addition avec des acides,
caractérisé en ce qu'il consiste

A. pour préparer une propanamine dans laquelle R3 et R4 sont l'hydrogène,

a) quand W représente le groupe =CH-QH ce qui correspond à une propanamine de formule (I.A.1)

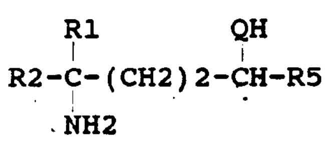

(I.A.1)

à réduire un composé nitré (III)

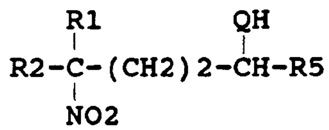 (III)

par hydrogénation catalysée par un élément du groupe VIII de la table périodique des éléments et
b) quand W représente le groupe =C[Q-(CH2)n-Q] ce qui correspond à une propanamine de formule (I.D.1)

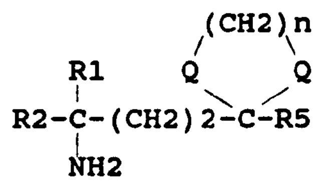 (I.D.1)

à réduire un composé nitré (IV)

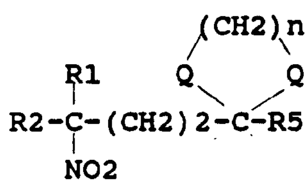 (IV)

par hydrogénation catalysée par un élément du groupe VIII de la table périodique des éléments,
B. pour préparer une propanamine dans laquelle R4 est l'hydrogène et est différent de R3,
a) quand W représente un groupe =CH-QH, ce qui correspond à une propanamine (I.A.2),
à alkyler une propanamine de formule (I.A.1) par un réactif d'alkylation R3-X1 dans lequel X1 est un atome d'halogène tel que le chlore, le brome ou l'iode, ou bien, à réduire par un hydrure métallique ou organométallique (Hm) : M1(t)M2H(r)RX(s) dans lequel M1 est un métal alcalin, M2 est un élément du groupe III de la classification périodique, RX est un groupe carbonitrile ou un radical alkyle ou alkoxy de $C_1$ à $C_4$, (t) est égal à zéro ou un, (r) est égal à un, trois ou quatre et (s) est égal à zéro, un, trois ou quatre et dans lequel (r) + (s) - (t) = 3, et de formule (Hm.2) dans laquelle M2 est l'aluminium ou, lorsque M2 est le bore (r) vaut trois, (s) et (t) ayant pour valeur zéro, un N-carboxamide de formule (V.A.2.) de formule :

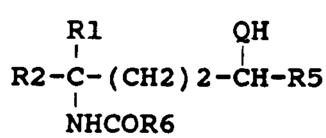 (V.A.2.)

dans laquelle R6 est l'hydrogène ou un radical homologue inférieur d'un atome de carbone à R3 (R3 =-CH2-R6),
b) quand W représente le groupe =C[Q-(CH2)n-Q], ce qui correspond à une propanamine (I.D.2), à alkyler une propanamine de formule (I.D.1) par un réactif d'alkylation R3-X1 dans lequel X1 est un atome d'halogène tel que le chlore, le brome ou l'iode, ou bien à réduire par un hydrure métallique ou organométallique (Hm.2) précédemment défini un N-carboxamide de formule (V.D.2)

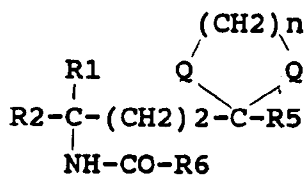 (V.D.2)

c) quand W représente le groupe =C=Q lorsque Q représente l'oxygène, ce qui correspond à une propanamine (I.C.2/O) de formule

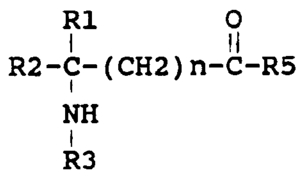 (I.C.2/O)

à oxyder par un diatone ou un ion polyatomique dont le potentiel normal redox E° est supérieur à 0,60V une propanamine (I.A.2) dans laquelle Q est l'oxygène,
ou bien à hydrolyser par une solution acide lorsque dans le groupe -Q(CH2)nQ- Q est l'oxygène et par une solution oxydante lorsque dans le groupe -Q(CH2)nQ- Q est le soufre, une propanamine (I.D.2),
d) quand W représente le groupe =C=Q lorsque Q représente le soufre, ce qui correspond à une propanamine (I.C.2/S) de formule

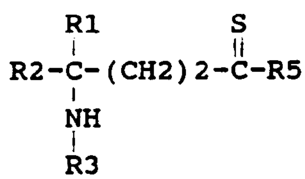 (I.C.2/S)

à faire réagir une propanamine (I.C.2/O) avec le pentasulfure de phosphore,
ou bien à effectuer une réaction de thio-oxo substitution entre un composé (VI.1) de formule

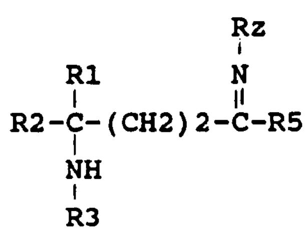 (VI.1)

dans laquelle Rz est l'hydrogène, un radical amine (-NH2), hydroxyle (-OH), un radical alkyle de $C_1$ à $C_4$ ou un radical phényle,
et l'hydrogène sulfuré, le sulfure de carbone, le monochlorure de soufre, un acide S-alkylthioïque dans lequel le radical alkyle comprend de un à cinq atomes de carbone en chaîne linéaire ou ramifiée ou un acide S-phénylthioïque comme l'acide S-thiobenzoïque,
C. pour préparer une propanamine (I) dans laquelle R3 et R4 ne sont pas l'hydrogène,
C.1. et, lorsque R3 et R4 sont identiques et représentent des radicaux alkyle ou alkényle de $C_1$ à $C_4$,
a) lorsque W représente un groupe =CH-QH, ce qui correspond à une propanamine (I.A.3) ou un groupe =C[Q-(CH2)n-Q] dans une propanamine (I.D.3),

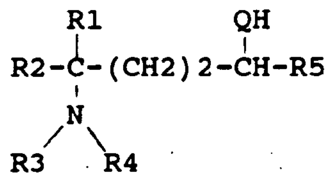

(I.A.3)

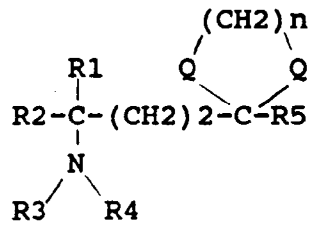

(I.D.3)

à dialkyler une propanamine de formule (I.A.1) ou de formule (I.D.1) par un aldéhyle R7-CHO dans lequel R7 est l'hydrogène ou un radical alkyle ou alkényle, homologue comprenant un atone de carbone de moins que R3 et R4 (R3=R3=CH2-R7), et avec un agent de réduction qui est un hydrure organo-métallique (Hm) pour lequel on préfère que M2 soit le bore et Rx est un groupe carbonitrile (Hm.3) ou l'acide formique ou un de ses sels,

b) lorsque W représente un groupe =C=O, ce qui correspond à une propanamine (I.C.3/O)

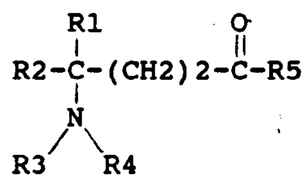

(I.C.3/O)

à oxyder une propanamine correspondante dans laquelle W est un groupe =CH-OH, par un diatome ou un ion polyatomique dont le potentiel normal redox E° est supérieur à 0,60V,
ou bien à hydrolyser une propanamine (I.D.3) par une solution acide lorsque dans le groupe [Q-(CH2)n-Q] Q est l'oxygène et par une solution oxydante, lorsque dans le groupe [Q-(CH2)n-Q] Q est le soufre, une propanamine (I.D.2),

c) lorsque W représente un groupe =C=S, ce qui correspond à une propanamine (I.C.3/S)

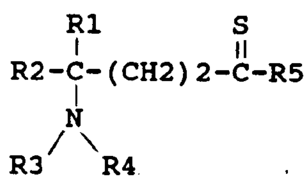

(I.C.3/S)

à faire réagir une propanamine (I.C.3/O) avec le pentasulfure de phosphore,
ou bien à effectuer une réaction de thio-oxo substitution entre un composé (VI.2) de formule

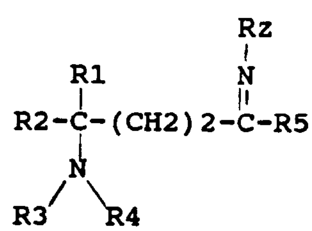

(VI.2)

dans laquelle Rz est l'hydrogène, un radical amine (-NH2), hydroxyle (-OH), un radical alkyle de $C_1$ à $C_4$

ou un radical phényle,
et l'hydrogène sulfuré, le sulfure de carbone, le mono chlorure de soufre, un acide S-alkylthioïque dans lequel le radical alkyle conprend de un à cinq atomes de carbone en chaîne linéaire ou ramifiée ou un acide S-phénylthioïque comme l'acide S-thiobenzoïque,
C.2. et, pour préparer une propanamine (I) dans laquelle R3 et R4 forment ensemble et avec l'atome d'azote auquel ils sont liés un hétérocycle saturé comprenant de 5 à 6 chaînons,
à dialkyler une propanamine (I.D.1) par un réactif dihalogéné X3-(CH2)q-X4 dans lequel X3 et X4 identiques ou différents sont le chlore, le brome ou l'iode et q a pour valeur 4 ou 5, pour obtenir une propanamine (I.D.3) de formule

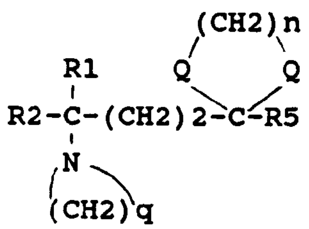

(I.D.3)

et à préparer une propanamine (I.C.3/O) correspondantes en hydrolysant par un acide ou un oxydant dans les condition précédemment définies une propanamine (I.D.3),
et à préparer une propanamine correspondante (I.C.3/S) en faisant réagir une propanamine (I.C.3/O) avec le pentasulfure de phosphore ou bien en préparant un composé intermédiaire (VI.2) que l'on engage dans une réaction de thio oxo substitution avec les réactifs précédemment cités,
et à préparer une propanamine (I.A.3/O) par réduction d'une propanamine (I.C.3/O), et une propanamine (I.A.3/S) par réduction d'une propanamine (I.C.3/S) avec un hydrure métallique ou organométallique (Hm) dans lequel on préfère que M2 soit le bore et M1 le sodium,
C.3. pour préparer une propanamine (I) dans laquelle R3 et R4 différents l'un de l'autre, ne sont pas l'hydrogène et,

a) lorsque W représente un groupe =C=Q, ce qui correspond à une propanamine (I.C.3) ou un groupe =C[Q-(CH2)n-Q], ce qui correspond à une propanamine (I.D.3)

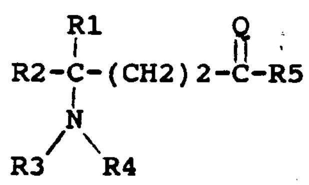

(I.C.3)

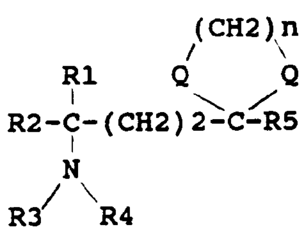

(I.D.3)

à alkyler une propanamine de formule (I.D.2) ou de formule (I.C.2) dans laquelle R4 est l'hydrogène par un réactif d'alkylation R4X5 dans lequel X5 est un atome de chlore, de brome ou d'iode et,

b) lorsque W représente un groupe =CH-QH) ce qui correspond à une propanamine (I.A.3) ou un groupe =C[Q-(CH2)n-Q] ce qui correspond à une propanamine (I.D.3), à faire réagir une propanamine (I.A.2) ou une propanamine (I.D.2) avec un aldéhyde R7-CHO homologue inférieur d'un atome de carbone à R4 (R4=-CH2-R7) et un hydrure réducteur (Hm) de formule (Hm.3) précédemment défini, ou bien à réduire par un hydrure métallique ou organo métallique (Hm) de formule (Hm.2) précédemment défini un N-carboxamide de formule (V.A.3) ou un carboxamide de formule (V.D.3)

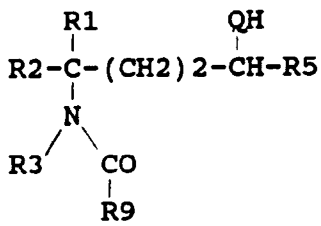

(V.A.3)

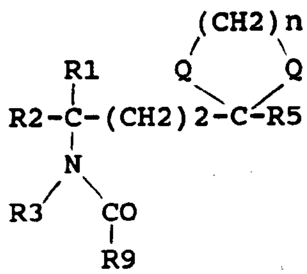

(V.D.3)

dans lequel R9 est l'hydrogène ou un radical carboné homologue inférieur d'un atome de carbone à R4 (R4=-CH2-R9), ou bien à substituer le radical carbonitrile d'un aminonitrile de formule (VII.1) ou de formule (VII.2)

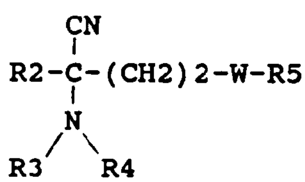

(VII.1)

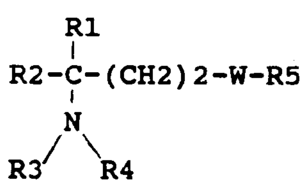

(VII.2)

par les radicaux carbonés R1 et R2 des réactifs organométalliques R1-M-X7 et R2-M-X8 dans lesquels
R1 a les significations indiquées pour les propanamines (I) à l'exception de radicaux halogénés,
R2 est alkyle de $C_1$ à $C_4$,
M est un atome métallique bivalent choisi parmi le magnésium, le cadmium et le zinc,
X7 et X8 sont des atomes d'halogène choisis entre le chlore, le brome ou l'iode, et
C.4. pour préparer des propanamines (I) dans lesquelles ni R3 ni R4 ne sont de l'hydrogène,
a) quand W est =CH-QH, ce qui correspond à une propanamine de formule (I.A.3)

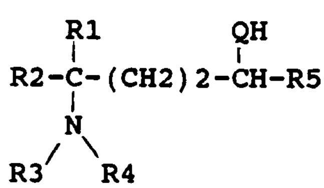

(I.A.3)

à réduire, par un hydrure métallique ou organo métallique (Hm) dans lequel M2 est de préférence le bore et M1 le sodium, une propanamine de formule (I.C.3),
b) et, lorsque W est =C=O ce qui correspond à une propanamine de formule (I.C.3/O), à oxyder, par des systèmes oxydo-réducteurs ioniques mono ou polyatomiques et dont le potentiel normal E° à 20°C est supérieur à 0,60 V, une propanamine (I.A.3/O),

ou bien, à hydrolyser, par une solution acide le groupe W lorsque Q est l'oxygène ou par une solution oxydante le groupe W lorsque Q est le soufre, une propanamine (I.D.3),
c) et, lorsque W est =C=S, ce qui correspond à une propanamine (I.C.3/S)
à effectuer une réaction de thioxo substitution avec un intermédiaire (VI.2) de formule

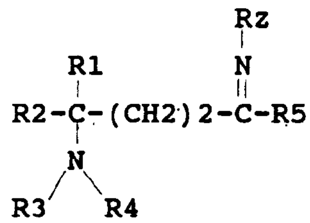

(VI.2)

d) et, lorsque W est un groupe =C[Q-(CH2)n-Q], ce qui correspond à une propanamine de formule (I.D.3)

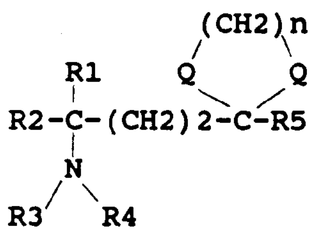

(I.D.3)

à condenser une propanamine (I.C.3) avec une réactif bifonctionnel HQ-(CH2)n-QH dans lequel n a pour valeur 2 ou 3, Q étant le soufre ou l'oxygène.

2. Procédé suivant la revendication 1, caractérisé en ce que R1 et R5 sont des radicaux phényle éventuellement mono, di ou trisubstitués.

3. Procédé suivant la revendication 1 ou 2, caractérisé en ce que W représente un groupe =C[Q-(CH2)n-Q], Q étant un atome d'oxygène.

4. Procédé suivant la revendication 3, caractérisé en ce que n est égal à 2.

5. Procédé suivant la revendication 2, caractérisé en ce que W représente un groupe carbonyle.

6. Procédé suivant l'une des revendications 1 à 5, caractérisé en ce que R3 est méthyle et R4 est hydrogène.

7. Procédé suivant l'une des revendications 1 à 5, caractérisé en ce que R3 et R4 sont chacun un radical méthyle.

8. Procédé suivant la revendication 1, caractérisé en ce que R1 est un radical trifluorométhylphényle, R2 est éthyle, R3 est hydrogène, R4 est méthyle, W est =C[O-(CH2)2-O], et R5 est 3,4,5-triméthoxyphényle.

9. Procédé suivant la revendication 1, caractérisé en ce que R1 est 4-méthylphényle, R2 est éthyle, R3 et R4 sont chacun méthyle, W est =C=O, et R5 est 3,4,5-triméthoxyphényle.

10. Procédé de préparation d'un médicament, caractérisé en ce qu'il consiste à mélanger une propanamine de formule (I) telle que définie à l'une des revendications 1 à 9, à un excipient pharmaceutiquement approprié.

11. Utilisation d'une propanamine (I) telle que définie à l'une des revendications 1 à 9, pour l'obtention d'un médicament destiné au traitement des états diarrhéïques.

**Revendications pour l'Etat contractant suivant : GR**

1. Propanamines de formule générale (I) :

```
        R1
        |
    R2-C-(CH2)2-W-R5
        |
        N                      (I)
       / \
    R3    R4
```

dans laquelle :

R1 est un radical phényle, éventuellement mono, di ou trisubstitué de manière identique ou différente par des atomes d'halogène, des radicaux alkyle de $C_1$ à $C_4$, haloalkyle de $C_1$ à $C_4$, alkoxy de $C_1$ à $C_4$,
ou est un radical hétéroaryle monocyclique de 5 ou 6 chaînons dans lequel l'unique hétéroatome est l'azote, l'oxygène ou le soufre,

R2 est un radical alkyle de $C_1$ à $C_4$,

R3 et R4 sont un atome d'hydrogène, un radical alkyle de $C_1$ à $C_4$, alkényl de $C_1$ à $C_4$, cycloalkylalkyle (avec cycloalkyle de $C_3$ à $C_7$ et alkyle de $C_1$ à $C_4$), et sont différents ou identiques sans toutefois être tous deux cycloalkylalkyle,
ou forment ensemble, avec l'atome d'azote auquel ils sont reliés, un hétérocycle saturé comprenant un seul hétéroatome et constitué de 5 à 6 chaînons,

R5 est un radical cycloalkyle de 5 à 7 chaînons, un radical phényle ou un radical hétéroaryle monocyclique de 5 ou 6 chaînons dans lequel l'unique hétéroatome est l'azote, l'oxygène ou le soufre et qui sont éventuellement mono, di ou trisubstitués de façon identique ou différente par des atomes d'halogène, des radicaux alkyle de $C_1$ à $C_4$, haloalkyle de $C_1$ à $C_4$ ou
alkoxy de $C_1$ à $C_4$, et W représente un groupe =CH-QH,
ou un hétérocycle =C[Q-(CH2)n-Q ],
ou un groupe =C=Q,
dans lesquels
Q est un atome d'oxygène ou de soufre,
n a pour valeur 2 ou 3,
W n'étant =C=Q que lorsque R3 et R4 ne sont pas tous deux l'hydrogène,
et leurs sels d'addition avec des acides.

2. Propanamines selon la revendication 1, caractérisées en ce que R1 et R5 sont des radicaux phényle éventuellement mono, di ou trisubstitués.

3. Propanamines selon la revendication 1 ou 2, caractérisées en ce que W représente un groupe =C[Q-(CH2)n-Q] avec Q représentant un atome d'oxygène.

4. Propanamines selon la revendication 3, caractérisées en ce que n est égal à 2.

5. Propanamines selon la revendication 2, caractérisées en ce que W représente un groupe carbonyle.

6. Propanamines selon l'une des revendications 1 à 5, caractérisées en ce que R3 est méthyle et R4 est l'hydrogène.

7. Propanamines selon l'une des revendications 1 à 5, caractérisées en ce que R3 et R4 sont chacun un radical méthyle.

8. Le2-[3-N-méthylamino-3-(4-trifluorométhylphényle)-pentan-1-yl]-2-(3,4,5-triméthoxyphényle)-1,3-dioxolane, et ses sels d'additions avec des acides.

9. Le 4-N,N-diméthylamino-4-(4-méthylphényle)-1-(3,4,5-triméthoxyphényle)-hexan-1-one, et ses sels d'additions avec des acides.

**10.** Procédé de préparation des propanamines de formule (I) telle que définie à la revendication 1, caractérisé en ce qu'il consiste

A. pour préparer une propanamine dans laquelle R3 et R4 sont l'hydrogène,

a) quand W représente le groupe =CH-QH ce qui correspond à une propanamine de formule (I.A.1)

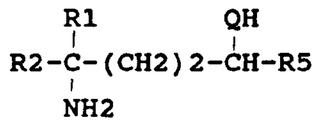 (I.A.1)

à réduire un composé nitré (III)

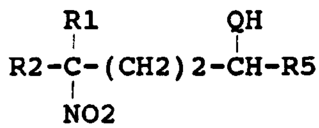 (III)

par hydrogénation catalysée par un élément du groupe VIII de la table périodique des éléments et

b) quand W représente le groupe =C[Q-(CH2)n-Q] ce qui correspond à une propanamine de formule (I.D.1)

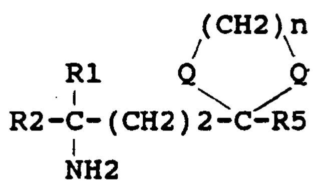 (I.D.1)

à réduire un composé nitré (IV)

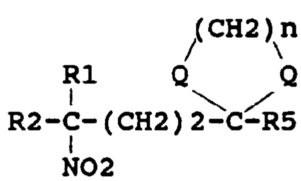 (IV)

par hydrogénation catalysée par un élément du groupe VIII de la table périodique des éléments,

B. pour préparer une propanamine dans laquelle R4 est l'hydrogène et est différent de R3,

a) quand W représente un groupe =CH-QH, ce qui correspond à une propanamine (I.A.2),

à alkyler une propanamine de formule (I.A.1) par un réactif d'alkylation R3-X1 dans lequel X1 est un atome d'halogène tel que le chlore, le brome ou l'iode,

ou bien, à réduire par un hydrure métallique ou organométallique (Hm) : M1(t)M2H(r)RX(s) dans lequel M1 est un métal alcalin, M2 est un élément du groupe III de la classification périodique, RX est un groupe carbonitrile ou un radical alkyle ou alkoxy de $C_1$ à $C_4$, (t) est égal à zéro ou un, (r) est égal à un, trois ou quatre et (s) est égal à zéro, un, trois ou quatre et dans lequel (r) + (s) - (t) = 3, et de formule (Hm.2) dans laquelle M2 est l'aluminium ou, lorsque M2 est le bore (r) vaut trois, (s) et (t) ayant pour valeur zéro, un N-carboxamide de formule (V.A.2.) de formule :

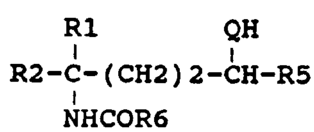 (V.A.2.)

dans laquelle R6 est l'hydrogène ou un radical homologue inférieur d'un atome de carbone à R3 (R3 = -CH2-R6),

b) quand W représente le groupe =C[Q-(CH2)n-Q], ce qui correspond à une propanamine (I.D.2), à alkyler une propanamine de formule (I.D.1) par un réactif d'alkylation R3-X1 dans lequel X1 est un atome d'halogène tel que le chlore, le brome ou l'iode,

ou bien à réduire par un hydrure métallique ou organométallique (Hm.2) précédemment défini un N-carboxamide de formule (V.D.2)

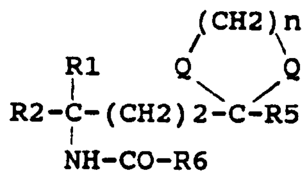

(V.D.2)

c) quand W représente le groupe =C=Q lorsque Q représente l'oxygène, ce qui correspond à une propanamine (I.C.2/O) de formule

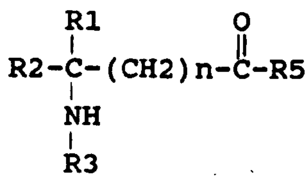

(I.C.2/O)

à oxyder par un diatome ou un ion polyatomique dont le potentiel normal redox E° est supérieur à 0,60V une propanamine (I.A.2) dans laquelle Q est l'oxygène,

ou bien à hydrolyser par une solution acide lorsque dans le groupe -Q(CH2)nQ- Q est l'oxygène et par une solution oxydante lorsque dans le groupe -Q(CH2)nQ- Q est le soufre, une propanamine (I.D.2),

d) quand W représente le groupe =C=Q lorsque Q représente le soufre, ce qui correspond à une propanamine (I.C.2/S) de formule

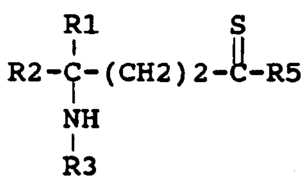

(I.C.2/S)

à faire réagir une propanamine (I.C.2/O) avec le pentasulfure de phosphore,

ou bien à effectuer une réaction de thio-oxo substitution entre un composé (VI.1) de formule

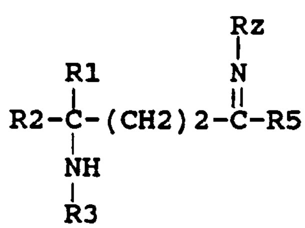

(VI.1)

dans laquelle Rz est l'hydrogène, un radical amine (-NH2), hydroxyle (-OH), un radical alkyle de $C_1$ à $C_4$ ou un radical phényle,

et l'hydrogène sulfuré, le sulfure de carbone, le monochlorure de soufre, un acide S-alkylthioïque dans lequel le radical alkyle comprend de un à cinq atomes de carbone en chaîne linéaire ou ramifiée ou un acide S-phénylthioïque comme l'acide S-thiobenzoïque,

C. pour préparer une propanamine (I) dans laquelle R3 et R4 ne sont pas l'hydrogène,

C.1. et, lorsque R3 et R4 sont identiques et représentent des radicaux alkyle ou alkényle de $C_1$ à $C_4$,

a) lorsque W représente un groupe =CH-QH, ce qui correspond à une propanamine (I.A.3) ou un groupe =C[Q-(CH2)n-Q] dans une propanamine (I.D.3),

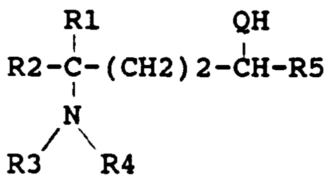

(I.A.3)

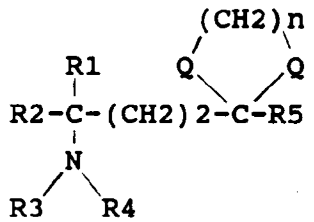

(I.D.3)

à dialkyler une propanamine de formule (I.A.1) ou de formule (I.D.1) par un aldéhyle R7-CHO dans lequel R7 est l'hydrogène ou un radical alkyle ou alkényle, homologue comprenant un atome de carbone de moins que R3 et R4 (R3=R3=CH2-R7), et avec un agent de réduction qui est un hydrure organo-métallique (Hm) pour lequel on préfère que M2 soit le bore et Rx est un groupe carbonitrile (Hm.3) ou l'acide formique ou un de ses sels,

b) lorsque W représente un groupe =C=O, ce qui correspond à une propanamine (I.C.3/O)

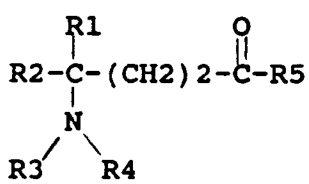

(I.C.3/O)

à oxyder une propanamine correspondante dans laquelle W est un groupe =CH-OH, par un diatome ou un ion polyatomique dont le potentiel normal redox E° est supérieur à 0,60V,

ou bien à hydrolyser une propanamine (I.D.3) par une solution acide lorsque dans le groupe [Q-(CH2)n-Q] Q est l'oxygène et par une solution oxydante, lorsque dans le groupe [Q-(CH2)n-Q] Q est le soufre, une propanamine (I.D.2),

c) lorsque W représente un groupe =C=S, ce qui correspond à une propanamine (I.C.3/S)

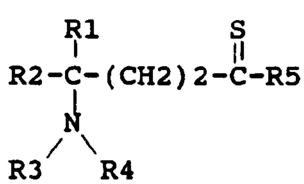

(I.C.3/S)

à faire réagir une propanamine (I.C.3/O) avec le pentasulfure de phosphore,

ou bien à effectuer une réaction de thio-oxo substitution entre un composé (VI.2) de formule

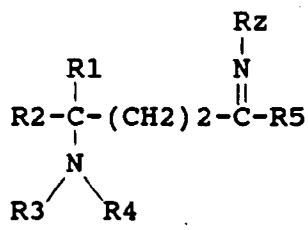

(VI.2)

dans laquelle Rz est l'hydrogène, un radical amine (-NH2), hydroxyle (-OH), un radical alkyle de $C_1$ à $C_4$ ou un radical phényle,
et l'hydrogène sulfuré, le sulfure de carbone, le mono chlorure de soufre, un acide S-alkylthioïque dans lequel le radical alkyle comprend de un à cinq atomes de carbone en chaîne linéaire ou ramifiée ou un acide S-phénylthioïque conne l'acide S-thiobenzoïque,
C.2. et, pour préparer une propanamine (I) dans laquelle R3 et R4 forment ensemble et avec l'atome d'azote auquel ils sont liés un hétérocycle saturé comprenant de 5 à 6 chaînons,
à dialkyler une propanamine (I.D.1) par un réactif dihalogéné X3-(CH2)q-X4 dans lequel X3 et X4 identiques ou différents sont le chlore, le brome ou l'iode et q a pour valeur 4 ou 5, pour obtenir une propanamine (I.D.3) de formule

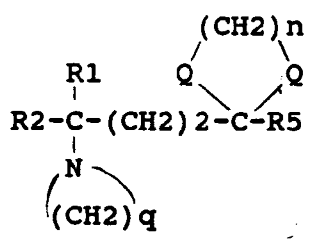

(I.D.3)

et à préparer une propanamine (I.C.3/O) correspondantes en hydrolysant par un acide ou un oxydant dans les condition précédemment définies une propanamine (I.D.3),
et à préparer une propanamine correspondante (I.C.3/S) en faisant réagir une propanamine (I.C.3/O) avec le pentasulfure de phosphore ou bien en préparant un composé intermédiaire (VI.2) que l'on engage dans une réaction de thio-oxo substitution avec les réactifs précédemment cités,
et à préparer une propanamine (I.A.3/O) par réduction d'une propanamine (I.C.3/O), et une propanamine (I.A.3/S) par réduction d'une propanamine (I.C.3/S) avec un hydrure métallique ou organométallique (Hm) dans lequel on préfère que M2 soit le bore et M1 le sodium,
C.3. pour préparer une propanamine (I) dans laquelle R3 et R4, différents l'un de l'autre, ne sont pas l'hydrogène et,

a) lorsque W représente un groupe =C=Q, ce qui correspond à une propanamine (I.C.3) ou un groupe =C[Q-(CH2)n-Q], ce qui correspond à une propanamine (I.D.3)

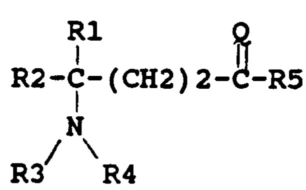

(I.C.3)

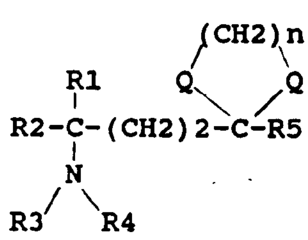

(I.D.3)

à alkyler une propanamine de formule (I.D.2) ou de formule (I.C.2) dans laquelle R4 est l'hydrogène

par un réactif d'alkylation R4X5 dans lequel X5 est un atome de chlore, de brome ou d'iode et,

b) lorsque W représente un groupe =CH-QH) ce qui correspond à une propanamine (I.A.3) ou un groupe =C[Q-(CH2)n-Q] ce qui correspond à une propanamine (I.D.3), à faire réagir une propanamine (I.A.2) ou une propanamine (I.D.2) avec un aldéhyde R7-CHO homologue inférieur d'un atome de carbone à R4 (R4=-CH2-R7) et un hydrure réducteur (Hm), de formule (Hm.3) précédemment définie,

ou bien à réduire par un hydrure métallique ou organo métallique (Hm), de formule (Hm.2) précédemment définie un N-carboxamide de formule (V.A.3) ou un carboxamide de formule (V.D.3)

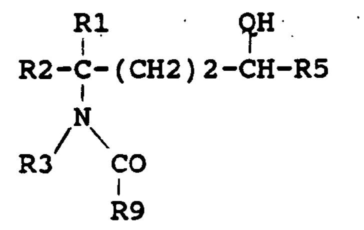

(V.A.3)

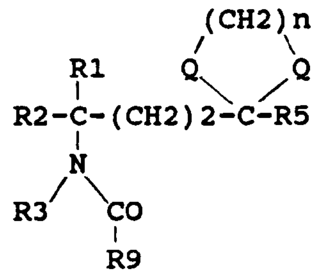

(V.D.3)

dans lequel R9 est l'hydrogène ou un radical carboné homologue inférieur d'un atome de carbone à R4 (R4=-CH2-R9), ou bien à substituer le radical carbonitrile d'un aminonitrile de formule (VII.1) ou de formule (VII.2)

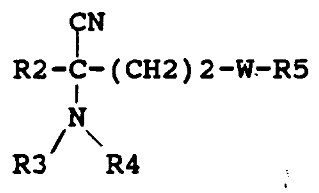

(VII.1)

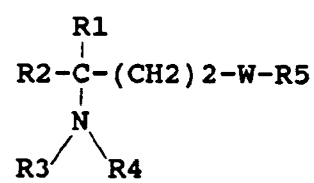

(VII.2)

par les radicaux carbonés R1 et R2 des réactifs organométalliques R1-M-X7 et R2-M-X8 dans lesquels

R1 a les significations indiquées pour les propanamines (I) à l'exception de radicaux halogénés,

R2 est alkyle de $C_1$ à $C_4$,

M est un atome métallique bivalent choisi parmi le magnésium, le cadmium et le zinc,

X7 et X8 sont des atomes d'halogène choisis entre le chlore, le brome ou l'iode, et

C.4. pour préparer des propanamines (I) dans lesquelles ni R3 ni R4 ne sont de l'hydrogène,

a) quand W est =CH-QH, ce qui correspond à une propanamine de formule (I.A.3)

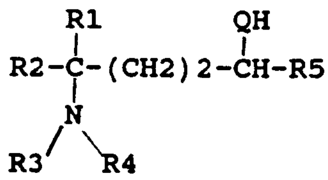

(I.A.3)

à réduire, par un hydrure métallique ou organo métallique (Hm) dans lequel M2 est de préférence le bore et M1 le sodium, une propanamine de formule (I.C.3),

b) et, lorsque W est =C=O ce qui correspond à une propanamine de formule (I.C.3/O), à oxyder, par des systèmes oxydo-réducteurs ioniques mono ou polyatomiques et dont le potentiel normal E° à 20°C est supérieur à 0,60 V, une propanamine (I.A.3/O),

ou bien, à hydrolyser, par une solution acide le groupe W lorsque Q est l'oxygène ou par une solution oxydante le groupe W lorsque Q est le soufre, une propanamine (I.D.3),

c) et, lorsque W est =C=S, ce qui correspond à une propanamine (I.C.3/S)

à effectuer une réaction de thioxo substitution avec un intermédiaire (VI.2) de formule

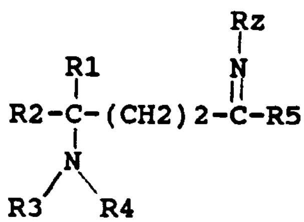

(VI.2)

d) et lorsque W est un groupe =C[Q-(CH2)n-Q], ce qui correspond à une propanamine de formule (I.D.3)

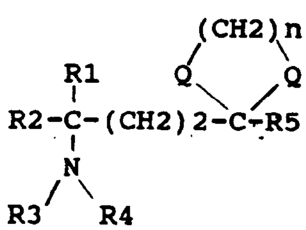

(I.D.3)

à condenser une propanamine (I.C.3) avec une réactif bifonctionnel HQ-(CH2)n-QH dans lequel n a pour valeur 2 ou 3, Q étant le soufre ou l'oxygène.

**11.** Procédé de préparation d'un médicament, caractérisé en ce qu'il consiste à mélanger une propanamine de formule (I) telle que définie à l'une des revendications 1 à 9, à un excipient pharmaceutiquenent approprié.

**12.** Utilisation d'une propanamine de formule (I) telle que définie à l'une des revendications 1 à 9 pour l'obtention d'un médicament destiné au traitement des états diarrhéïques.

**13.** Intermédiaires de formule (XX)

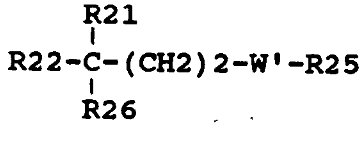

(XX)

dans laquelle

R21 est un radical phényle éventuellement mono, di ou trisubstitué de manière identique ou différente par des atomes d'halogène, des radicaux alkyle de $C_1$ à $C_4$, haloalkyle de $C_1$ à $C_4$, alkoxy de $C_1$ à $C_4$, ou est un radical hétéroaryle monocyclique de 5 ou 6 chaînons dans lequel l'unique hétéroatome est l'azote, l'oxygène ou le soufre, ou est un radical carbonitrile lorsque R22 est alkyle de $C_1$ à $C_4$,

R22 est, quand R21 est un radical phényle ou hétéroaryle comme défini ci-dessus, un radical alkyle inférieur ou un radical carbonitrile,

R25 est un radical cycloalkyle de 5 à 7 chaînons, un radical phényle ou un radical hétéroaryle monocyclique de 5 à 6 chaînons dans lequel l'unique hétéroatome est l'azote, l'oxygène ou le soufre et qui sont éventuellement mono, di ou trisubstitués de façon identique ou différente par des atomes d'halogène, des radicaux alkyle de $C_1$ à $C_4$, haloalkyle de $C_1$ à $C_4$ ou alkoxy de $C_1$ à $C_4$,

R26 est un radical nitro lorsque ni R21 ni R22 ne sont carbonitrile,
ou lorsque l'un de R21 ou de R22 est carbonitrile, est un groupe -N(R23)R24 dans lequel R23 et R24 sont un radical alkyle de $C_1$ à $C_4$, alkényle de $C_1$ à $C_4$, cycloalkylalkyle (avec cycloalkyle de $C_3$ à $C_7$ et alkyle de $C_1$ à $C_4$), différents ou identiques sans toutefois être tous deux cycloalkylalkyle,
ou R23 et R24 forment ensemble, avec l'atome d'azote auquel ils sont reliés, un hétérocycle saturé comprenant un seul hétéroatome et constitué de 5 à 6 chaînons, et

W' représente un groupe =CH-QH ou un hétérocycle =C[Q-(CH2)n-Q] quand l'un de R21 ou R22 sont carbonitrile, et dans lesquels Q est un atome d'oxygène ou de soufre, ou un groupe =C=Q lorsque R26 représente un groupe nitro, Q étant alors un atome d'oxygène, de soufre ou un groupe N-Ry dans lequel Ry est l'hydrogène ou un radical alkyle de $C_1$ à $C_4$, n a pour valeur 2 ou 3.

## Patentansprüche

### Patentansprüche für folgende Vertragsstaaten : AT, BE, CH, DE, DK, FR, GB, IT, LI, LU, NL, SE

1. Propanamine der allgemeinen Formel (I):

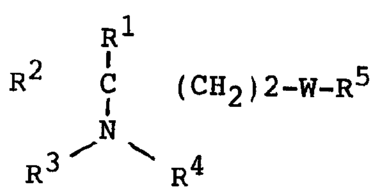

(I)

in der:

$R^1$ ein Phenylrest ist, der ggf. durch Halogenatome oder Alkyl-, Haloalkyl- oder Alkoxyreste mit jeweils $C_1$ bis $C_4$ in gleicher oder verschiedener Weise einfach, zwei- oder dreifach substituiert ist,
oder ein monocyclischer Heteroarylrest mit 5 oder 6 Gliedern ist, in dem das einzige Heteroatom Stickstoff, Sauerstoff oder Schwefel ist,

$R^2$ ein Alkylrest mit $C_1$ bis $C_4$ ist,

$R^3$ und $R^4$ ein Wasserstoffatom, ein Alkyl- oder Alkenylrest mit jeweils $C_1$ bis $C_4$ oder ein Cycloalkylalkylrest (mit Cycloalkyl zu $C_3$ bis $C_7$ und Alkyl zu $C_1$ bis $C_4$) sind und verschieden oder gleich sind, ohne jedoch beide Cycloalkylalkyl zu sein,
oder zusammen mit dem Stickstoffatom, an das sie gebunden sind, eine gesättigte heterocyclische Verbindung bilden, die ein einziges Heteroatom besitzt und aus 5 bis 6 Glieder besteht,

$R^5$ ein Cycloalkylrest mit 5 bis 7 Gliedern, ein Phenylrest oder ein monocyclischer Heteroarylrest mit 5 oder 6 Gliedern ist, in dem das einzige Heteroatom Stickstoff, Sauerstoff oder Schwefel ist, und die ggf. in gleicher oder verschiedener Weise durch Halogenatome oder Alkyl-, Haloalkyl- oder Alkoxyreste mit jeweils $C_1$ bis $C_4$ einfach, zwei- oder dreifach substituiert sind,
und W eine Gruppe =CH-QH,
oder eine heterocyclische Verbindung $=C[Q-(CH_2)_n-Q]$
oder eine Gruppe =C=Q ist,

in denen

- Q ein Sauerstoff- oder Schwefelatom ist,
- n den Wert 2 oder 3 hat,
- W nur dann =C=Q ist, wenn $R^3$ und $R^4$ nicht beide Wasserstoff sind,

und ihre Säureadditionssalze.

**2.** Propanamine nach Anspruch 1, dadurch gekennzeichnet, daß $R^1$ und $R^5$ ggf. einfach, zwei- oder dreifach substituierte Phenylreste sind.

**3.** Propanamine nach Anspruch 1 oder 2, dadurch gekennzeichnet, daß W eine Gruppe $=C[Q-(CH_2)_n-Q]$ ist, wobei Q ein Sauerstoffatom darstellt.

**4.** Propanamine nach Anspruch 3, dadurch gekennzeichnet, daß n gleich 2 ist.

**5.** Propanamine nach Anspruch 2, dadurch gekennzeichnet, daß W eine Carbonylgruppe ist.

**6.** Propanamine nach einem der Ansprüche 1 bis 5, dadurch gekennzeichnet, daß $R^3$ Methyl und $R^4$ Wasserstoff ist.

**7.** Propanamine nach einem der Ansprüche 1 bis 5, dadurch gekennzeichnet, daß $R^3$ und $R^4$ jeweils ein Methylrest sind.

**8.** 2-[3-N-Methylamino-3-(4-trifluormethylphenyl)-pentan-1-yl]-2-(3,4,5-trimethoxyphenyl)-1,3-dioxolan und seine Säureadditionssalze.

**9.** 4-N,N-dimethylamino-4-(4-methylphenyl)-1(3,4,5-trimethoxyphenyl)-hexan-1-on und seine Säureadditionssalze.

**10.** Verfahren zur Herstellung der Propanamine der Formel (I) gemäß Anspruch 1, dadurch gekennzeichnet, daß

A. zur Herstellung eines Propanamins, in dem $R^3$ und $R^4$ Wasserstoff sind,

a) wenn W die Gruppe =CH-QH ist, was einem Propanamin der Formel (I.A.1)

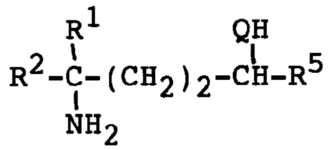

(I.A.1)

entspricht,
eine nitrierte Verbindung (III)

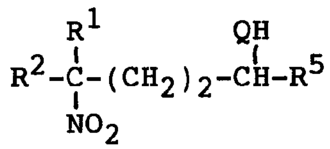

(III)

durch katalysierte Hydrierung durch ein Element der Gruppe VIII des periodischen Systems der Elemente reduziert wird und

b) wenn W die Gruppe $=C[Q-(CH_2)_n-Q]$ ist, was einem Propanamin der Formel (I.D.1)

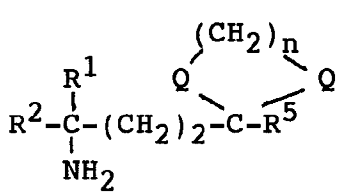

(I.D.1)

entspricht,
eine nitrierte Verbindung (IV)

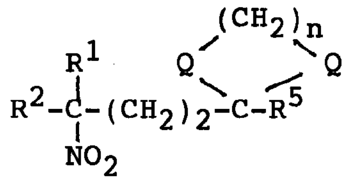

(IV)

durch katalysierte Hydrierung durch ein Element der Gruppe VIII des periodischen Elementesystems reduziert wird,

B. zur Herstellung eines Propanamins, in dem $R^4$ Wasserstoff und von $R^3$ verschieden ist,

a) wenn W eine Gruppe =CH-QH ist, was einem Propanamin (I.A.2) entspricht,
ein Propanamin der Formel (I.A.1) durch ein Alkylierungsreagenz $R^3$-X1 alkyliert wird, in dem X1 ein Halogenatom wie Chlor, Brom oder Jod ist, oder durch ein Metall- oder Organo-Metallhydrid der Formel (Hm):
$M^1_{(t)}M^2H_{(r)}RX_{(s)}$, in dem $M^1$ ein Alkalimetall, $M^2$ ein Element der Gruppe III des periodischen Systems, RX eine Carbonitrilgruppe oder ein Alkyl- oder Alkoxyrest mit $C_1$ bis $C_4$ ist, (t) gleich 0 oder 1, (r) gleich 1, 3 oder 4 und (s) gleich 0, 1, 3 oder 4 ist und in dem (r) + (s) - (t) = 3 ist, und der Formel (Hm.2), in der $M^2$ Aluminium ist oder, wenn $M^2$ Bor ist, (r) 3 beträgt, wobei (s) und (t) den Wert 0 haben, ein N-Carboxamid der Formel (V.A.2.) reduziert wird:

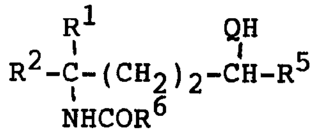

(V.A.2.)

in der $R^6$ Wasserstoff oder ein Rest ist, der das um ein Kohlenstoffatom niedrigere Homologe zu $R^3$ ist ($R^3$=-$CH_2$-$R^6$),

b) wenn W die Gruppe =C[Q-$(CH_2)_n$-Q] ist, was einem Propanamin (I.D.2) entspricht, ein Propanamin der Formel (I.D.1) durch ein Alkylierungsreagenz $R^3$-X1 alkyliert wird,in dem X1 ein Halogenatom wie Chlor, Brom oder Jod ist, oder durch ein oben beschriebenes Metall- oder Organo-Metallhydrid (Hm.2) ein N-Carboxamid der Formel (V.D.2)

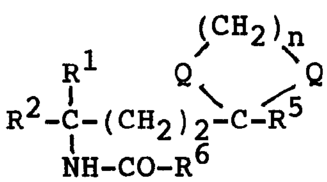

(V.D.2)

reduziert wird,

c) wenn W die Gruppe =C=Q ist, wenn Q Sauerstoff ist, was einem Propanamin (I.C.2/0) der Formel

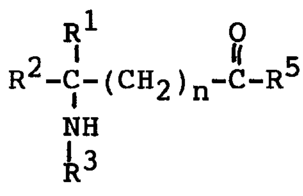

(I.C.2/0)

entspricht,
ein Propanamin (I.A.2), in dem Q Wasserstoff ist,durch ein zweiatomiges oder ein mehratomiges Ion, dessen Redoxnormalpotential E° größer als 0,60 V ist, oxidiert wird oder ein Propanamin (I.D.2), wenn in der Gruppe -Q$(CH_2)_n$Q- Q Sauerstoff ist, durch eine saure Lösung und, wenn in der Gruppe -Q$(CH_2)_n$Q- Q Schwefel ist, durch eine oxidierende Lösung hydrolysiert wird,

d) wenn W die Gruppe =C=Q ist, wenn Q Schwefel darstellt, was einem Propanamin (I.C.2/S) der Formel

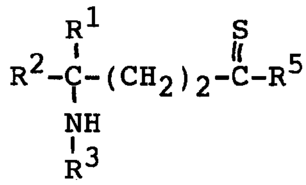

(I.C.2/S)

entspricht,
man ein Propanamin (I.C.2/0) mit Phosphorpentasulfid reagieren läßt
oder zwischen einer Verbindung (VI.I) der Formel

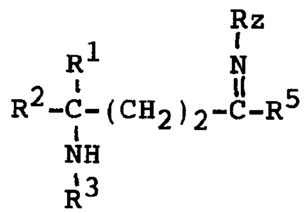

(VI.1)

in der Rz Wasserstoff, ein Aminrest ($-NH_2$), ein Hydroxylrest (-OH), ein niedriger Alkylrest oder ein Phenylrest ist,
und Schwefelwasserstoff, Schwefelkohlenstoff, Schwefelmonochlorid, einer S-Alkylthiosäure, in der der Alkylrest 1 bis 5 Kohlenstoffatome mit verzweigter oder unverzweigter Kette besitzt, oder einer S-Phenylthiosäure wie S-Thiobenzoesäure eine Thio-oxo-Substitutionsreaktion ausführt,

C. zur Herstellung eines Propanamins (I), in dem $R^3$ und $R^4$ nicht Wasserstoff sind,

C.1. und wenn $R^3$ und $R^4$ gleich sind und Alkyl- oder Alkenylreste mit $C_1$ bis $C_4$ sind,

a) wenn W eine Gruppe =CH-QH ist, was einem Propanamin (I.A.3) entspricht, oder eine Gruppe =C[Q-$(CH_2)_n$-Q] in einem Propanamin (I.D.3) ist,

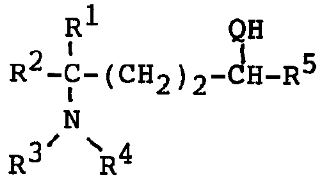

(I.A.3)

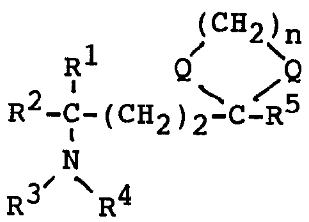

(I.D.3)

ein Propanamin der Formel (I.A.1) oder der Formel (I.D.1) durch einen Aldehyd $R^7$-CHO, in dem $R^7$ Wasserstoff oder ein homologer Alkyl- oder Alkenylrest, der das um ein C-Atom niedrigere Homologe zu $R^3$ und $R^4$ ist ($R^3$=$R^4$=$CH_2$-$R^7$), und mit einem Reduktionsmittel dialkyliert wird, das ein Organo-Metallhydrid (Hm) ist, bei dem bevorzugt $M^2$ Bor und Rx eine Carbonitrilgruppe (Hm.3) oder Ameisensäure oder eine ihrer Salze ist,

b) wenn W eine Gruppe =C=O ist, was einem Propanamin (I.C.3/0) entspricht

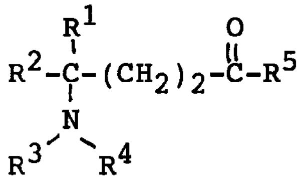

(I.C.3/0)

ein entsprechendes Propanamin, in dem W eine Gruppe =CH-OH ist, durch ein zweiatomiges oder mehratomiges Ion oxidiert wird, dessen Redoxnormalpotential E° größer als 0,60 V ist,
oder ein Propanamin (I.D.3) durch eine sauere Lösung, wenn in der Gruppe [Q-$(CH_2)_n$-Q] Q Sauerstoff ist, und Propanamin (I.D.2) durch eine oxidierende Lösung, wenn in der Gruppe [Q-$(CH_2)_n$-Q] Q Schwefel ist, hydrolysiert wird,
c) wenn W eine Gruppe =C=S ist, was einem Propanamin (I.C.3/S) entspricht,

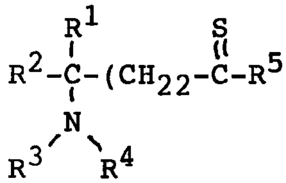

(I.C.3/S)

man ein Propanamin (I.C.3/0) mit Phosphorpentasulfid reagieren läßt oder zwischen einer Verbindung (VI.2) der Formel

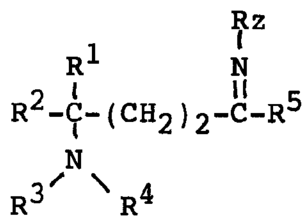

(VI.2)

in der Rz Wasserstoff, ein Aminrest ($-NH_2$), ein Hydroxylrest (-OH), ein Alkylrest mit $C_1$ bis $C_4$ oder ein Phenylrest ist, und Schwefelwasserstoff, Schwefelkoh-lenstoff, Schwefelmonochlorid, einer S-Alkylthiosäure, in der der Alkylrest 1 bis 5 Kohlenstoffatome in verzweigter oder unverzweigter Kette besitzt, oder einer S-Phenylthiosäure wie S-Thiobenzoesäure eine Thioxo-Substitutionsreaktion durchführt,
C.2. und zur Herstellung eines Propanamins (I), in dem $R^3$ und $R^4$ zusammen und mit dem Stickstoffatom, an das sie gebunden sind, eine gesättigte heterocyclische Verbindung mit 5 bis 6 Gliedern bilden,
ein Propanamin (I.D.1) durch ein dihalogeniertes Reagenz X3-$(CH_2)_q$-X4 dialkyliert wird, in dem X3 und X4 gleich oder verschieden sind und Chlor, Brom oder Jod sind und q den Wert 4 oder 5 hat, um ein Propanamin (I.D.3) der Formel

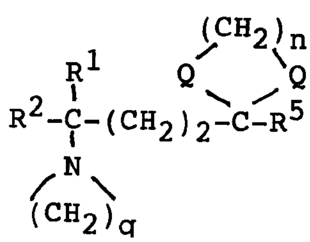

(I.D.3)

zu erhalten,
und ein entsprechendes Propanamin (I.C.3/0) hergestellt wird, indem ein Propanamin (I.D.3) durch eine Säure oder ein Oxidierungsmittel unter den oben beschriebenen Bedingungen hydrolysiert wird,
und ein entsprechendes Propanamin (I.C.3/S) hergestellt wird, indem man ein Propanamin (I.C.3/0) mit Phosphorpentasulfid reagieren läßt oder indem man eine Zwischenverbindung (VI.2) herstellt,die einer Thio-oxo-Substitutionsreaktion mit den obengenannten Reagenzien unterzogen wird,

und ein Propanamin (I.A.3/0) durch Reduktion eines Propanamins (I.C.3/0) und ein Propanamin (I.A.3/S) durch Reduktion eines Propanamins (I.C.3/S) mit einem Metall- oder Organo-Metallhydrid (Hm) hergestellt wird, in dem bevorzugt $M^2$ Bor und $M^1$ Natrium ist,

C.3. zur Herstellung eines Propanamins (I), in dem $R^3$ und $R^4$ voneinander verschieden sind und nicht Wasserstoff sind, und

a) wenn W eine Gruppe =C=Q ist, was einem Propanamin (I.C.3) entspricht, oder eine Gruppe =C[Q-$(CH_2)_n$-Q] ist, was einem Propanamin (I.D.3) entspricht

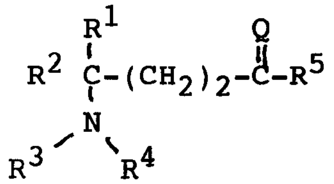

(I.C.3)

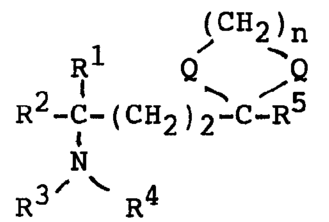

(I.D.3)

ein Propanamin der Formel (I.D.2) oder der Formel (I.C.2), in der $R^4$ Wasserstoff ist, durch ein Alkylierungsreagenz $R^4X5$ alkyliert wird, in dem X5 ein Chlor-, Brom- oder Jodatom ist, und

b) wenn W eine Gruppe =CH-QH, was einem Propanamin (I.A.3) entspricht, oder eine Gruppe =C[Q-$(CH_2)_n$-Q] ist, was einem Propanamin (I.D.3) entspricht, man ein Propanamin (I.A.2) oder ein Propanamin (I.D.2) mit einem Aldehyd $R^7$-CHO, das das um ein Kohlenstoffatom niedrigere Homologe zu $R^4$ ($R^4$=-$CH_2$-$R^7$) ist, und einem oben definierten reduzierenden Hydrid (Hm) der Formel (Hm.3) reagieren läßt,

oder durch ein Metall- oder Organo-Metallhydrid (Hm) der oben definierten Formel (Hm.2) ein N-Carboxamid der Formel (V.A.3) oder ein Carboxamid der Formel (V.D.3)

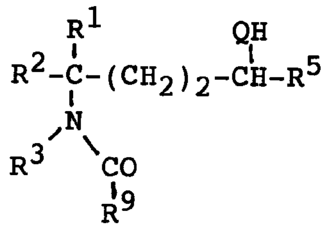

(V.A.3)

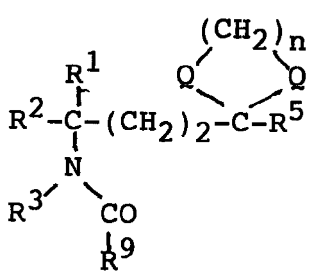

(V.D.3)

reduziert,

in der $R^9$ Wasserstoff oder ein Kohlenstoffrest ist, der das um ein Kohlenstoffatom niedrigere Homologe zu $R^4$ ist ($R^4$ = -$CH_2$-$R^9$), oder den Carbonitrilrest eines Aminonitrils der Formel (VII.1) oder Formel (VII.2)

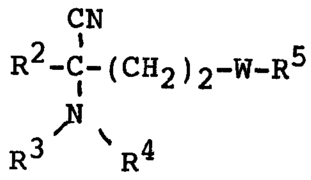

(VII.1)

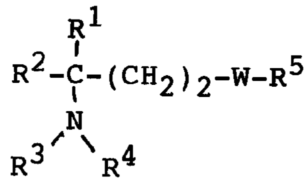

(VII.2)

durch die Kohlenstoffreste $R^1$ und $R^2$ der Organo-Metall-Reagenzien $R^1$-M-X7 und $R^2$-M-X8 substituiert, in denen $R^1$ die für die Propanamine (I) mit Ausnahme der halogenierten Reste angegebenen Bedeutungen hat, $R^2$ Alkyl mit $C_1$ bis $C_4$,
M ein zweiwertiges Metallatom, ausgewählt aus Mag-nesium, Cadmium und Zink, ist,
X7 und X8 aus Chlor, Brom und Jod ausgewählte Halogenatome sind, und
C.4. zur Herstellung der Propanamine (I), in denen weder $R^3$ noch $R^4$ Wasserstoff ist,
a) wenn W =CH-QH ist, was einem Propanamin der Formel (I.A.3)

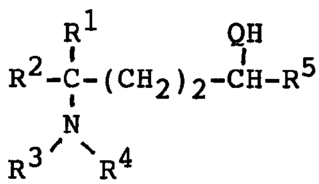

(I.A.3)

entspricht,
ein Propanamin der Formel (I.C.3) durch ein Metall- oder Organo-Metallhydrid (Hm) reduziert wird, in dem $M^2$ vorzugsweise Bor und $M^1$ Natrium entspricht,
b) und wenn W =C=O ist, was einem Propanamin der Formel (I.C.3/0) ist, ein Propanamin (I.A.3/0) durch ein- oder mehratomische ionische oxydo-reduzierende Systeme, deren Normalpotential E° bei 20°C über 0,60 V beträgt, oxidiert wird,
oder ein Propanamin (I.D.3), wenn in der Gruppe W Q Sauerstoff ist, durch eine sauere Lösung oder, wenn in der Gruppe W Q Schwefel ist, durch eine oxidierende Lösung hydrolysiert wird,
c) und, wenn W =C=S ist, was einem Propanamin (I.C.3/S) entspricht,
eine Thio-oxo-Substitutionsreaktion mit einem Zwischenprodukt (VI.2) der Formel

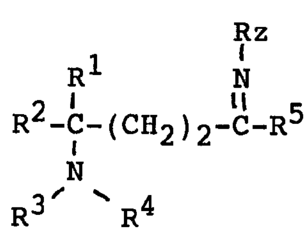

(VI.2)

durchgeführt wird,
d) und wenn W eine Gruppe =C[Q-$(CH_2)_n$-Q] ist, was einem Propanamin der Formel (I.D.3) entspricht

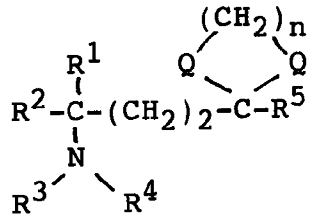

(I.D.3)

ein Propanamin (I.C.3) mit einem bifunktionellen Reagenz HQ-$(CH_2)_n$-QH kondensiert wird, in dem n den Wert 2 oder 3 hat, wobei Q Schwefel oder Sauerstoff ist.

**11.** Arzneimittel, dadurch gekennzeichnet, daß es ein Propanamin (I) nach einem der Ansprüche 1 bis 9 enthält.

**12.** Verwendung eines Propanamins (I) nach einem der Ansprüche 1 bis 9 zur Herstellung eines Arzneimittels für die Behandlung von diarrhoeischen Zuständen.

**13.** Zwischenprodukt der Formel (XX)

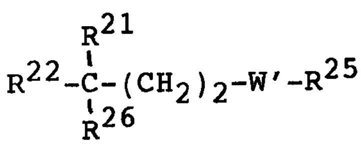

(XX)

in der

| | |
|---|---|
| $R^{21}$ | ein Phenylrest ist, der ggf. auf gleiche oder verschiedene Weise durch Halogenatome, Alkyl-, Haloalkyl- oder Alkoxyreste mit jeweils $C_1$ bis $C_4$ einfach, zweifach oder dreifach substituiert ist, oder ein monocyclischer Heteroarylrest mit 5 oder 6 Gliedern ist, in dem das einzige Heteroatom Stickstoff, Sauerstoff oder Schwefel ist, oder ein Carbonitrilrest ist, wenn $R^{22}$ Alkyl mit $C_1$ bis $C_4$ ist, |
| $R^{22}$, | wenn $R^{21}$ ein Phenyl- oder Heteroarylrest, wie oben angegeben, ist, ein Alkylrest mit $C_1$ bis $C_4$ oder ein Carbonitrilrest ist, |
| $R^{25}$ | ein Cycloalkylrest mit 5 bis 7 Gliedern, ein Phenylrest oder ein monocyclischer Heteroarylrest mit 5 bis 6 Gliedern, in dem das einzige Heteroatom Stickstoff, Sauerstoff oder Schwefel ist und die ggf. auf gleiche oder verschiedene Weise durch Halogenatome oder Alkyl-, Haloalkyl- oder Alkoxyreste mit jeweils $C_1$ bis $C_4$ einfach, zweifach oder dreifach substituiert sind, |
| $R^{26}$ | ein Nitrorest ist, wenn weder $R^{21}$ noch $R^{22}$ Carbonitril ist, oder, wenn $R^{21}$ oder $R^{22}$ Carbonitril ist, eine Gruppe -N($R^{23}$)$R^{24}$ ist, in der $R^{23}$ und $R^{24}$ ein Alkyl- oder Alkenylrest mit jeweils $C_1$ bis $C_4$ oder ein Cycloalkylalkylrest (mit Cycloalkyl zu $C_3$ bis $C_7$ und Alkyl zu $C_1$ bis $C_4$) ist, die verschieden oder gleich sind, ohne jedoch beide Cycloalkylalkyl zu sein, oder $R^{23}$ und $R^{24}$ zusammen mit dem Stickstoffatom, an das sie gebunden sind, eine gesättigte heterocyclische Verbindung bilden, die ein einziges Heteroatom besitzt und aus 5 bis 6 Gliedern besteht, und |
| W' | eine Gruppe =CH-QH oder eine heterocyclische Verbindung =C[Q-$(CH_2)_n$-Q] ist, worin Q ein Sauerstoff- oder Schwefelatom ist, wenn $R^{21}$ oder $R^{22}$ Carbonitril ist, oder eine Gruppe =C=Q ist, wenn $R^{26}$ eine Nitrogruppe ist, wobei Q hierbei ein Sauerstoff- oder Schwefelatom oder eine Gruppe N-Ry ist, in der Ry Wasserstoff oder ein Alkylrest mit $C_1$ bis $C_4$ ist, wobei n den Wert 2 oder 3 hat. |

**Patentansprüche für folgenden Vertragsstaat : ES**

**1.** Propanamine der allgemeinen Formel (I):

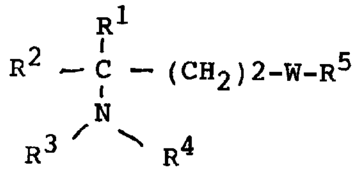

(I)

in der:

R[1] ein Phenylrest ist, der ggf. durch Halogenatome oder Alkyl-, Haloalkyl- oder Alkoxyreste mit jeweils $C_1$ bis $C_4$ in gleicher oder verschiedener Weise einfach, zwei- oder dreifach substituiert ist,
oder ein monocyclischer Heteroarylrest mit 5 oder 6 Gliedern ist, in dem das einzige Heteroatom Stickstoff, Sauerstoff oder Schwefel ist,

R[2] ein Alkylrest mit $C_1$ bis $C_4$ ist,

R[3] und R[4] ein Wasserstoffatom, ein Alkyl- oder Alkenylrest mit jeweils $C_1$ bis $C_4$ oder ein Cycloalkylalkylrest (mit Cycloalkyl zu $C_3$ bis $C_7$ und Alkyl zu $C_1$ bis $C_4$) sind und verschieden oder gleich sind, ohne jedoch beide Cycloalkylalkyl zu sein,
oder zusammen mit dem Stickstoffatom, an das sie gebunden sind, eine gesättigte heterocyclische Verbindung bilden, die ein einziges Heteroatom besitzt und aus 5 bis 6 Glieder besteht,

R[5] ein Cycloalkylrest mit 5 bis 7 Gliedern, ein Phenylrest oder ein monocyclischer Heteroarylrest mit 5 oder 6 Gliedern ist, in dem das einzige Heteroatom Stickstoff, Sauerstoff oder Schwefel ist, und die ggf. in gleicher oder verschiedener Weise durch Halogenatome oder Alkyl-, Haloalkyloder Alkoxyreste mit jeweils $C_1$ bis $C_4$ einfach, zwei- oder dreifach substituiert sind,
und W eine Gruppe =CH-QH, (Gruppe I.A)
oder eine heterocyclische Verbindung $=C[Q-(CH_2)_n-Q]$ (Gruppe I.D)
oder eine Gruppe =C=Q (Gruppe I.C) ist,

in denen

Q ein Sauerstoff- oder Schwefelatom ist,

n den Wert 2 oder 3 hat,

W nur dann =C=Q ist, wenn R[3] und R[4] nicht beide Wasserstoff sind,

und ihre Säureadditionssalze, dadurch gekennzeichnet, daß

A. zur Herstellung eines Propanamins, in dem R[3] und R[4] Wasserstoff sind,

a) wenn W die Gruppe =CH-QH ist, was einem Propanamin der Formel (I.A.1)

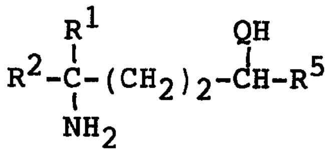

(I.A.1)

entspricht,
eine nitrierte Verbindung (III)

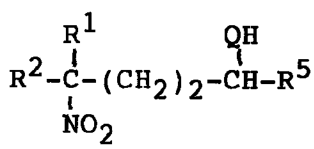

(III)

durch katalysierte Hydrierung durch ein Element der Gruppe VIII des periodischen Systems der Elemente reduziert wird und

b) wenn W die Gruppe $=C[Q-(CH_2)_n-Q]$ ist, was einem Propanamin der Formel (I.D.1)

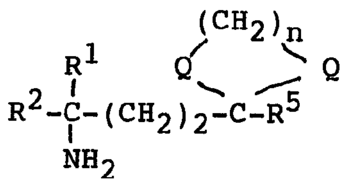

(I.D.1)

entspricht,
eine nitrierte Verbindung (IV)

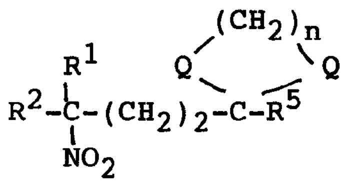

(IV)

durch katalysierte Hydrierung durch ein Element der Gruppe VIII des periodischen Elementesystems reduziert wird,

B. zur Herstellung eines Propanamins, in dem $R^4$ Wasserstoff und von $R^3$ verschieden ist,

a) wenn W eine Gruppe =CH-QH ist, was einem Propanamin (I.A.2) entspricht,
ein Propanamin der Formel (I.A.1) durch ein Alkylierungsreagenz $R^3$-X1 alkyliert wird, in dem X1 ein Halogenatom wie Chlor, Brom oder Jod ist, oder durch ein Metall- oder Organo-Metallhydrid der Formel (Hm): $M^1_{(t)}M^2H_{(r)}RX_{(s)}$, in dem $M^1$ ein Alkalimetall, $M^2$ ein Element der Gruppe III des periodischen Systems, RX eine Carbonitrilgruppe oder ein Alkyl- oder Alkoxyrest mit $C_1$ bis $C_4$ ist, (t) gleich 0 oder 1, (r) gleich 1, 3 oder 4 und (s) gleich 0, 1, 3 oder 4 ist und in dem (r) + (s) - (t) = 3 ist, und der Formel (Hm.2), in der $M^2$ Aluminium ist oder, wenn $M^2$ Bor ist, (r) 3 beträgt, wobei (s) und (t) den Wert 0 haben, ein N-Carboxamid der Formel (V.A.2.) reduziert wird:

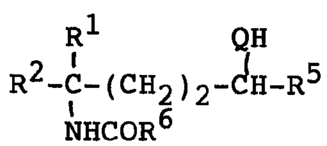

(V.A.2.)

in der $R^6$ Wasserstoff oder ein Rest ist, der das um ein Kohlenstoffatom niedrigere Homologe zu $R^3$ ist ($R^3 = -CH_2-R^6$),

b) wenn W die Gruppe =C[Q-$(CH_2)_n$-Q] ist, was einem Propanamin (I.D.2) entspricht, ein Propanamin der Formel (I.D.1) durch ein Alkylierungsreagenz $R^3$-X1 alkyliert wird,in dem X1 ein Halogenatom wie Chlor, Brom oder Jod ist, oder durch ein oben beschriebenes Metall- oder Organo-Metallhydrid (Hm.2) ein N-Carboxamid der Formel (V.D.2)

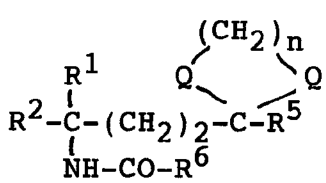

(V.D.2)

reduziert wird,

c) wenn W die Gruppe =C=Q ist, wenn Q Sauerstoff ist, was einem Propanamin (I.C.2/0) der Formel

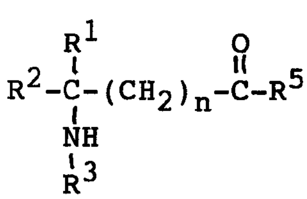

(I.C.2/0)

entspricht,
ein Propanamin (I.A.2), in dem Q Wasserstoff ist,durch ein zweiatomiges oder ein mehratomiges Ion, dessen Redoxnormalpotential E° größer als 0,60 V ist, oxidiert wird oder ein Propanamin (I.D.2), wenn in der Gruppe $-Q(CH_2)_nQ-$ Q Sauerstoff ist, durch eine saure Lösung und, wenn in der Gruppe $-Q(CH_2)_nQ-$ Q Schwefel ist, durch eine oxidierende Lösung hydrolysiert wird,
d) wenn W die Gruppe =C=Q ist, wenn Q Schwefel darstellt, was einem Propanamin (I.C.2/S) der Formel

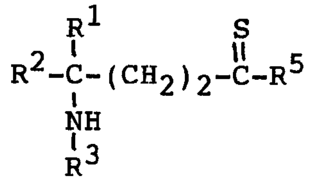

(I.C.2/S)

entspricht,
man ein Propanamin (I.C.2/0) mit Phosphorpentasulfid reagieren läßt
oder zwischen einer Verbindung (VI.I) der Formel

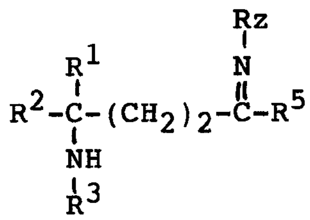

(VI.1)

in der Rz Wasserstoff, ein Aminrest ($-NH_2$), ein Hydroxylrest (-OH), ein Alkylrest mit $C_1$ bis $C_4$ oder ein Phenylrest ist,
und Schwefelwasserstoff, Schwefelkohlenstoff, Schwefelmonochlorid, einer S-Alkylthiosäure, in der der Alkylrest 1 bis 5 Kohlenstoffatome mit verzweigter oder unverzweigter Kette besitzt, oder einer S-Phenylthiosäure wie S-Thiobenzoesäure eine Thio-oxo-Substitutionsreaktion ausführt,
C. zur Herstellung eines Propanamins (I), in dem $R^3$ und $R^4$ nicht Wasserstoff sind,
C.1. und wenn $R^3$ und $R^4$ gleich sind und Alkyl- oder Alkenylreste mit $C_1$ bis $C_4$ sind,
a) wenn W eine Gruppe =CH-QH ist, was einem Propanamin (I.A.3) entspricht, oder eine Gruppe $=C[Q-(CH_2)_n-Q]$ in einem Propanamin (I.D.3) ist,

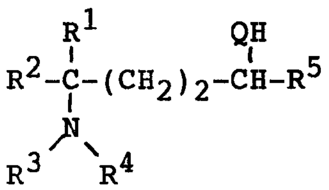

(I.A.3)

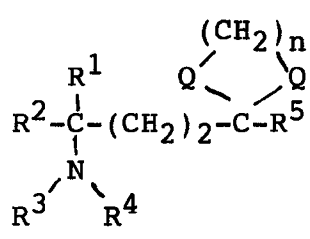

(I.D.3)

ein Propanamin der Formel (I.A.1) oder der Formel (I.D.1) durch einen Aldehyd $R^7$-CHO, in dem $R^7$ Wasserstoff oder ein homologer Alkyl- oder Alkenylrest, der das um ein C-Atom niedrigere Homologe zu $R^3$ und $R^4$ ist ($R^3=R^4=CH_2-R^7$), und mit einem Reduktionsmittel dialkyliert wird, das ein Organo-Metallhydrid (Hm) ist, bei dem bevorzugt $M^2$ Bor und Rx eine Carbonitrilgruppe (Hm.3) oder Ameisensäure oder eine ihrer Salze ist,

b) wenn W eine Gruppe =C=O ist, was einem Propanamin (I.C.3/0) entspricht

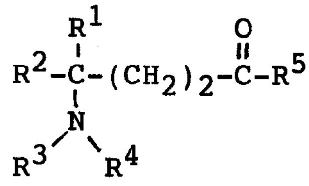

(I.C.3/0)

ein entsprechendes Propanamin, in dem W eine Gruppe =CH-OH ist, durch ein zweiatomiges oder mehratomiges Ion oxidiert wird, dessen Redoxnormalpotential E° größer als 0,60 V ist,
oder ein Propanamin (I.D.3) durch eine sauere Lösung, wenn in der Gruppe [Q-$(CH_2)_n$-Q] Q Sauerstoff ist, und Propanamin (I.D.2) durch eine oxidierende Lösung, wenn in der Gruppe [Q-$(CH_2)_n$-Q] Q Schwefel ist, hydrolysiert wird,
c) wenn W eine Gruppe =C=S ist, was einem Propanamin (I.C.3/S) entspricht,

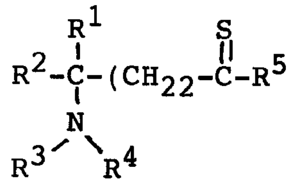

(I.C.3/S)

man ein Propanamin (I.C.3/0) mit Phosphorpentasulfid reagieren läßt oder zwischen einer Verbindung (VI.2) der Formel

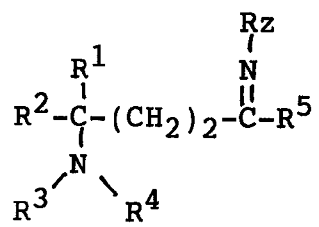

(VI.2)

in der Rz Wasserstoff, ein Aminrest ($-NH_2$), ein Hydroxylrest (-OH), ein Alkylrest mit $C_1$ bis $C_4$ oder ein Phenylrest ist, und Schwefelwasserstoff, Schwefelkohlenstoff, Schwefelmonochlorid, einer S-Alkylthiosäure, in der der Alkylrest 1 bis 5 Kohlenstoffatome in verzweigter oder unverzweigter Kette besitzt, oder einer S-Phenylthiosäure wie S-Thiobenzoesäure eine Thioxo-Substitutionsreaktion durchführt,
C.2. und zur Herstellung eines Propanamins (I), in dem $R^3$ und $R^4$ zusammen und mit dem Stickstoffatom, an das sie gebunden sind, eine gesättigte heterocyclische Verbindung mit 5 bis 6 Gliedern bilden,
ein Propanamin (I.D.1) durch ein dihalogeniertes Reagenz X3-$(CH_2)_q$-X4 dialkyliert wird, in dem X3 und X4 gleich oder verschieden sind und Chlor, Brom oder Jod sind und q den Wert 4 oder 5 hat, um ein Propanamin (I.D.3) der Formel

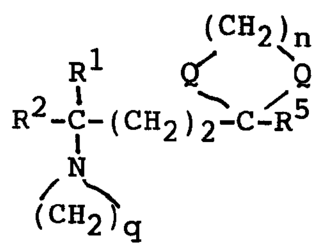

(I.D.3)

zu erhalten,
und ein entsprechendes Propanamin (I.C.3/0) hergestellt wird, indem ein Propanamin (I.D.3) durch eine Säure oder ein Oxidierungsmittel unter den oben beschriebenen Bedingungen hydrolysiert wird,
und ein entsprechendes Propanamin (I.C.3/S) hergestellt wird, indem man ein Propanamin (I.C.3/0) mit

Phosphorpentasulfid reagieren läßt oder indem man eine Zwischenverbindung (VI.2) herstellt,die einer Thio-oxo-Substitutionsreaktion mit den obengenannten Reagenzien unterzogen wird,
und ein Propanamin (I.A.3/0) durch Reduktion eines Propanamins (I.C.3/0) und ein Propanamin (I.A.3/S) durch Reduktion eines Propanamins (I.C.3/S) mit einem Metall- oder Organo-Metallhydrid (Hm) hergestellt wird, in dem bevorzugt $M^2$ Bor und $M^1$ Natrium ist,
C.3. zur Herstellung eines Propanamins (I), in dem $R^3$ und $R^4$ voneinander verschieden sind und nicht Wasserstoff sind, und

a) wenn W eine Gruppe =C=Q ist, was einem Propanamin (I.C.3) entspricht, oder eine Gruppe =C[Q-$(CH_2)_n$-Q] ist, was einem Propanamin (I.D.3) entspricht

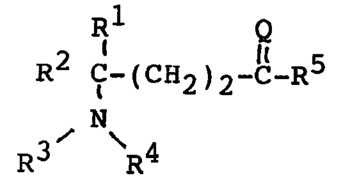

(I.C.3)

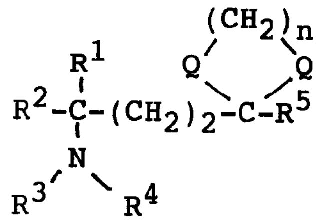

(I.D.3)

ein Propanamin der Formel (I.D.2) oder der Formel (I.C.2), in der $R^4$ Wasserstoff ist, durch ein Alkylierungsreagenz $R^4X5$ alkyliert wird, in dem X5 ein Chlor-, Brom- oder Jodatom ist, und

b) wenn W eine Gruppe =CH-QH, was einem Propanamin (I.A.3) entspricht, oder eine Gruppe =C[Q-$(CH_2)_n$-Q] ist, was einem Propanamin (I.D.3) entspricht, man ein Propanamin (I.A.2) oder ein Propanamin (I.D.2) mit einem Aldehyd $R^7$-CHO, das das um ein Kohlenstoffatom niedrigere Homologe zu $R^4$ ($R^4$=-$CH_2$-$R^7$) ist, und einem oben definierten reduzierenden Hydrid (Hm) der Formel (Hm.3) reagieren läßt,
oder durch ein Metall- oder Organo-Metallhydrid (Hm) der oben definierten Formel (Hm.2) ein N-Carboxamid der Formel (V.A.3) oder ein Carboxamid der Formel (V.D.3)

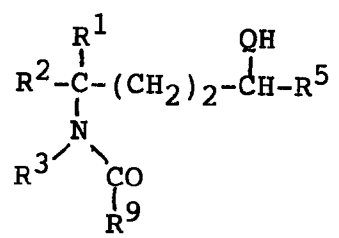

(V.A.3)

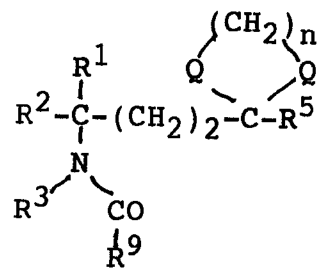

(V.D.3)

reduziert,
in der $R^9$ Wasserstoff oder ein Kohlenstoffrest ist, der das um ein Kohlenstoffatom niedrigere Homologe zu $R^4$ ist ($R^4$ = -$CH_2$-$R^9$), oder den Carbonitrilrest eines Aminonitrils der Formel (VII.1) oder Formel (VII.2)

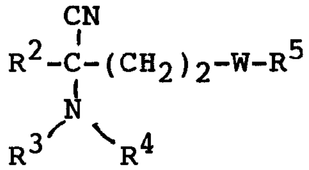

(VII.1)

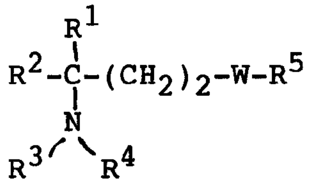

(VII.2)

durch die Kohlenstoffreste $R^1$ und $R^2$ der Organo-Metall-Reagenzien $R^1$-M-X7 und $R^2$-M-X8 substituiert, in denen $R^1$ die für die Propanamine (I) mit Ausnahme der halogenierten Reste angegebenen Bedeutungen hat, $R^2$ Alkyl mit $C_1$ bis $C_4$,
M ein zweiwertiges Metallatom, ausgewählt aus Magnesium, Cadmium und Zink, ist,
X7 und X8 aus Chlor, Brom und Jod ausgewählte Halogenatome sind, und
C.4. zur Herstellung der Propanamine (I), in denen weder $R^3$ noch $R^4$ Wasserstoff ist,
a) wenn W =CH-QH ist, was einem Propanamin der Formel (I.A.3)

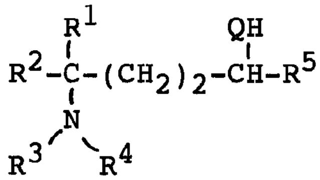

(I.A.3)

entspricht,
ein Propanamin der Formel (I.C.3) durch ein Metall-oder Organo-Metallhydrid (Hm) reduziert wird, in dem $M^2$ vorzugsweise Bor und $M^1$ Natrium entspricht,
b) und wenn W =C=O ist, was einem Propanamin der Formel (I.C.3/0) ist, ein Propanamin (I.A.3/0) durch ein- oder mehratomische ionische oxydo-reduzierende Systeme, deren Normalpotential E° bei 20°C über 0,60 V beträgt, oxidiert wird,
oder ein Propanamin (I.D.3), wenn in der Gruppe W Q Sauerstoff ist, durch eine sauere Lösung oder, wenn in der Gruppe W Q Schwefel ist, durch eine oxidierende Lösung hydrolysiert wird,
c) und, wenn W =C=S ist, was einem Propanamin (I.C.3/S) entspricht,
eine Thio-oxo-Substitutionsreaktion mit einem Zwischenprodukt (VI.2) der Formel

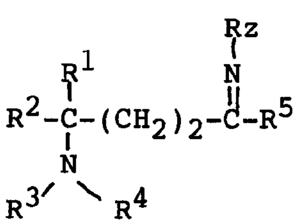

(VI.2)

durchgeführt wird,
d) und wenn W eine Gruppe $=C[Q-(CH_2)_n-Q]$ ist, was einem Propanamin der Formel (I.D.3) entspricht

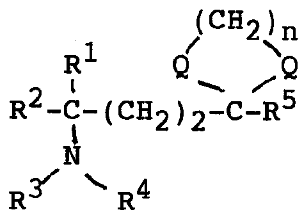

(I.D.3)

ein Propanamin (I.C.3) mit einem bifunktionellen Reagenz $HQ-(CH_2)_n-QH$ kondensiert wird, in dem n den Wert 2 oder 3 hat, wobei Q Schwefel oder Sauerstoff ist.

2. Verfahren nach Anspruch 1, gekennzeichnet, daß $R^1$ und $R^5$ ggf. einfach, zwei- oder dreifach substituierte Phenylreste sind.

3. Verfahren nach Anspruch 1 oder 2, dadurch gekennzeichnet, daß W eine Gruppe $=C[Q-(CH_2)_n-Q]$ ist, wobei Q ein Sauerstoffatom darstellt.

4. Verfahren nach Anspruch 3, dadurch gekennzeichnet, daß n gleich 2 ist.

5. Verfahren nach Anspruch 2, dadurch gekennzeichnet, daß W eine Carbonylgruppe ist.

6. Verfahren nach einem der Ansprüche 1 bis 5, dadurch gekennzeichnet, daß $R^3$ Methyl und $R^4$ Wasserstoff ist.

7. Verfahren nach einem der Ansprüche 1 bis 5, dadurch gekennzeichnet, daß $R^3$ und $R^4$ jeweils ein Methylrest sind.

8. Verfahren nach Anspruch 1, dadurch gekennzeichnet, daß $R^1$ ein Trifluormethylphenylrest, $R^2$ Ethyl , $R^3$ Wasserstoff, $R^4$ Methyl, $W =C[O-(CH_2)_2-O]$ und $R^5$ 3,4,5-Trimethoxyphenyl ist.

9. Verfahren nach Anspruch 1, dadurch gekennzeichnet, daß $R^1$ 4-Methylphenyl, $R^2$ Ethyl, $R^3$ und $R^4$ jeweils Methyl, W =C=O und $R^5$ 3,4,5-Trimethoxyphenyl ist.

10. Verfahren zur Herstellung eines Arzneimittels, dadurch gekennzeichnet, daß ein Propanamin der Formel (I) gemäß einem der Ansprüche 1 bis 9 mit einer pharmazeutisch akzeptablen Grundsubstanz gemischt wird.

11. Verwendung eines Propanamins (I) nach einem der Ansprüche 1 bis 9 zur Herstellung eines Arzneimittels für die Behandlung von diarrhoeischen Zuständen.

**Patentansprüche für folgenden Vertragsstaat : GR**

1. Propanamine der allgemeinen Formel (I):

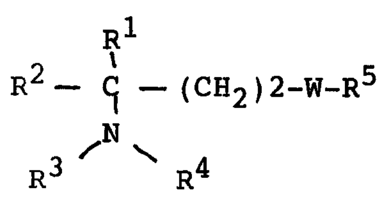

(I)

in der:

$R^1$ ein Phenylrest ist, der ggf. durch Halogenatome oder Alkyl-, Haloalkyl- oder Alkoxyreste mit jeweils $C_1$ bis $C_4$ in gleicher oder verschiedener Weise einfach, zwei- oder dreifach substituiert ist,
oder ein monocyclischer Heteroarylrest mit 5 oder 6 Gliedern ist, in dem das einzige Heteroatom Stickstoff, Sauerstoff oder Schwefel ist,

$R^2$ ein Alkylrest mit $C_1$ bis $C_4$ ist,

$R^3$ und $R^4$ ein Wasserstoffatom, ein Alkyl- oder Alkenylrest mit jeweils $C_1$ bis $C_4$ oder ein Cycloalkylalkylrest (mit Cycloalkyl zu $C_3$ bis $C_7$ und Alkyl zu $C_1$ bis $C_4$) sind und verschieden oder gleich sind, ohne jedoch beide Cycloalkylalkyl zu sein,
oder zusammen mit dem Stickstoffatom, an das sie gebunden sind, eine gesättigte heterocyclische Verbindung bilden, die ein einziges Heteroatom besitzt und aus 5 bis 6 Glieder besteht,

$R^5$ ein Cycloalkylrest mit 5 bis 7 Gliedern, ein Phenylrest oder ein monocyclischer Heteroarylrest mit 5 oder 6 Gliedern ist, in dem das einzige Heteroatom Stickstoff, Sauerstoff oder Schwefel ist, und die ggf. in gleicher oder verschiedener Weise durch Halogenatome oder Alkyl-, Haloalkyl- oder Alkoxyreste mit jeweils $C_1$ bis $C_4$ einfach, zwei- oder dreifach substituiert sind,
und W eine Gruppe =CH-QH,
oder eine heterocyclische Verbindung $=C[Q-(CH_2)_n-Q]$
oder eine Gruppe =C=Q ist,

in denen

Q ein Sauerstoff- oder Schwefelatom ist,

n den Wert 2 oder 3 hat,

W nur dann =C=Q ist, wenn $R^3$ und $R^4$ nicht beide Wasserstoff sind,
und ihre Säureadditionssalze.

**2.** Propanamine nach Anspruch 1, dadurch gekennzeichnet, daß $R^1$ und $R^5$ ggf. einfach, zwei- oder dreifach substituierte Phenylreste sind.

**3.** Propanamine nach Anspruch 1 oder 2, dadurch gekennzeichnet, daß W eine Gruppe $=C[Q-(CH_2)_n-Q]$ ist, wobei Q ein Sauerstoffatom darstellt.

**4.** Propanamine nach Anspruch 3, dadurch gekennzeichnet, daß n gleich 2 ist.

**5.** Propanamine nach Anspruch 2, dadurch gekennzeichnet, daß W eine Carbonylgruppe ist.

**6.** Propanamine nach einem der Ansprüche 1 bis 5, dadurch gekennzeichnet, daß $R^3$ Methyl und $R^4$ Wasserstoff ist.

**7.** Propanamine nach einem der Ansprüche 1 bis 5, dadurch gekennzeichnet, daß $R^3$ und $R^4$ jeweils ein Methylrest sind.

**8.** 2-[3-N-Methylamino-3-(4-trifluormethylphenyl)-pentan-1-yl]-2-(3,4,5-trimethoxyphenyl)-1,3-dioxolan und seine Säureadditionssalze.

**9.** 4-N,N-dimethylamino-4-(4-methylphenyl)-1-(3,4,5-trimethoxyphenyl)-hexan-1-on und seine Säureadditionssalze.

**10.** Verfahren zur Herstellung der Propanamine der Formel (I) gemäß Anspruch 1, dadurch gekennzeichnet, daß
A. zur Herstellung eines Propanamins, in dem $R^3$ und $R^4$ Wasserstoff sind,
a) wenn W die Gruppe =CH-QH ist, was einem Propanamin der Formel (I.A.1)

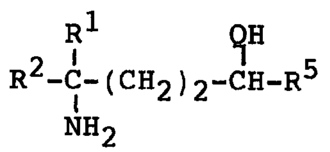

(I.A.1)

entspricht,
eine nitrierte Verbindung (III)

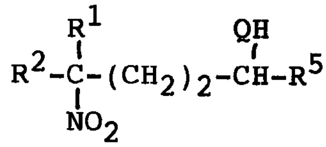

(III)

durch katalysierte Hydrierung durch ein Element der Gruppe VIII des periodischen Systems der Elemente reduziert wird und

b) wenn W die Gruppe $=C[Q-(CH_2)_n-Q]$ ist, was einem Propanamin der Formel (I.D.1)

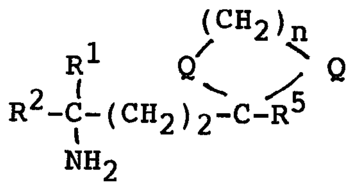

(I.D.1)

entspricht,

eine nitrierte Verbindung (IV)

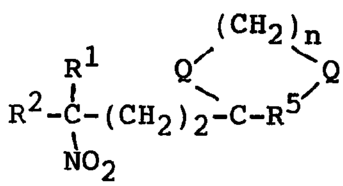

(IV)

durch katalysierte Hydrierung durch ein Element der Gruppe VIII des periodischen Elementesystems reduziert wird,

B. zur Herstellung eines Propanamins, in dem $R^4$ Wasserstoff und von $R^3$ verschieden ist,

a) wenn W eine Gruppe =CH-QH ist, was einem Propanamin (I.A.2) entspricht,

ein Propanamin der Formel (I.A.1) durch ein Alkylierungsreagenz $R^3$-X1 alkyliert wird, in dem X1 ein Halogenatom wie Chlor, Brom oder Jod ist, oder durch ein Metall- oder Organo-Metallhydrid der Formel (Hm): $M^1_{(t)}M^2H_{(r)}RX_{(s)}$, in dem $M^1$ ein Alkalimetall, $M^2$ ein Element der Gruppe III des periodischen Systems, RX eine Carbonitrilgruppe oder ein Alkyl- oder Alkoxyrest mit $C_1$ bis $C_4$ ist, (t) gleich 0 oder 1, (r) gleich 1, 3 oder 4 und (s) gleich 0, 1, 3 oder 4 ist und in dem (r) + (s) - (t) = 3 ist, und der Formel (Hm.2), in der $M^2$ Aluminium ist oder, wenn $M^2$ Bor ist, (r) 3 beträgt, wobei (s) und (t) den Wert 0 haben, ein N-Carboxamid der Formel (V.A.2.) reduziert wird:

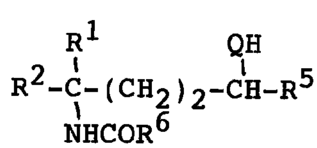

(V.A.2.)

in der $R^6$ Wasserstoff oder ein Rest ist, der das um ein Kohlenstoffatom niedrigere Homologe zu $R^3$ ist ($R^3 = -CH_2-R^6$),

b) wenn W die Gruppe $=C[Q-(CH_2)_n-Q]$ ist, was einem Propanamin (I.D.2) entspricht, ein Propanamin der Formel (I.D.1) durch ein Alkylierungsreagenz $R^3$-X1 alkyliert wird,in dem X1 ein Halogenatom wie Chlor, Brom oder Jod ist, oder durch ein oben beschriebenes Metall- oder Organo-Metallhydrid (Hm.2) ein N-Carboxamid der Formel (V.D.2)

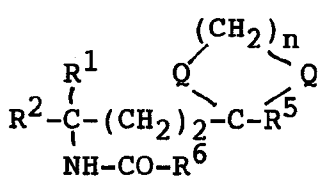

(V.D.2)

reduziert wird,

c) wenn W die Gruppe =C=Q ist, wenn Q Sauerstoff ist, was einem Propanamin (I.C.2/0) der Formel

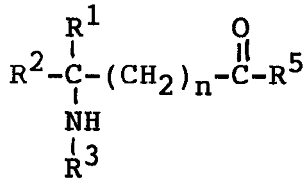

(I.C.2/0)

entspricht,

ein Propanamin (I.A.2), in dem Q Wasserstoff ist,durch ein zweiatomiges oder ein mehratomiges Ion, dessen Redoxnormalpotential E° größer als 0,60 V ist, oxidiert wird oder ein Propanamin (I.D.2), wenn in der Gruppe $-Q(CH_2)_nQ-$ Q Sauerstoff ist, durch eine saure Lösung und, wenn in der Gruppe $-Q(CH_2)_nQ-$ Q Schwefel ist, durch eine oxidierende Lösung hydrolysiert wird,

d) wenn W die Gruppe =C=Q ist, wenn Q Schwefel darstellt, was einem Propanamin (I.C.2/S) der Formel

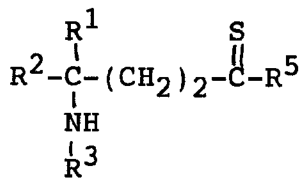

(I.C.2/S)

entspricht,

man ein Propanamin (I.C.2/0) mit Phosphorpentasulfid reagieren läßt
oder zwischen einer Verbindung (VI.I) der Formel

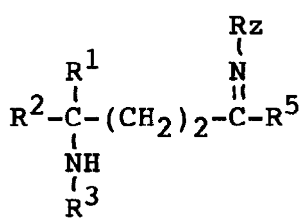

(VI.1)

in der Rz Wasserstoff, ein Aminrest ($-NH_2$), ein Hydroxylrest (-OH), ein niedriger Alkylrest oder ein Phenylrest ist,

und Schwefelwasserstoff, Schwefelkohlenstoff, Schwefelmonochlorid, einer S-Alkylthiosäure, in der der Alkylrest 1 bis 5 Kohlenstoffatome mit verzweigter oder unverzweigter Kette besitzt, oder einer S-Phenylthiosäure wie S-Thiobenzoesäure eine Thio-oxo-Substitutionsreaktion ausführt,

C. zur Herstellung eines Propanamins (I), in dem $R^3$ und $R^4$ nicht Wasserstoff sind,

C.1. und wenn $R^3$ und $R^4$ gleich sind und Alkyl- oder Alkenylreste mit $C_1$ bis $C_4$ sind,

a) wenn W eine Gruppe =CH-QH ist, was einem Propanamin (I.A.3) entspricht, oder eine Gruppe $=C[Q-(CH_2)_n-Q]$ in einem Propanamin (I.D.3) ist,

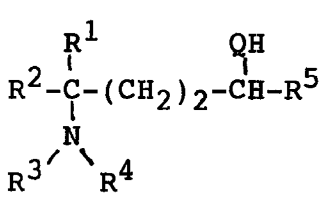

(I.A.3)

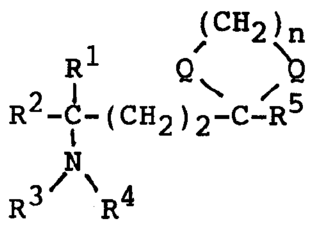

(I.D.3)

ein Propanamin der Formel (I.A.1) oder der Formel (I.D.1) durch einen Aldehyd $R^7$-CHO, in dem $R^7$ Wasserstoff oder ein homologer Alkyl- oder Alkenylrest, der das um ein C-Atom niedrigere Homologe zu $R^3$ und $R^4$ ist ($R^3$=$R^4$=$CH_2$-$R^7$), und mit einem Reduktionsmittel dialkyliert wird, das ein Organo-Metallhydrid (Hm) ist, bei dem bevorzugt $M^2$ Bor und Rx eine Carbonitrilgruppe (Hm.3) oder Ameisensäure oder eine ihrer Salze ist,
b) wenn W eine Gruppe =C=O ist, was einem Propanamin (I.C.3/0) entspricht

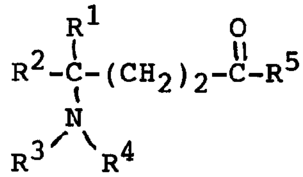

(I.C.3/0)

ein entsprechendes Propanamin, in dem W eine Gruppe =CH-OH ist, durch ein zweiatomiges oder mehratomiges Ion oxidiert wird, dessen Redoxnormalpotential E° gröBer als 0,60 V ist,
oder ein Propanamin (I.D.3) durch eine sauere Lösung, wenn in der Gruppe [Q-$(CH_2)_n$-Q] Q Sauerstoff ist, und Propanamin (I.D.2) durch eine oxidierende Lösung, wenn in der Gruppe [Q-$(CH_2)_n$-Q] Q Schwefel ist, hydrolysiert wird,
c) wenn W eine Gruppe =C=S ist, was einem Propanamin (I.C.3/S) entspricht,

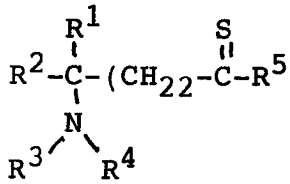

(I.C.3/S)

man ein Propanamin (I.C.3/0) mit Phosphorpentasulfid reagieren läßt oder zwischen einer Verbindung (VI.2) der Formel

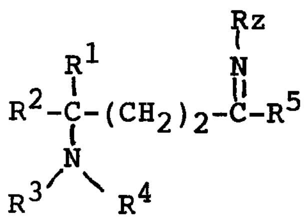

(VI.2)

in der Rz Wasserstoff, ein Aminrest (-$NH_2$), ein Hydroxylrest (-OH), ein Alkylrest mit $C_1$ bis $C_4$ oder ein Phenylrest ist, und Schwefelwasserstoff, Schwefelkohlenstoff, Schwefelmonochlorid, einer S-Alkylthiosäure, in der der Alkylrest 1 bis 5 Kohlenstoffatome in verzweigter oder unverzweigter Kette besitzt, oder einer S-Phenylthiosäure wie S-Thiobenzoesäure eine Thioxo-Substitutionsreaktion durchführt,
C.2. und zur Herstellung eines Propanamins (I), in dem $R^3$ und $R^4$ zusammen und mit dem Stickstoffatom, an das sie gebunden sind, eine gesättigte heterocyclische Verbindung mit 5 bis 6 Gliedern bilden,
ein Propanamin (I.D.1) durch ein dihalogeniertes Reagenz X3-$(CH_2)_q$-X4 dialkyliert wird, in dem X3 und X4 gleich oder verschieden sind und Chlor, Brom oder Jod sind und q den Wert 4 oder 5 hat, um ein Propanamin (I.D.3) der Formel

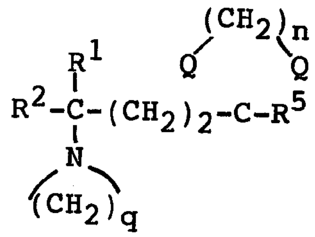

(I.D.3)

zu erhalten,
und ein entsprechendes Propanamin (I.C.3/0) hergestellt wird, indem ein Propanamin (I.D.3) durch eine Säure oder ein Oxidierungsmittel unter den oben beschriebenen Bedingungen hydrolysiert wird,
und ein entsprechendes Propanamin (I.C.3/S) hergestellt wird, indem man ein Propanamin (I.C.3/0) mit Phosphorpentasulfid reagieren läßt oder indem man eine Zwischenverbindung (VI.2) herstellt,die einer Thio-oxo-Substitutionsreaktion mit den obengenannten Reagenzien unterzogen wird,
und ein Propanamin (I.A.3/0) durch Reduktion eines Propanamins (I.C.3/0) und ein Propanamin (I.A.3/S) durch Reduktion eines Propanamins (I.C.3/S) mit einem Metall- oder Organo-Metallhydrid (Hm) hergestellt wird, in dem bevorzugt $M^2$ Bor und $M^1$ Natrium ist,
C.3. zur Herstellung eines Propanamins (I), in dem $R^3$ und $R^4$ voneinander verschieden sind und nicht Wasserstoff sind, und

a) wenn W eine Gruppe =C=Q ist, was einem Propanamin (I.C.3) entspricht, oder eine Gruppe =C[Q-$(CH_2)_n$-Q] ist, was einem Propanamin (I.D.3) entspricht

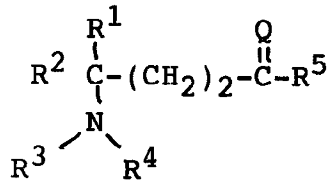

(I.C.3)

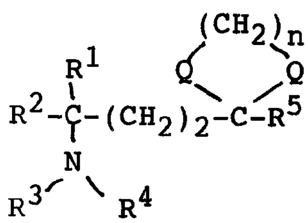

(I.D.3)

ein Propanamin der Formel (I.D.2) oder der Formel (I.C.2), in der $R^4$ Wasserstoff ist, durch ein Alkylierungsreagenz $R^4X5$ alkyliert wird, in dem X5 ein Chlor-, Brom- oder Jodatom ist, und

b) wenn W eine Gruppe =CH-QH, was einem Propanamin (I.A.3) entspricht, oder eine Gruppe =C[Q-$(CH_2)_n$-Q] ist, was einem Propanamin (I.D.3) entspricht, man ein Propanamin (I.A.2) oder ein Propanamin (I.D.2) mit einem Aldehyd $R^7$-CHO, das das um ein Kohlenstoffatom niedrigere Homologe zu $R^4$ ($R^4$=-$CH_2$-$R^7$) ist, und einem oben definierten reduzierenden Hydrid (Hm) der Formel (Hm.3) reagieren läßt,

oder durch ein Metall- oder Organo-Metallhydrid (Hm) der oben definierten Formel (Hm.2) ein N-Carboxamid der Formel (V.A.3) oder ein Carboxamid der Formel (V.D.3)

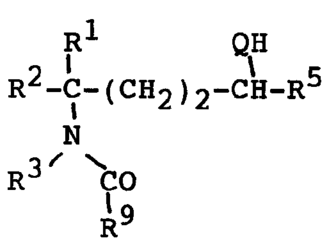

(V.A.3)

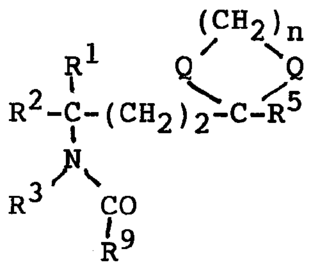

(V.D.3)

reduziert,
in der $R^9$ Wasserstoff oder ein Kohlenstoffrest ist, der das um ein Kohlenstoffatom niedrigere Homologe zu $R^4$ ist ($R^4$ = $-CH_2-R^9$), oder den Carbonitrilrest eines Aminonitrils der Formel (VII.1) oder Formel (VII.2)

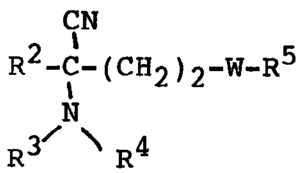

(VII.1)

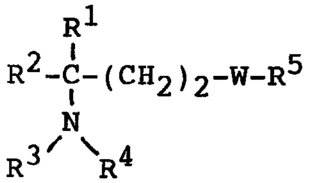

(VII.2)

durch die Kohlenstoffreste $R^1$ und $R^2$ der Organo-Metall-Reagenzien $R^1$-M-X7 und $R^2$-M-X8 substituiert, in denen $R^1$ die für die Propanamine (I) mit Ausnahme der halogenierten Reste angegebenen Bedeutungen hat, $R^2$ Alkyl mit $C_1$ bis $C_4$,
M ein zweiwertiges Metallatom, ausgewählt aus Magnesium, Cadmium und Zink, ist,
X7 und X8 aus Chlor, Brom und Jod ausgewählte Halogenatome sind, und
C.4. zur Herstellung der Propanamine (I), in denen weder $R^3$ noch $R^4$ Wasserstoff ist,
a) wenn W =CH-QH ist, was einem Propanamin der Formel (I.A.3)

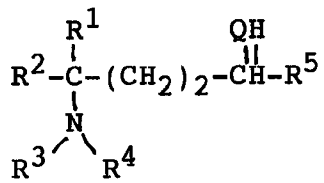

(I.A.3)

entspricht,
ein Propanamin der Formel (I.C.3) durch ein Metalloder Organo-Metallhydrid (Hm) reduziert wird, in dem $M^2$ vorzugsweise Bor und $M^1$ Natrium entspricht,
b) und wenn W =C=O ist, was einem Propanamin der Formel (I.C.3/0) ist, ein Propanamin (I.A.3/0) durch ein- oder mehratomische ionische oxydo-reduzierende Systeme, deren Normalpotential E° bei 20°C über 0,60 V beträgt, oxidiert wird,
oder ein Propanamin (I.D.3), wenn in der Gruppe W Q Sauerstoff ist, durch eine sauere Lösung oder, wenn in der Gruppe W Q Schwefel ist, durch eine oxidierende Lösung hydrolysiert wird,
c) und, wenn W =C=S ist, was einem Propanamin (I.C.3/S) entspricht,
eine Thio-oxo-Substitutionsreaktion mit einem Zwischenprodukt (VI.2) der Formel

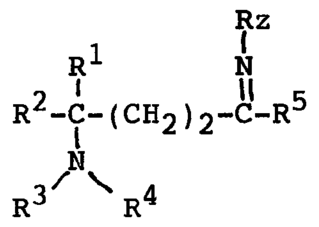

(VI.2)

durchgeführt wird,

d) und wenn W eine Gruppe $=C[Q-(CH_2)_n-Q]$ ist, was einem Propanamin der Formel (I.D.3) entspricht

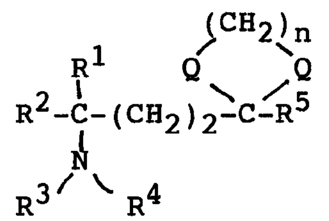

(I.D.3)

ein Propanamin (I.C.3) mit einem bifunktionellen Reagenz $HQ-(CH_2)_n-QH$ kondensiert wird, in dem n den Wert 2 oder 3 hat, wobei Q Schwefel oder Sauerstoff ist.

**11.** Verfahren zur Herstellung eines Arzneimittels, dadurch gekennzeichnet, daß ein Propanamin der Formel (I) nach einem der Ansprüche 1 bis 9 mit einer pharmazeutisch geeigneten Grundsubstanz gemischt wird.

**12.** Verwendung eines Propanamins der Formel (I) nach einem Ansprüche 1 bis 9 zur Herstellung eines Arzneimittels für Behandlung von diarrhoeischen Zuständen.

**13.** Zwischenprodukt der Formel (XX)

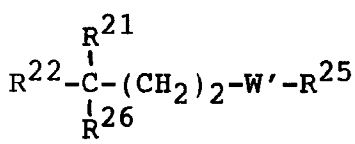

(XX)

in der

$R^{21}$ ein Phenylrest ist, der ggf. auf gleiche oder verschiedene Weise durch Halogenatome, Alkyl-, Haloalkyl- oder Alkoxyreste mit jeweils $C_1$ bis $C_4$ einfach, zweifach oder dreifach substituiert ist, oder ein monocyclischer Heteroarylrest mit 5 oder 6 Gliedern ist, in dem das einzige Heteroatom Stickstoff, Sauerstoff oder Schwefel ist, oder ein Carbonitrilrest ist, wenn $R^{22}$ Alkyl mit $C_1$ bis $C_4$ ist,

$R^{22}$, wenn $R^{21}$ ein Phenyl- oder Heteroarylrest, wie oben angegeben, ist, ein Alkylrest mit $C_1$ bis $C_4$ oder ein Carbonitrilrest ist,

$R^{25}$ ein Cycloalkylrest mit 5 bis 7 Gliedern, ein Phenylrest oder ein monocyclischer Heteroarylrest mit 5 bis 6 Gliedern, in dem das einzige Heteroatom Stickstoff, Sauerstoff oder Schwefel ist und die ggf. auf gleiche oder verschiedene Weise durch Halogenatome oder Alkyl-, Haloalkyl- oder Alkoxyreste mit jeweils $C_1$ bis $C_4$ einfach, zweifach oder dreifach substituiert sind,

$R^{26}$ ein Nitrorest ist, wenn weder $R^{21}$ noch $R^{22}$ Carbonitril ist,
oder, wenn $R^{21}$ oder $R^{22}$ Carbonitril ist, eine Gruppe $-N(R^{23})R^{24}$ ist, in der $R^{23}$ und $R^{24}$ ein Alkyl- oder Alkenylrest mit jeweils $C_1$ bis $C_4$ oder ein Cycloalkylalkylrest (mit Cycloalkyl zu $C_3$ bis $C_7$ und Alkyl zu $C_1$ bis $C_4$) ist, die verschieden oder gleich sind, ohne jedoch beide Cycloalkylalkyl zu sein,
oder $R^{23}$ und $R^{24}$ zusammen mit dem Stickstoffatom, an das sie gebunden sind, eine gesättigte heterocyclische Verbindung bilden, die ein einziges Heteroatom besitzt und aus 5 bis 6 Gliedern besteht, und

W' eine Gruppe =CH-QH oder eine heterocyclische Verbindung $=C[Q-(CH_2)_n-Q]$ ist, worin Q ein Sauerstoff- oder Schwefelatom ist, wenn $R^{21}$ oder $R^{22}$ Carbonitril ist, oder eine Gruppe =C=Q ist, wenn $R^{26}$ eine Nitrogruppe ist, wobei Q hierbei ein Sauerstoff- oder Schwefelatom oder eine Gruppe N-Ry ist, in der Ry Wasserstoff oder ein Alkylrest mit $C_1$ bis $C_4$ ist, wobei n den Wert 2 oder 3 hat.

## Claims

**Claims for the following Contracting States : AT, BE, CH, DE, DK, FR, GB, IT, LI, NL, SE, LU**

1. Propanamines of general formula (I):

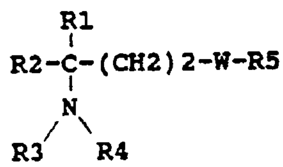

(I)

in which:

R1 is a phenyl radical, optionally mono-, di- or trisubstituted in an identical or different manner by halogen atoms, $C_1$ to $C_4$ alkyl radicals, $C_1$ to $C_4$ haloalkyl, $C_1$ to $C_4$ alkoxy, or is a 5 or 6 membered heteroaryl monocyclic radical in which the single heteroatom is nitrogen, oxygen or sulphur,

R2 is a $C_1$ to $C_4$ alkyl radical,

R3 and R4 are a hydrogen atom, a $C_1$ to $C_4$ alkyl radical, $C_1$ to $C_4$ alkenyl, cycloalkylalkyl with $C_3$ to $C_7$ cycloallkyl and $C_1$ to $C_4$ alkyl, and are different or identical without, however, both being cycloalkylalkyl,
or, together with the nitrogen atom to which they are attached, form a saturated 5 or 6 membered heterocycle,

R5 is a 5 to 7 membered radical cycloalkyl, a phenyl radical or a 5 or 6 membered monocyclic heterocycle radical in which the single heteroatom is nitrogen, oxygen or sulphur and which are optionally mono-, di- or trisubstituted in an identical or different manner by halogen atoms, $C_1$ to $C_4$ alkyl radicals, $C_1$ to $C_4$ haloalkyls or $C_1$ to $C_4$ alkoxy,
and W represents a group =CH-QH
or a heterocycle =C[Q-(CH2)n-Q]
or a group =C=Q
in which
Q is an oxygen or sulphur atom,
n has the value 2 or 3
W being =C=Q only when R3 and R4 are not both hydrogen,
and their salts of addition with acids.

2. Propanamines according to claim 1, characterised in that R1 and R5 are phenyl radicals optionally mono-, di- or trisubstituted.

3. Propanamines according to claims 1 or 2 characterised in that W represents a group =C[Q-(CH2)n-Q] with Q representing an oxygen atom.

4. Propanamines according to claim 3, characterised in that n is equal to 2.

5. Propanamines according to claim 2, characterised in that W represents a carbonyl group.

6. Propanamines according to claims 1 to 5, characterised in that R3 is methyl and R4 is hydrogen.

7. Propanamines according to claims 1 to 5, characterised in that R3 and R4 and each a methyl radical.

8. 2-[3-N-methylamino-3-(4-trifluoromethylphenyl)-pentan-1-yl]-2-(3,4,5-trimethoxyphenyl)-1,3,dioxolane,

and its salts of addition with acids.

9. 4-N,N-dimethylamino-4-(4-methylphenyl)-1-(3,4,5-trimethoxyphenyl)-hexan-1-one, and its salts of addition with acids.

10. Process for the preparation of propanamines of formula (I) as defined in claim 1, characterised in that it comprises:
A. for preparing a propanamine in which R3 and R4 are hydrogen,
a) when W represents the group =CH-QH which corresponds to a propanamine of formula (I.A.1.)

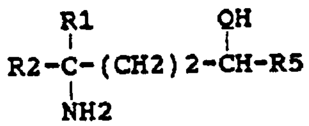

(I.A.1)

in reducing a nitro compound (III)

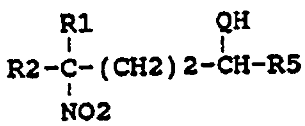

(III)

by hydrogenation catalysed by an element from group VIII of the Periodic Table of the Elements and
b) when W represents the group =C[Q-(CH2)n-Q] which corresponds to a propanamine of formula (I.D.1)

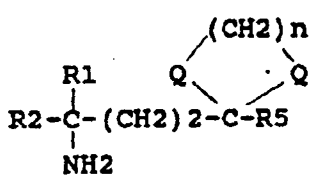

(I.D.1)

in reducing a nitro compound (IV)

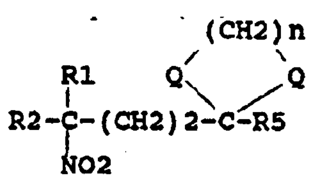

(IV)

by hydrogenation catalysed by an element from group VIII of the Periodic Table of the Elements,
B. for preparing a propanamine in which R4 is hydrogen and is different from R3,
a) when W represent a group =CH-QH, which corresponds to a propanamine (I.A.2),
in alkylating a propanamine of formula (I.A.1) by an alkylation reagent R3-X1 in which X1 is a halogen atom such a chlorine, bromine or iodine,
or, in reducing by a metallic or organometallic hydride (Hm): M1(t)M2H(r)RX(s) in which M1 is an alkaline metal, M2 is an element from group III of the Periodic Classification, RX is a carbonitrile group or an alkyl radical or $C_1$ to $C_4$ alkoxy, (t) is equal to zero or one, (r) is equal to three or four and (s) is equal to zero, one, three or four and in which (r) + (s) -(t) =3, and in formula (HM.2) in which M2 is aluminium or, when M2 is boron (r) becomes 3, (s) and (t) have the value zero, an N-carboxamide of formula (V.A.2.)

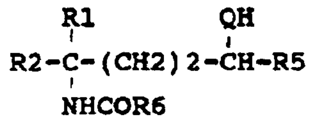 (V.A.2.)

in which R6 is hydrogen or a lower homologous radical smaller by one carbon atom than R3 (R3 = -CH2-R6),
b) when W represents the group =C[Q-(CH2)n-Q], which corresponds to a propanamine (I.D.2), in alkylating a propanamine of formula (I.D.1) by an alkylation reagent R3-X1 in which X1 is a halogen atom such as chlorine, bromine or iodine,
or in reducing by a previously defined metallic or organometallic hydride (Hm.2) an N-carboxamide of formula (V.D.2)

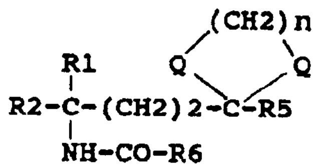 (V.D.2)

c) when W represents the group =C=Q where Q represents oxygen, which corresponds to a propanamine (I.C.2/O) of formula

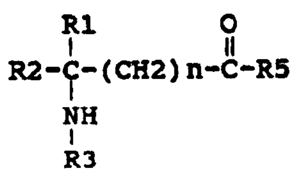 (I.C.2/O)

to oxidise by a diatomic species or a polyatomic ion whose normal redox potential E is higher than 0.60V a propanamine (I.A.2) in which Q is oxygen,
or in hydrolysing with an acid solution where in the group -Q(CH2)nQ- Q is oxygen and by an oxidising solution where in the group -Q(CH2)nQ- Q is sulphur, a propanamine (I.D.2),
d) when W represents the group =C=Q in which Q represents sulphur, this corresponds to a propanamine (I.C.2/S) of formula

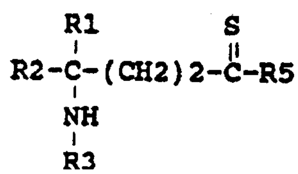 (I.C.2/S)

by reacting a propanamine (I.C.2/O) with phosphorous pentasulphide, or in carrying out a thio-oxo substitution reaction between a compound (VI.1) of formula

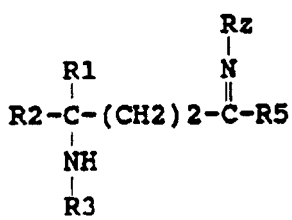 (VI.1)

in which Rz is hydrogen, an amine radical (-NH2), hydroxyl (-OH), a lower alkyl radical or a phenyl radical, and hydrogen sulphide, carbon sulphide, sulphur monochloride, an S-alkylthioic acid in which the alkyl radical comprises a linear or branched chain or one to five carbon atoms or an S-phenylthioc acid such as S-thiobenzoic acid,

C. to prepare a propanamine (I) in which R3 and R4 are not hydrogen, C.1. and, when R3 and R4 are identical and represent $C_1$ to $C_4$ alkyl or alkenyl radicals,

a) when W represent a group =CH-QH, this corresponds to a propanamine (I.A.3) or a group =([Q-(CH2)n-Q) in a propanamine (I.D.3),

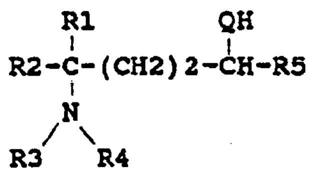

(I.A.3)

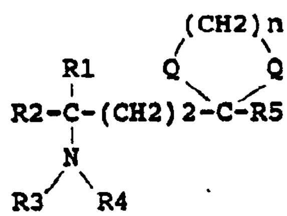

(I.D.3)

in dialkylating a propanamine of formula (I.A.1) of formula (I.D.1) with an aldehyde R7-CHO in which R7 is hydrogen or a homologous alkyl or alkenyl radical having one carbon atom less than R3 and R4 (R3=R3=CH2-R7), and with a reducing agent which is an organometallic hydride (Hm) in which it is preferred that M2 is boron and Rx is a carbonitrile group (Hm.3) or formic acid or one of its salts,

b) when W represents a group =C=O, this corresponds to a propanamine (I.C.3/O)

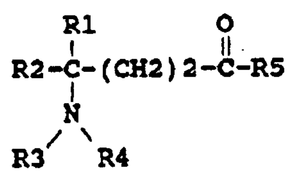

(I.C.3/O)

in oxidising a corresponding propanamine in which W is a group =CH-OH, by a diatomic species or a polyatomic ion whose normal redox potential E is higher than 0.60V,
or in hydrolysing a propanamine (I.D.3) by an acid solution when in the group [Q-(CH2)n-Q] Q is oxygen and by an oxydising solution , when in the group [Q-(CH2)n-Q) Q is sulphur, a propanamine (I.D.2),

c) when C represent a group =C=S, this corresponds to a propanamine (I.C.3/S)

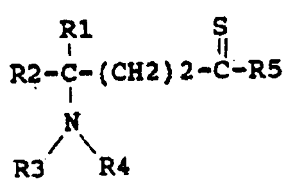

(I.C.3/S)

by reacting a propanamine (I.C.3/O) with phosphorous pentasulphide, or in carrying out a thio-oxo substitution reaction between a compound (VI.2) of formula

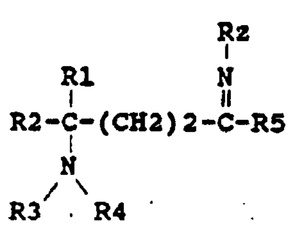

(VI.2)

in which Rz is hydrogen, an amine radical (-NH2), hydroxyl (-OH) a $C_1$ to $C_4$ alkyl radical or a phenyl radical, and hydrogen sulphide, carbon sulphide, sulphur monochloride, and S-alkylthioic acid in which the alkyl radical contains a linear or branched chain or an S-phenylthioic acid such as S-thiobenzoic acid,

C.2. and, to prepare a propanamine (I) in which R3 and R4 together and with the nitrogen atom to which they are attached, form a 5 to 6 membered saturated heterocycle,

in dialkylating a propanamine (I.D.1) with a dihalogenated reagent X3-(CH2)q-X4 in which X3 and X4 are identical or different are chlorine, bromide or iodine and q has a value 4 or 5, to obtain propanamine (I.D.3) of formula

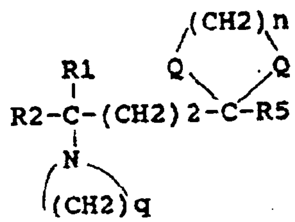

(I.D.3)

and in preparing a corresponding propanamine (I.C.3/O) by hydrolysing with an acid or an oxidant in the previous case defines a propanamine (I.D.3),

and in preparing a corresponding propanamine (I.C.3/S) by reacting a propanamine (I.C.3/O) with phosphorous pentasulphide or in preparing an intermediate compound (VI.2) which is used in a thio-oxo substitution reaction with the reagents previously cited,

and in preparing a propanamine (I.A.3/O) by reduction with a propanamine (I.C.3/O), and a propanamine (I.A.3/S) by reduction with a propanamine (I.C.3/S) with a metallic or organometallic hydride (Hm) in which it is preferred that M2 is boron and M1 is sodium,

C.3. for preparing a propanamine (I) in which R3 and R4 are different from each other, and are not hydrogen and,

a) when W represents a group =C=Q, this corresponds to a propanamine (I.C.3) or a group =C[Q-CH2)n-Q] which corresponds to a propanamine (I.D.3)

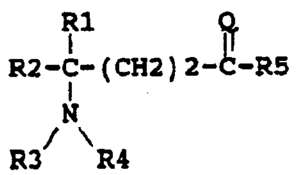

(I.C.3)

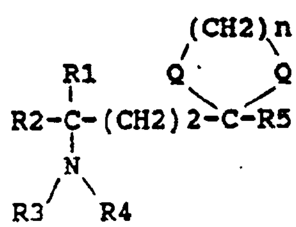

(I.D.3)

in alkylating a propanamine of formula (I.D.2) or of formula (I.C.2) in which R4 is hydrogen with an alkylating reagent R4X5 in which X5 is a chlorine, bromine or iodine atom and,

b) when W represents a group =CH-QH this corresponds to a propanamine (I.A.3) or a group =C[Q-(CH2)n-Q] this corresponds to a propanamine (I.D.3), in reacting a propanamine (I.A.2) or a propanamine (I.D.2) with an aldehyde R7-CHO which is a homologue smaller by one atom than R4 (R4 = -CH2-R7) and a reducing hydride (Hm) of formula (Hm.3) previously defined,

or in reducing with a metallic or organometallic hydride (Hm), of formula (Hm.2) previously defined, an N-carboxamide of formula (V.A.3) or a carboxamide of formula (V.D.3)

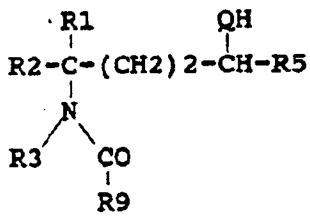

(V.A.3)

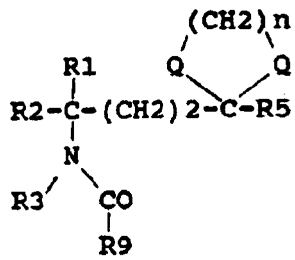

(V.D.3)

in which R9 is hydrogen or a homologous carbon-based radical smaller by one atom than R4 (R4 = -CH2-R9), or in substituting the carbonitrile radical with an aminonitrile of formula (VII.1) or of formula (VII.2)

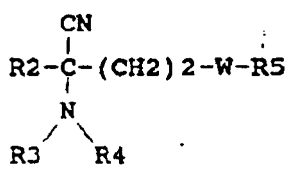

(VII.1)

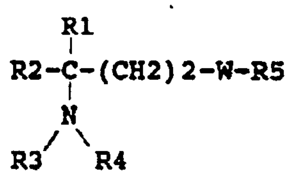

(VII.2)

by the carbon-based radicals R1 and R2 of the organometallic reagents R1-M-X7 and R2-M-X8 in which R1 represents the meanings indicated for the propanamines (I) with the exception of the halogenated radicals,
R2 is a $C_1$ to $C_4$ alkyl,
M is a divalent metal atom chosen from magnesium, cadmium and zinc,
X7 and X8 are halogen atoms chosen from chlorine, bromine and iodine, and
C.4. for preparing propanamines (I) in which neither R3 nor R4 are hydrogen,
a) when W is =CH-QH this corresponds to a propanamine of formula (I.A.3)

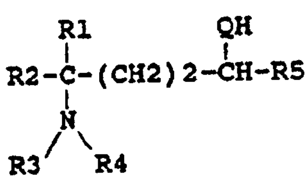

(I.A.3)

in reducing, with a metallic or organometallic hydride (Hm) in which by preference M2 is boron and M2 is sodium, and propanamine of formula (I.C.3),
b) and, when W is =C=O this corresponds to a propanamine of formula (I.C.3/O), in oxidising, by mono- or polyatomic ionic oxidation-reduction systems where the normal potential E at 20 C is higher than 0.60V, a propanamine (I.A.3/O),
or, in hydrolysing with an acid solution the group W when Q is oxygen or by an solution oxidising the group W when Q is sulphur, a propanamine (I.D.3),

c) and, when W is =C=S, this corresponds to propanamine (I.C.3/S) to produce a thio-oxo substitution reaction with an intermediate (VI.2) of formula

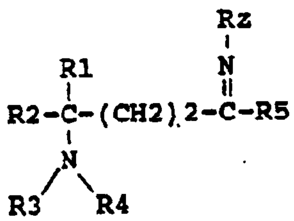

(VI.2)

d) and when W is a group =C[Q-CH2)n-Q] this corresponds to a propanamine of formula (I.D.3)

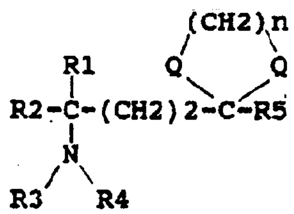

(I.D.3)

in condensing a propanamine (I.C.3) with a bifunctional reagent HQ-(CH2)n-QH in which n has a value 2 or 3, Q being sulphur or oxygen.

**11.** Pharmaceutical composition characterised in that it comprises a propanamine (I) as defined in claims 1 to 9.

**12.** Use of a propanamine (I), as defined in claims 1 to 9, for production of a pharmaceutical composition intended for treatment diarrhoeic conditions.

**13.** Intermediates of formula (XX)

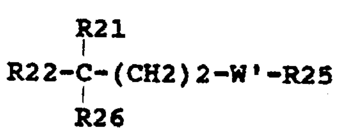

(XX)

in which

R21 is a phenyl radical optionally mono-, di- or trisubstituted in an identical or different manner with halogen atoms, $C_1$ to $C_4$ alkyl radicals, $C_1$ to $C_4$ haloalkyl radicals, $C_1$ to $C_4$ alkoxy radicals or a 5 or 6 membered monocyclic heteroaryl radical in which the single heteroatom is nitrogen oxygen or sulphur, or is a carbonitrile radical when R22 is $C_1$ to $C_4$ alkyl

R22 is, when R21 is a phenyl or heteroaryl radical as defined above, a $C_1$ to $C_4$ alkyl radical or a carbonitrile radical

R25 is a 5 to 7 membered cycloalkyl radical, a phenyl radical or a 5 to 6 membered monocyclic heteroaryl radical in which the single heteroatom is nitrogen, oxygen or sulphur and which are optionally mono-, di- or trisubstituted in an identical or different manner by the halogen atoms, the $C_1$ to $C_4$ alkyl radicals, $C_1$ to $C_4$ haloalkyl radicals or $C_1$ to $C_4$ alkoxy,

R26 is a nitro radical when neither R21 nor R22 are carbonitrile or when one ofR21 or R22 is carbonitrile is a grdup -N(R23)R24 in which R23 and R24 are a $C_1$ to $C_4$ alkyl radical, $C_1$ to $C_4$ alkenyl radical, cycloalkylalkyl with $C_3$ to $C_7$ cycloalkyl and $C_1$ to $C_4$ alkyl, which may be different or identical without both being cycloalkylalkyl, or R23 and R24 together, with the nitrogen atom to which they are attached, form a saturated 5 to 6 membered heterocycle containing only one heteroatom, and

W′ represents a group =CH-QH or a heterocycle =C[Q-(CH2)n-Q] when R21 or R22 are carbonitrile,

and in which Q is an oxygen or sulphur atom, or a group =C=Q when R26 represents a nitro group, Q then being an oxygen atom, sulphur or a group N-Ry in which Ry is hydrogen or a $C_1$ to $C_4$ alkyl radical, n has the value 2 or 3.

**Claims for the following Contracting State : ES**

1. Process for the preparation of propanamines of general formula (I):

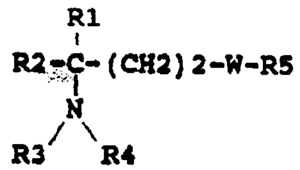

(I)

in which:

| | |
|---|---|
| R1 | is a phenyl radical, optionally mono-, di- or trisubstituted in an identical or different manner by halogen atoms, $C_1$ to $C_4$ alkyl radicals, $C_1$ to $C_4$ haloalkyl, $C_1$ to $C_4$ alkoxy, or is a 5 or 6 membered heteroaryl monocyclic radical in which the single heteroatom is nitrogen, oxygen or sulphur, |
| R2 | is a $C_1$ to $C_4$ alkyl radical, |
| R3 and R4 | are a hydrogen atom, a $C_1$ to $C_4$ alkyl radical, $C_1$ to $C_4$ alkenyl, cycloalkylalkyl with $C_3$ to $C_7$ cycloalkyl and $C_1$ to $C_4$ alkyl, and are different or identical without, however, both being cycloalkylalkyl, or, together with the nitrogen atom to which they are attached, form a saturated 5 or 6 membered heterocycle, |
| R5 | is a 5 to 7 membered radical cycloalkyl, a phenyl radical or a 5 or 6 membered monocyclic heterocycle radical in which the single heteroatom is nitrogen, oxygen or sulphur and which are optionally mono-, di- or trisubstituted in an identical or different manner by halogen atoms, $C_1$ to $C_4$ alkyl radicals, $C_1$ to $C_4$ haloalkyls or $C_1$ to $C_4$ alkoxy, and W represents a group =CH-QH (group I.A)<br>or a heterocycle =C[Q-(CH2)n-Q] )group I.D)<br>or a group =C=Q (group (I.C),<br>in which<br>Q is an oxygen or sulphur atom,<br>n has the value 2 or 3<br>W being =C=Q only when R3 and R4 are not both hydrogen,<br>and their salts of addition with acids,<br>characterised in that it comprises: |

A. for preparing a propanamine in which R3 and R4 are hydrogen,

a) when W represents the group =CH-QH which corresponds to a propanamine of formula (I.A.1.)

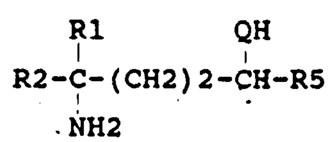

(I.A.1)

in reducing a nitro compound (III)

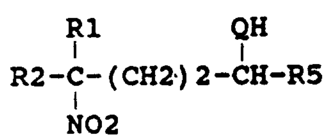

(III)

by hydrogenation catalysed by an element from group VIII of the Periodic Table of the Elements and

b) when W represents the group =C[Q-(CH2)n-Q] which corresponds to a propanamine of formula

(I.D.1)

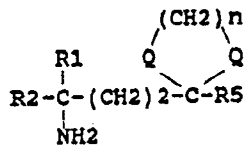

(I.D.1)

in reducing a nitro compound (IV)

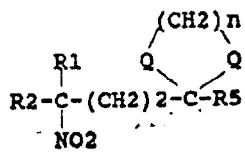

(IV)

by hydrogenation catalysed by an element from group VIII of the Periodic Table of the Elements,

B. for preparing a propanamine in which R4 is hydrogen and is different from R3,

a) when W represent a group =CH-QH, which corresponds to a propanamine (I.A.2),

in alkylating a propanamine of formula (I.A.1) by an alkylation reagent R3-X1 in which X1 is a halogen atom such a chlorine, bromine or iodine,

or, in reducing by a metallic or organometallic hydride (Hm): M1(t)M2H(r)RX(s) in which M1 is an alkaline metal, M2 is an element from group III of the Periodic Classification, RX is a carbonitrile group or an alkyl radical or $C_1$ to $C_4$ alkoxy, (t) is equal to zero or one, (r) is equal to three or four and (s) is equal to zero, one, three of four and in which (r) + (s) -(t) =3, and formula (HM.2) in which M2 is aluminium or, when M2 is boron (r) becomes 3, (s) and (t) have the value zero, an N-carboxamide of formula (V.A.2.)

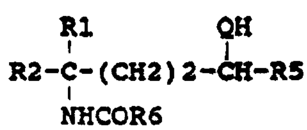

(V.A.2.)

in which R6 is hydrogen or a lower homologous radical smaller by one carbon atom than R3 (R3 = -CH2-R6),

b) when W represents the group =C[Q-(CH2)n-Q], which corresponds to a propanamine (I.D.2), in alkylating a propanamine of formula (I.D.1) by an alkylation reagent R3-X1 in which X1 is a halogen atom such as chlorine, bromine or iodine,

or in reducing by a previously defined metallic or organometallic hydride (Hm.2) an N-carboxamide of formula (V.D.2)

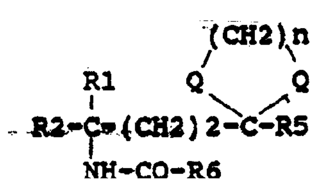

(V.D.2)a

c) when W represents the group =C=Q where Q represents oxygen, which corresponds to a propanamine (I.C.2/O) of formula

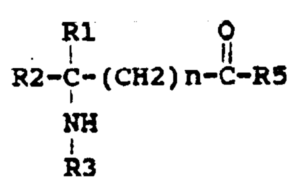

(I.C.2/O)

to oxidise by a diatomic species or a polyatomic ion whose normal redox potential E is higher than 0.60V a propanamine (I.A.2) in which Q is oxygen,
or in hydrolysing with an acid solution where in the group -Q(CH2)nQ- Q is oxygen and by an oxidising solution where in the group -Q(CH2)nQ- Q is sulphur, a propanamine (I.D.2),
d) when W represents the group =C=Q in which Q represents sulphur, this corresponds to a propanamine (I.C.2.S) of formula

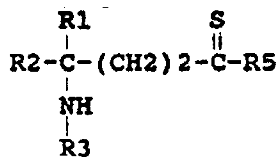

(I.C.2/S)

by reacting a propanamine (I.C.2/O) with phosphorous pentasulphide, or in carrying out a thio-oxo substitution reaction between a compound (VI.1) of formula

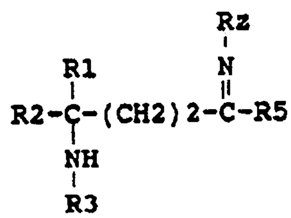

(VI.1)

in which Rz is hydrogen, an amine radical (-NH2), hydroxyl (-OH), a lower alkyl radical or a phenyl radical,
and hydrogen sulphide, carbon sulphide, sulphur monochloride, an S-alkylthioic acid in which the alkyl radical comprises a linear or branched chain or one to five carbon atoms or an S-phenylthioc acid such as S-thiobenzoic acid,
C. to prepare a propanamine (I) in which R3 and R4 are not hydrogen, C.1. and, when R3 and R4 are identical and represent $C_1$ to $C_4$ alkyl or alkenyl radicals,
a) when W represent a group =CH-QH, this corresponds to a propanamine (I.A.3) or a group =([Q-(CH2)n-Q) in a propanamine (I.D.3)

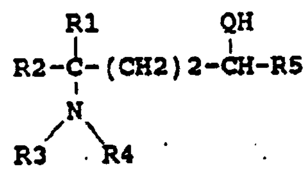

(I.A.3)

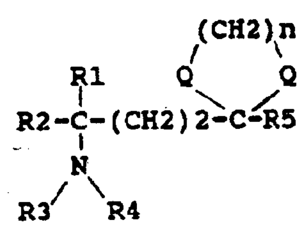

(I.D.3)

in dialkylating a propanamine of formula (I.A.1) of formula (I.D.1) with an aldehyde R7-CHO in which R7 is hydrogen or a homologous alkyl or alkenyl radical having one carbon atom less than R3 and R4 (R3=R3=CH2-R7), and with a reducing agent which is an organometallic hydride (Hm) in which it is preferred that M2 is boron and Rx is a carbonitrile group (Hm.3) or formic acid or one of its salts,
b) when W represents a group =C=O, this corresponds to a propanamine (I.C.3/O)

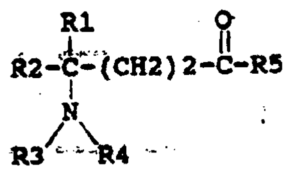

(I.C.3/O)

in oxidising a corresponding propanamine in which W is a group =CH-OH, by a diatomic species or a polyatomic ion whose normal redox potential E is higher than 0.60V,
or in hydrolysing a propanamine (I.D.3) by an acid solution when in the group [Q-(CH2)n-Q] Q is oxygen and by an oxydising solution, when in the group [Q-(CH2)n-Q) Q is sulphur, a propanamine (I.D.2),
c) when C represent a group =C=S, this corresponds to a propanamine (I.C.3/S)

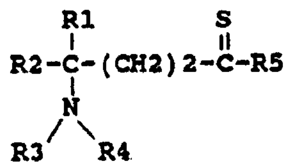

(I.C.3/S)

by reacting a propanamine (I.C.3/O) with phosphorous pentasulphide, or carrying out a thio-oxo substitution reaction between a compound (VI.2) of formula

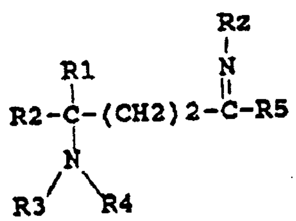

(VI.2)

in which Rz is hydrogen, an amine radical (-NH2), hydroxyl (-OH) a $C_1$ to $C_4$ alkyl radical or a phenyl radical, and hydrogen sulphide, carbon sulphide, sulphur monochloride, and S-alkylthioic acid in which the alkyl radical contains a linear or branched chain or an S-phenylthioic acid such as S-thiobenzoic acid,
C.2. and, to prepare a propanamine (I) in which R3 and R4 together and with the nitrogen atom to which they are attached, form a 5 to 6 membered saturated heterocycle,
in dialkylating a propanamine (I.D.1) with a dihalogenated reagent X3-(CH2)q-X4 in which X3 and X4 are identical or different are chlorine, bromide or iodine and q has a value 4 or 5, to obtain propanamine (I.D.3) of formula

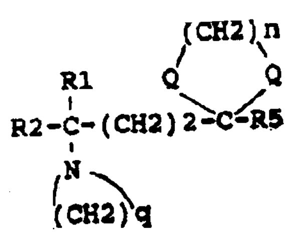

(I.D.3)

and in preparing a corresponding propanamine (I.C.3/O) by hydrolysing with an acid or an oxidant in the previous case defines a propanamine (I.D.3),
and in preparing a corresponding propanamine (I.C.3/S) by reacting a propanamine (I.C.3/O) with phosphorous pentasulphide or in preparing an intermediate compound (VI.2) which is used in a thio-oxo substitution reaction with the reagents previously cited,
and in preparing a propanamine ((I.A.3/O) by reduction with a propanamine (I.C.3/O), and a propanamine (I.A.3/S) by reduction with a propanamine (I.C.3/S) with a metallic or organometallic hydride (Hm) in which it is preferred that M2 is boron and M1 is sodium,

C.3. for preparing a propanamine (I) in which R3 and R4 are different from each other, and are not hydrogen and,

a) when W represents a group =C=Q, this corresponds to a propanamine (I.C.3) or a group =C[Q-CH2)n-Q] which corresponds to a propanamine (I.D.3)

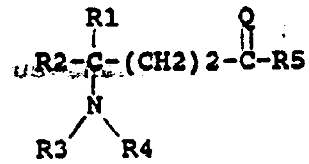

(I.C.3)

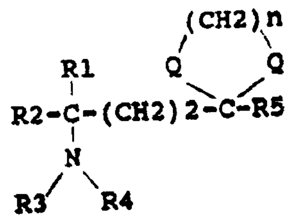

(I.D.3)

in alkylating a propanamine of formula (I.D.2) or of formula (I.C.2) in which R4 is hydrogen with an alkylating reagent R4X5 in which X5 is a chlorine, bromine or iodine atom and,

b) when W represents a group =CH-QH this corresponds to a propanamine (I.A.3) or a group =C[Q-(CH2)n-Q] this corresponds to a propanamine (I.D.3), in reacting a propanamine (I.A.2) or a propanamine (I.D.2) with an aldehyde R7-CHO which is a homologue smaller by one atom than R4 (R4 = -CH2-R7) and a reducing hydride (Hm) of formula (Hm.3) previously defined,

or in reducing with a metallic or organometallic hydride (Hm), of formula (Hm.2) previously defined, an N-carboxamide of formula (V.A.3) or a carboxamide of formula (V.D.3)

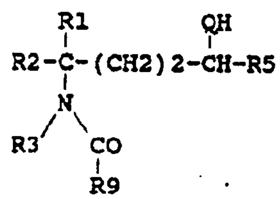

(V.A.3)

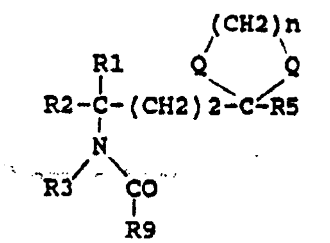

(V.D.3)

in which R9 is hydrogen or a homologous carbon-based radical smaller by one atom than R4 (R4 = -CH2-R9), or in substituting the carbonitrile radical with an aminonitrile of formula (VII.1) or of formula (VII.2)

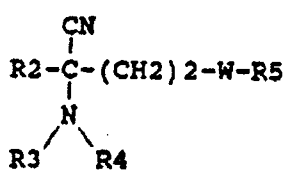

(VII.1)

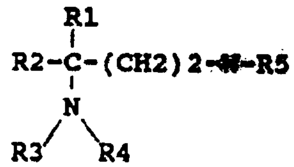

(VII.2)

by the carbon-based radicals R1 and R2 of the organometallic reagents R1-M-X7 and R2-M-X8 in which R1 represents the meanings indicated for the propanamines (I) with the exception of the halogenated radicals R2 is a $C_1$ to $C_4$alkyl,

M is a divalent metal atom chosen from magnesium, cadmium and zinc, X7 and X8 are halogen atoms chosen from chlorine, bromine and iodine, and

C.4. for preparing propanamines (I) in which neither R3 nor R4 are hydrogen,

a) when W is =CH-QH this corresponds to a propanamine of formula (I.A.3)

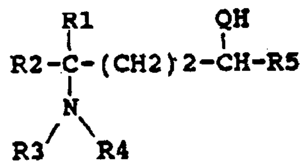

(I.A.3)

in reducing, with a metallic or organometallic hydride (Hm) in which by preference M2 is boron and M2 is sodium, and propanamine of formula (I.C.3),

b) and, when W is =C=O this corresponds to a propanamine of formula (I.C.3/O), in oxidising, by mono- or polyatomic ionic oxidation-reduction systems where the normal potential E at 20 C is higher than 0.60V, a propanamine (I.A.3/O),

or, in hydrolysing with an acid solution the group W when Q is oxyegn or by an solution oxidising the group W when Q is sulphur, a propanamine (I.D.3),

c) and, when W is =C=S, this corresponds to propanamine (I.C.3/S) to produce a thio-oxo substitution reaction with an intermediate (VI.2) of formula

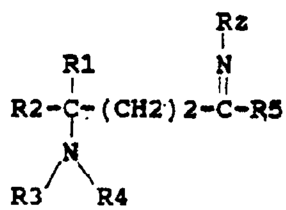

(VI.2)

d) and when W is a group =C[Q-CH2)n-Q] this corresponds to a propanamine of formula (I.D.3)

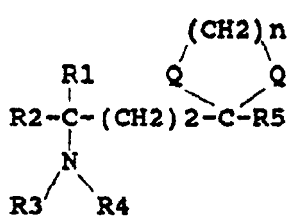

(I.D.3)

in condensing a propanamine (I.C.3) with a bifunctional reagent HQ-(CH2)n-QH in which n has a value 2 or 3, Q being sulphur or oxygen.

2. Process according to claim 1, characterised in that R1 and R5 are phenyl radicals optionally mono-, di- or trisubstituted.

3. Process according to claims 1 or 2 characterised in that W represents a group =C[Q-(CH2)n-Q] with Q representing an oxygen atom.

4. Process according to claim 3, characterised in that n is equal to 2.

5. Process according to claim 2, characterised in that W represents a carbonyl group.

6. Process according to claims 1 to 5, characterised in that R3 is methyl and R4 is hydrogen.

7. Propanamines according to claims 1 to 5, characterised in that R3 and R4 and each a methyl radical.

8. Process according to claim 1, characterised in that R1 is a trifluoromethylphenyl radical, R2 is ethyl, R3 is hydrogen, R4 is methyl, W is =C[O-(CH2)2-O] and R5 is 3,4,5-trimethoxyphenyl.

9. Process according to claim 1, characterised in that R1 is 4-methylphenyl, R2 is ethyl, R3 and R4 are both methyl, W is =C=O, and R5 is 3,4,5-trimethoxyphenyl.

10. Process for preparing a medicament, characterised in that it comprises mixing a propanamine of formual (I) as defined in any of claims 1 to 9, with an pharmaceutically acceptable excipient.

11. Use of a propanamine (I) as defined in any of claims 1 to 9 to obtain a medicament for the treatment of diarrhoic conditions.

**Claims for the following Contracting State : GR**

1. Propanamines of general formula (I):

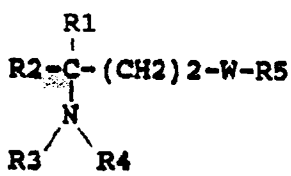

(I)

in which:

R1 is a phenyl radical, optionally mono-, di- or trisubstituted in an identical or different manner by halogen atoms, $C_1$ to $C_4$ alkyl radicals, $C_1$ to $C_4$ haloalkyl, $C_1$ to $C_4$ alkoxy,
or is a 5 or 6 membered heteroaryl monocyclic radical in which the single heteroatom is nitrogen, oxygen or sulphur,

R2 is a $C_1$ to $C_4$ alkyl radical,

R3 and R4 are a hydrogen atom, a $C_1$ to $C_4$ alkyl radical, $C_1$ to $C_4$ alkenyl, cycloalkylalkyl with $C_3$ to $C_7$ cycloalkyl and $C_1$ to $C_4$ alkyl, and are different or identical without, however, both being cycloalkylalkyl,
or, together with the nitrogen atom to which they are attached, form a saturated 5 or 6 membered heterocycle,

R5 is a 5 to 7 membered radical cycloalkyl, a phenyl radical or a 5 or 6 membered monocyclic heterocycle radical in which the single heteroatom is nitrogen, oxygen or sulphur and which are optionally mono-, di- or trisubstituted in an identical or different manner by $C_1$ to $C_4$ alkyl radicals, $C_1$ to $C_4$ haloalkyls or $C_1$ to $C_4$ alkoxy,
and W represents a group =CH-QH
or a heterocycle =C[Q-(CH2)n-Q]
or a group =C=Q
in which
Q is an oxygen or sulphur atom,
n has the value 2 or 3
W being =C=Q only when R3 and R4 are not both hydrogen,
and their salts of addition with acids.

2. Propanamines according to claim 1, characterised in that R1 and R5 are phenyl radicals optionally mono-,

di- or trisubstituted.

3. Propanamines according to claims 1 or 2 characterised in that W represents a group =C[Q-(CH2)n-Q] with Q representing an oxygen atom.

4. Propanamines according to claim 3, characterised in that n is equal to 2.

5. Propanamines according to claim 2, characterised in that W represents a carbonyl group.

6. Propanamines according to claims 1 to 5, characterised in that R3 is methyl and R4 is hydrogen.

7. Propanamines according to claims 1 to 5, characterised in that R3 and R4 and each a methyl radical.

8. 2-[3-N-methylamino-3-(4-trifluoromethylphenyl)-pentan-1-yl]-2-(3,4,5-trimethoxyphenyl)-1,3,dioxolane, and its salts of addition with acids.

9. 4-N,N-dimethylamino-4-(4-methylphenyl)-1-(3,4,5-trimethoxyphenyl)-hexan-1-one, and its salts of addition with acids.

10. Process for the preparation of propanamines of formula (I) as defined in claim 1, characterised in that it comprises:
A. for preparing a propanamine in which R3 and R4 are hydrogen,
a) when W represents the group =CH-QH which corresponds to a propanamine of formula (I.A.1.)

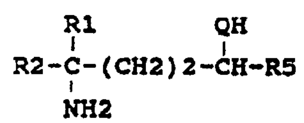

(I.A.1)

in reducing a nitro compound (III)

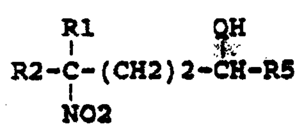

(III)

by hydrogenation catalysed by an element from group VIII of the Periodic Table of the Elements and
b) when W represents the group =C[Q-(CH2)n-Q] which corresponds to a propanamine of formula (I.D.1)

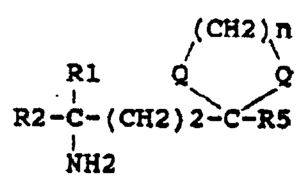

(I.D.1)

in reducing a nitro compound (IV)

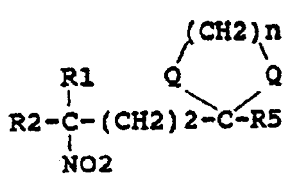

(IV)

by hydrogenation catalysed by an element from group VIII of the Periodic Table of the Elements,
B. for preparing a propanamine in which R4 is hydrogen and is different from R3,
a) when W represent a group =CH-QH, which corresponds to a propanamine (I.A.2),
in alkylating a propanamine of formula (I.A.1) by an alkylation reagent R3-X1 in which X1 is a halogen

atom such a chlorine, bromine or iodine,

or, in reducing by a metallic or organometallic hydride (Hm): M1(t)M2H(r)RX(s) in which M1 is an alkaline metal, M2 is an element from group III of the Periodic Classification, RX is a carbonitrile group or an alkyl radical or $C_1$ to $C_4$ alkoxy, (t) is equal to zero or one, (r) is equal to three or four and (s) is equal to zero, one, three of four and in which (r) + (s) -(t) =3, and in formula (HM.2) in which M2 is aluminium or, when M2 is boron (r) becomes 3, (s) and (t) have the value zero, an N-carboxamide of formula (V.A.2.)

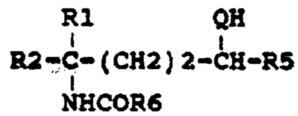

(V.A.2.)

in which R6 is hydrogen or a lower homologous radical smaller by one carbon atom than R3 (R3 = -CH2-R6),

b) when W represents the group =C[Q-(CH2)n-Q], which corresponds to a propanamine (I.D.2), in alkylating a propanamine of formula (I.D.1) by an alkylation reagent R3-X1 in which X1 is a halogen atom such as chlorine, bromine or iodine,

or in reducing by a previously defined metallic or organometallic hydride (Hm.2) an N-carboxamide of formula (V.D.2)

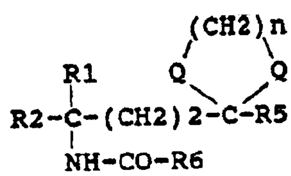

(V.D.2)

c) when W represents the group =C=Q where Q represents oxygen, which corresponds to a propanamine (I.C.2/O) of formula

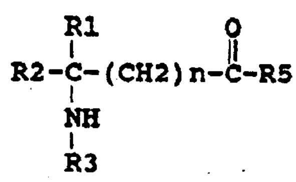

(I.C.2/O)

to oxidise by a diatomic species or a polyatomic ion whose normal redox potential E is higher than 0.60V a propanamine (I.A.2) in which Q is oxygen,

or in hydrolysing with an acid solution where in the group -Q(CH2)nQ- Q is oxygen and by an oxidising solution where in the group -Q(CH2)nQ- Q is sulphur, a propanamine (I.D.2),

d) when W represents the group =C=Q in which Q represents sulphur, this corresponds to a propanamine (I.C.2/S) of formula

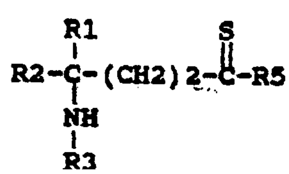

(I.C.2/S)

by reacting a propanamine (I.C.2/O) with phosphorous pentasulphide, or in carrying out a thio-oxo substitution reaction between a compound (VI.1) of formula

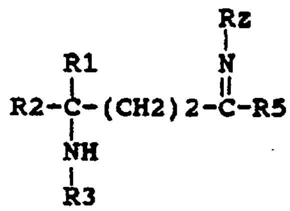

(VI.1)

in which Rz is hydrogen, an amine radical (-NH2), hydroxyl (-OH), a lower alkyl radical or a phenyl radical,

and hydrogen sulphide, carbon sulphide, sulphur monochloride, an S-alkylthioic acid in which the alkyl radical comprises a linear or branched chain or one to five carbon atoms or an S-phenylthioc acid such as S-thiobenzoic acid,

C. to prepare a propanamine (I) in which R3 and R4 are not hydrogen, C.1. and, when R3 and R4 are identical and represent $C_1$ to $C_4$ alkyl or alkenyl radicals,

a) when W represent a group =CH-QH, this corresponds to a propanamine (I.A.3) or a group =([Q-(CH2)n-Q) in a propanamine (I.D.3),

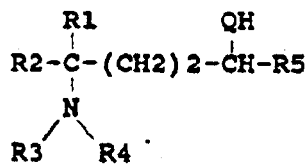

(I.A.3)

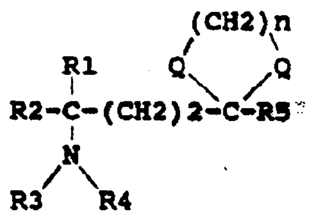

(I.D.3)

in dialkylating a propanamine of formula (I.A.1) of formula (I.D.1) with an aldehyde R7-CHO in which R7 is hydrogen or a homologous alkyl or alkenyl radical having one carbon atom less than R3 and R4 (R3=R3=CH2-R7), and with a reducing agent which is an organometallic hydride (Hm) in which it is preferred that M2 is boron and Rx is a carbonitrile group (Hm.3) or formic acid or one of its salts,

b) when W represents a group =C=O, this corresponds to a propanamine (I.C.3/O)

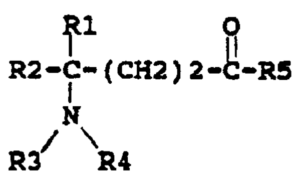

(I.C.3/O)

in oxidising a corresponding propanamine in which W is a group =CH-OH, by a diatomic species or a polyatomic ion whose normal redox potential E is higher than 0.60V,

or in hydrolysing a propanamine (I.D.3) by an acid solution when in the group [Q-(CH2)n-Q] Q is oxygen and by an oxydising solution, when in the group [Q-(CH2)n-Q) Q is sulphur, a propanamine (I.D.2),

c) when C represent a group =C=S, this corresponds to a propanamine (I.C.3/S)

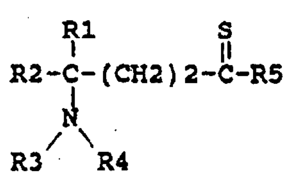

(I.C.3/S)

by reacting a propanamine (I.C.3/O) with phosphorous pentasulphide, or in carrying out a thio-oxo substitution reaction between a compound (VI.2) of formula

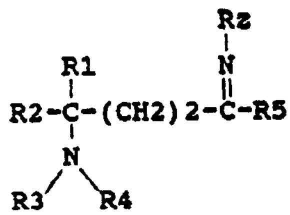

(VI.2)

in which Rz is hydrogen, an amine radical (-NH2), hydroxyl (-OH) a $C_1$ to $C_4$ alkyl radical or a phenyl radical, and hydrogen sulphide, carbon sulphide, sulphur monochloride, and S-alkylthioic acid in which the alkyl radical contains a linear or branched chain or an S-phenylthioic acid such as S-thiobenzoic acid,

C.2. and, to prepare a propanamine (I) in which R3 and R4 together and with the nitrogen atom to which they are attached, form a 5 to 6 membered saturated heterocycle,

in dialkylating a propanamine (I.D.1) with a dihalogenated reagent X3-(CH2)q-X4 in which X3 and X4 are identical or different are chlorine, bromide or iodine and n has a value 4 or 5, to obtain propanamine (I.D.3) of formula

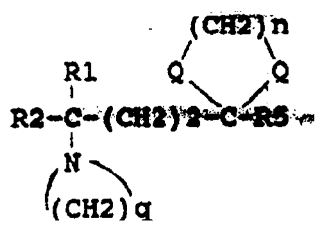

(I.D.3)

and in preparing a corresponding propanamine (I.C.3/O) by hydrolysing with an acid or an oxidant in the previous case defines a propanamine (I.D.3),

and in preparing a corresponding propanamine (I.C.3/S) by reacting a propanamine (I.C.3/O) with phosphorous pentasulphide or in preparing an intermediate compound (VI.2) which is used in a thio-oxo substitution reaction with the reagents previously cited,

and to prepare a propanamine ((I.A.3/O) by reduction with a propanamine (I.C.3/O), and a propanamine (I.A.3/S) by reduction with a propanamine (I.C.3/S) with a metallic or organometallic hydride (Hm) in which it is preferred that M2 is boron and M1 is sodium,

C.3. for preparing a propanamine (I) in which R3 and R4 are different from each other, and are not hydrogen and,

a) when W represents a group =C=Q, this corresponds to a propanamine (I.C.3) or a group =C[Q-CH2)n-Q] which corresponds to a propanamine (I.D.3)

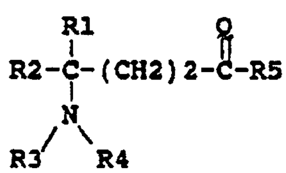

(I.C.3)

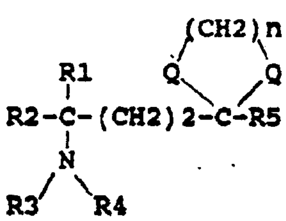

(I.D.3)

in alkylating a propanamine of formula (I.D.2) or of formula (I.C.2) in which R4 is hydrogen with an alkylating reagent R4X5 in which X5 is a chlorine, bromine or iodine atom and,

b) when W represents a group =CH-QH this corresponds to a propanamine (I.A.3) or a group =C[Q-

(CH2)n-Q] this corresponds to a propanamine (I.D.3), in reacting a propanamine (I.A.2) or a propanamine (I.D.2) with an aldehyde R7-CHO which is a homologue smaller by one atom than R4 (R4 = -CH2-R7) and a reducing hydride (Hm) of formula (Hm.3) previously defined,
or in reducing with a metallic or organometallic hydride (Hm), of formula (Hm.2) previously defined, an N-carboxamide of formula (V.A.3) or a carboxamide of formula (V.D.3)

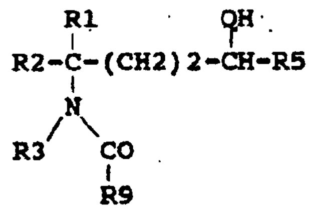

(V.A.3)

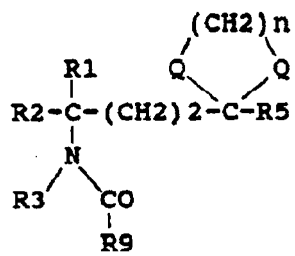

(V.D.3)

in which R9 is hydrogen or a homologous carbon-based radical smaller by one atom than R4 (R4 = -CH2-R9), or in substituting the carbonitrile radical with an aminonitrile of formula (VII.1) or of formula (VII.2)

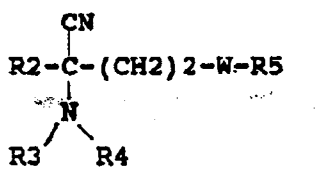

(VII.1)

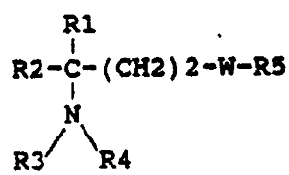

(VII.2)

by the carbon-based radicals R1 and R2 of the organometallic reagents R1-M-X7 and R2-M-X8 in which R1 represents the meanings indicated for the propanamines (I) with the exception of the halogenated radicals, R2 is a $C_1$ to $C_4$alkyl,
M is a divalent metal atom chosen from magnesium, cadmium and zinc,
X7 and X8 are halogen atoms chosen from chlorine, bromine and iodine, and
C.4. for preparing propanamines (I) in which neither R3 nor R4 are hydrogen,
a) when W is =CH-QH this corresponds to a propanamine of formula (I.A.3)

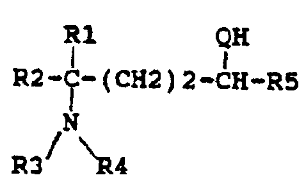

(I.A.3)

in reducing, with a metallic or organometallic hydride (Hm) in which by preference M2 is boron and M2 is sodium, and propanamine of formula (I.C.3),

b) and, when W is =C=O this corresponds to a propanamine of formula (I.C.3/O), in oxidising, by mono- or polyatomic ionic oxidation-reduction systems where the normal potential E at 20 C is higher than 0.60V, a propanamine (I.A.3/O),

or, in hydrolysing with an acid solution the group W when Q oxygen or by a solution oxidising the group W when Q is sulphur, a propanamine (I.D.3),

c) and, when W is =C=S, this corresponds to propanamine (I.C.3/S) to produce a thio-oxo substitution reaction with an intermediate (VI.2) of formula

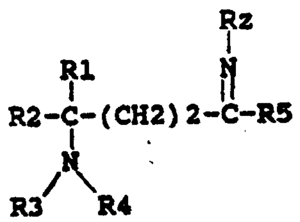

(VI.2)

d) and when W is a group =C[Q-CH2)n-Q] this corresponds to a propanamine of formula (I.D.3)

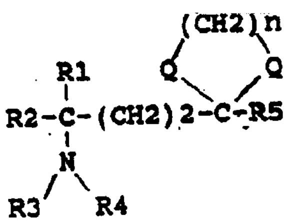

(I.D.3)

in condensing a propanamine (I.C.3) with a bifunctional reagent HQ-(CH2)n-QH in which n has a value 2 or 3, Q being sulphur or oxygen.

**11.** Process for preparation of a medicament characterised in that it consists of mixing a propanamine of formual (I) as defined in any of claims 1 to 9 with a pharmaceutically acceptable excipient.

**12.** Use of a propanamine (I), as defined in claims 1 to 9, for production of a medicament intended for treatment of diarrhoeic conditions.

**13.** Intermediates of formula (XX)

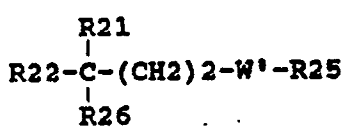

(XX)

in which

R21 is a phenyl radical optionally mono-, di- or trisubstituted in an identical or different manner with halogen atoms, $C_1$ to $C_4$ alkyl radicals, $C_1$ to $C_4$ haloalkyl radicals, $C_1$ to $C_4$ alkoxy radicals or a 5 or 6 membered monocyclic heteroaryl radical in which the single heteroatom is nitrogen oxygen or sulphur, or is a carbonitrile radical when R22 is $C_1$ to $C_4$ alkyl

R22 is, when R21 is a phenyl or heteroaryl radical as defined above, a $C_1$ to $C_4$ alkyl radical or a carbonitrile radical

R25 is a 5 to 7 membered cycloalkyl radical, a phenyl radical or a 5 to 6 membered monocyclic heteroaryl radical in which the single heteroatom is nitrogen, oxygen or sulphur and which are optionally mono-, di- or trisubstituted in an identical or different manner by the halogen atoms, the $C_1$ to $C_4$ alkyl radicals, $C_1$ to $C_4$ haloalkyl radicals or $C_1$ to $C_4$ alkoxy,

R26 is a nitro radical when neither R21 nor R22 are carbonitrile or when one of R21 or R22 is carbonitrile is a group -N(R23)R24 in which R23 and R24 are a $C_1$ to $C_4$ alkyl radical, $C_1$ to $C_4$ alkenyl radical, cycloalkylalkyl with $C_3$ to $C_7$ cycloalkyl and $C_1$ to $C_4$ alkyl, which may be different or identical without both being cycloalkylalkyl, or R23 and R24 together, with the nitrogen atom to which they are attached, form a saturated 5 to 6 membered heterocycle containing only one heteroatom, and

W' represents a group =CH-QH or a heterocycle =C[Q-(CH2)n-Q] when R21 or R22 are carbonitrile, and in which Q is an oxygen or sulphur atom, or a group =C=Q when R26 represents a nitro group, Q then being an oxygen atom, sulphur or a group N-Ry in which Ry is hydrogen or a $C_1$ to $C_4$ alkyl radical, n has the value 2 or 3.